# EUROPEAN PATENT SPECIFICATION

(11) **EP 1 392 846 B1**
(45) Date of publication and mention of the grant of the patent: **11.06.2008**
(21) Application number: 02731102.6
(22) Date of filing: 26.02.2002
(51) Int. Cl.: C12N 15/62, C12N 15/52, C12N 15/63, C12N 9/78, C07K 14/47, C07K 19/00, C12N 5/10, A61K 38/17, A61K 38/50

(54) **METHODS AND COMPOSITIONS FOR MODIFYING APOLIPOPROTEIN B mRNA EDITING**
VERFAHREN UND ZUSAMMENSETZUNGEN ZUR MODIFIKATION DER BEARBEITUNG VON APOLIPOPROTEIN B-mRNA
PROCEDES ET COMPOSITIONS PERMETTANT DE MODIFIER L'EDITION DES ARN M DE L'APOLIPOPROTEINE B

(30) Priority: 27.02.2001 US 271856 P
(43) Date of publication of application: 03.03.2004
(73) Proprietor: UNIVERSITY OF ROCHESTER, Rochester, New York 14627 (US)
(72) Inventor: SMITH, Harold, C., Rochester, NY 14623 (US); YANG, Yan, Rochester, NY 14620 (US); SOWDEN, Mark, P., Penfield, NY 14526 (US)
(74) Representative: Gardner, Rebecca Katherine
(86) International application number: PCT/US2002/005824
(87) International publication number: WO 2002/068676

(56) References cited:
- US-A- 5 804 185
- US-A- 6 087 108
- MEHTA ANURADHA ET AL: "Molecular cloning of apobec-1 complementation factor, a novel RNA-binding protein involved in the editing of apolipoprotein B mRNA" MOLECULAR AND CELLULAR BIOLOGY, vol. 20, no. 5, March 2000 (2000-03), pages 1846-1854, XP002336417 ISSN: 0270-7306
- YANG Y ET AL: "Induction of cytidine to uridine editing on cytoplasmic apolipoprotein B mRNA by overexpressing APOBEC-1." THE JOURNAL OF BIOLOGICAL CHEMISTRY. 28 JUL 2000, vol. 275, no. 30, 28 July 2000 (2000-07-28), pages 22663-22669, XP002336418 ISSN: 0021-9258
- NAGAHARA H ET AL: "Transduction of full-length TAT fusion proteins into mammalian cells: TAT-p27Kip1 induces cell migration." NATURE MEDICINE. DEC 1998, vol. 4, no. 12, December 1998 (1998-12), pages 1449-1452, XP002336816 ISSN: 1078-8956

## Description

### FIELD OF THE INVENTION

The present invention related generally to the chimeric proteins, compositions and products containing one or more chimeric proteins, as well as the use thereof to modify apolipoprotein B processing, to treat or prevent atherogenic diseases or disorders, and to modify the intravascular lipoprotein population.

### BACKGROUND OF THE INVENTION

Cholesterol is carried in blood by specific carrier proteins called apolipoproteins and from one tissue to another as lipoprotein particles. Apolipoprotein B is an integral and non-exchangeable structural component of lipoprotein particles referred to as chylomicrons, very low density lipoprotein ("VLDL"), and low density lipoprotein ("LDL"). Apolipoprotein B circulates in human plasma as two isoforms, apolipoprotein B100 and apolipoprotein B48. Apolipoprotein B48 is generated by an RNA editing mechanism which changes codon 2153 (CAA) to a translation stop codon (UAA) (Chen et al., "Apolipoprotein B-48 is the product of a messenger RNA with an organ-specific in-frame stop codon," Science 238:363-366 (1987); Powell et al., "A novel form of tissue-specific RNA processing produces apolipoprotein-B48 in intestine," Cell 50:831-840 (1987)). Editing is a site-specific deamination event catalyzed by apolipoprotein B mRNA editing catalytic subunit 1 (known as APOBEC-1) (Teng et al., "Molecular cloning of an apo B messenger RNA editing protein," Science 260:18116-1819 (1993)) with the help of auxiliary factors (Teng et al., "Molecular cloning of an apo B messenger RNA editing protein," Science 260:18116-1819 (1993)), Yang et al., "Partial characterization of the auxiliary factors involved in apo B mRNA editing through APOBEC-1 affinity chromatography," J. Biol. Chem. 272:27700-27706 (1997)); Yang et al., "Multiple protein domains determine the cell type-specific nuclear distribution of the catalytic subunit required for apo B mRNA editing," Proc. Natl. Acad. Sci. USA 94: 13075-13080 (1997)); Lellek et al., "Purification and Molecular cloning of a novel essential component of the apo B mRNA editing enzyme complex," J. Biol. Chem. 275: 19848-19856 (2000); Mehta et al., "Molecular cloning of apobec-1 complementation factor, a novel RNA-binding protein involved in the editing of apolipoprotein B mRNA," Mol. Cell. Biol. 20:1846-1854 (2000); Yang et al., "Induction of cytidine to uridine editing on cytoplasmic apolipoprotein B mRNA by overexpressing APOBEC-1," J. Biol. Chem. 275:22663-22669 (2000); Blanc et al., "Identification of GRY-RBP as an apoB mRNA binding protein that interacts with both apobec-1 and with apobec-1 complementation factor (ACF) to modulate C to U editing," J. Biol. Chem. 276:10272-10283 (2001)) as a holoenzyme or editosome (Smith et al. "In vitro apolipoprotein B mRNA editing: Identification of a 27S editing complex," Proc. Natl. Acad. Sci. USA 88:1489-1493 (1991)); Harris et al., "Extract-specific heterogeneity in high-order complexes containing apo B mRNA editing activity and RNA-binding proteins," J. Biol. Chem. 268:7382-7392 (1993))). Apolipoprotein B100 and apolipoprotein B48 play different roles in lipid metabolism, most importantly, apolipoprotein B100-associated lipoproteins (VLDL and LDL) are much more atherogenic than apolipoprotein B48-associated lipoproteins (chylomicrons and their remnants and VLDL).

Specifically, the apolipoprotein B48-associated lipoproteins are cleared from serum more rapidly than the apolipoprotein B 100-associated lipoproteins. As a result, apolipoprotein B48-VLDL usually are not present in serum for an amount of time sufficient for serum lipases to convert the VLDL to LDL. In contrast, the apolipoprotein B100-VLDL are present in the serum for sufficient amounts of time, allowing serum lipases to convert the VLDL to LDL. Elevated serum levels of LDL are of particular biomedical significance as they are associated with an increased risk of atherogenic diseases or disorders. Lipoprotein analyses have shown that the ability of mammalian liver to edit results in a lowering of the VLDL + LDL : HDL ratio. Therefore, it would be desirable to identify an approach for modifying apolipoprotein B editing which would favor an increase in the relative concentration of apolipoprotein B48 in proportion to apolipoprotein B100 (or total apolipoprotein concentration), thereby clearing a greater concentration of lipoproteins from serum and minimizing the atherogenic risks associated with high serum levels of VLDL and LDL.

Current lipid-lowering therapies include statins and bile-acid-binding resins. Statins are competitive inhibitors of hydroxymethylglutaryl-coenzyme A (HMG-CoA) reductase, which catalyzes the committed step in the synthesis of cholesterol (Davignon et al., "HMG-CoA reductase inhibitors: a look back and a look ahead," Can. J. Cardiol. 8:843-64 (1992)). Bile-acid-binding resins sequester bile acids in the intestine, thereby interrupting the enterohepatic circulation of bile acids and increasing the elimination of cholesterol from the body. These are effective therapies for some patients with hyperlipidemia; however, adverse effects have been observed in up to 30% of the patients, suggesting the need for alternative therapies. Mutations in the gene encoding the LDL-receptor or apolipoprotein B can cause a human genetic disease known as familial hypercholesterolemia, characterized by an elevated level of cholesterol and early atherosclerosis due to the defect in LDL-receptor mediated cholesterol uptake by cells (Goldstein et al., Familial hypercholesterolemia," In The Metabolic and Molecular Bases of Inherited Disease, Vol. 2., p1981-2030, Scriver et al. (eds.), McGraw-Hill, New York (1995)). Therapy for children with this disorder is needed in order to prevent morbidity or mortality, however the National Cholesterol Education Program (NCEP) recommends consideration of drug treatment only for children 10 years of age or older due to the risk that prolonged drug therapy may impair growth and pubertal development. Developing alternative approaches for lowering serum LDL levels is therefore essential for the sectors of the population still at risk.

Stimulating hepatic apolipoprotein B mRNA editing is a means of reducing serum LDL through the reduction in synthesis and secretion of apolipoprotein B100 containing VLDL. In most mammals (including humans), apolipoprotein B mRNA editing is carried out only in the small intestine. The presence of substantial editing in liver (found in 4 species) is associated with a less atherogenic lipoprotein profile compared with animals that do not have liver editing activity (Greeve et al., "Apolipoprotein B mRNA editing in 12 different mammalian species: hepatic expression is reflected in low concentrations of apoB-containing plasma lipoproteins," J. Lipid Res. 34:1367-1383 (1993)). APOBEC-1 is expressed in all tissues that cany out apolipoprotein B mRNA editing (Teng et al., "Molecular cloning of an apo B messenger RNA editing protein," Science 260:18116-1819 (1993)). Human liver does not express APOBEC-1 but it does express sufficient auxiliary proteins to complement exogenous APOBEC-1 in apolipoprotein B mRNA editing in transfected cells (Teng et al., "Molecular cloning of an apo B messenger RNA editing protein," Science 260:18116-1819 (1993)); Sowden et al., "Apolipoprotein B RNA Sequence 3' of the mooring sequence and cellular sources of auxiliary factors determine the location and extent of promiscuous editing," Nucleic Acids Res. 26:1644-1652 (1998)).

Transgenic experiments aiming to enhance hepatic editing through *apobec-1* gene transfer have shown a marked lowering of plasma apolipoprotein B 100 and significant reduction of serum LDL (Teng et al., "Adenovirus-mediated gene transfer of rat apolipoprotein B mRNA editing protein in mice virtually eliminates apolipoprotein B100 and normal low density lipoprotein production," J. Biol. Chem. 269:29395-29404 (1994)); Hughs et al., "Gene transfer of cytidine deaminase APOBEC-1 lowers lipoprotein(a) in transgenic mice and induces apolipoprotein B mRNA editing in rabbits," Hum. Gene Ther. 7:39-49 (1996)); Nakamuta et al., "Complete phenotypic characterization of the apobec-1 knockout mice with a wild-type genetic background and a human apolipoprotein B transgenic background, and restoration of apolipoprotein B mRNA editing by somatic gene transfer of Apobec-1," J. Biol. Chem. 271:25981-25988 (1996)); Kozarsky et al., "Hepatic expression of the catalytic subunit of the apolipoprotein B mRNA editing enzyme ameliorates hypercholesterolemia in LDL receptor-deficient rabbits," Hum. Gene Ther. 7:943-957 (1996)); Farese et al., "Phenotypic analysis of mice expressing exclusively apolipoprotein B48 or apolipoprotein B100," Proc. Natl. Acad. Sci. USA 93:6393-6398 (1996)); Qian et al., "Low expression of the apolipoprotein B mRNA editing transgene in mice reduces LDL but does not cause liver dysplasia or tumors," Arteriosc. Thromb. Vasc. Biol. 18:1013-1020 (1998)); Wu et al., "Normal perinatal rise in serum cholesterol is inhibited by hepatic delivery of adenoviral vector expressing apolipoprotein B mRNA editing enzyme in rabbits," J. Surg. Res. 85:148-157 (1999)). Apolipoprotein B 100 is not essential for life as mice that synthesize exclusively apolipoprotein B48 (apolipoprotein B48-only mice) generated through targeted mutagenesis developed normally, were healthy and fertile. Compared with wild-type mice fed on a chow diet, the level of LDL-cholesterol was lower in apolipoprotein B48-only mice (Farese et al., "Phenotypic analysis of mice expressing exclusively apolipoprotein B48 or apolipoprotein B100," Proc. Natl. Acad. Sci. USA 93:6393-6398 (1996)). However, the induction of apolipoprotein B mRNA editing activity through *apobec-1* gene transfer and tissue-specific overexpression poses a significant challenge in that it has induced hepatocellular dysplasia and carcinoma in transgenic mice and rabbits (Yamanaka et al., "Apolipoprotein B mRNA editing protein induces hepatocellular carcinoma and dysplasia in transgenic animals.," Proc. Natl. Acad. Sci. USA 92: 8483-8487 (1995)); Yamanaka et al., "Hyperediting of multiple cytidines of apolipoprotein B mRNA by APOBEC-1 requires auxiliary protein(s) but not a mooring sequence motif," J. Biol. Chem. 271:11506-11510 (1996)); Yamanaka et al., "A novel translational repressor mRNA is edited extensively in livers containing tumors caused by the transgene expression of the apoB mRNA editing enzyme," Genes & Dev. 11:321-333 (1997)). This was proposed to be due to persistent high levels of APOBEC-1 expression resulting in unregulated and nonspecific mRNA editing (Sowden et al., "Overexpression of APOBEC-1 results in mooring-sequence-dependent promiscuous RNA editing," J. Biol. Chem. 271:3011-3017 (1996)); Yamanaka et al., "A novel translational repressor mRNA is edited extensively in livers containing tumors caused by the transgene expression of the apoB mRNA editing enzyme," Genes & Dev. 11:321-333 (1997)); Sowden et al., "Apolipoprotein B RNA Sequence 3' of the mooring sequence and cellular sources of auxiliary factors determine the location and extent of promiscuous editing," Nucleic Acids Res. 26:1644-1652 (1998)). Adverse effects were not observed in transgenic animals with low to moderate levels of APOBEC-1 expression (Teng et al., "Adenovirus-mediated gene transfer of rat apolipoprotein B mRNA editing protein in mice virtually eliminates apolipoprotein B100 and normal low density lipoprotein production," J. Biol. Chem. 269:29395-29404 (1994)); Qian et al., "Low expression of the apolipoprotein B mRNA editing transgene in mice reduces LDL but does not cause liver dysplasia or tumors," Arteriosc. Thromb. Vasc. Biol. 18:1013-1020 (1998)); Wu et al., "Normal perinatal rise in serum cholesterol is inhibited by hepatic delivery of adenoviral vector expressing apolipoprotein B mRNA editing enzyme in rabbits," J. Sure. Res. 85:148-157 (1999)). Despite the limited success of *apobec-1* gene therapy in modifying apolipoprotein B mRNA editing, such gene therapy poses too great a risk of adverse effects stemming from either persistent elevated levels of APOBEC-1 expression or problems associated with the use of infective transformation vectors (e.g., adenoviral vectors).

For these reasons, it would be desirable to identify an approach to achieve apolipoprotein B mRNA editing, where its induction can be maintained at low levels and importantly, achieved in a transient manner. Moreover, it would be desirable to identify an approach to achieve apolipoprotein B mRNA editing which is substantially free of the side-effects observed with reported gene therapy approaches. The present invention is directed to overcoming these and other deficiencies in the art.

### SUMMARY OF THE INVENTION

A first aspect of the present invention relates to a chimeric protein including: a first polypeptide that includes a protein transduction domain; and a second polypeptide that includes APOBEC-1 or a fragment thereof which can edit mRNA encoding apolipoprotein B.

A second aspect of the present invention relates to a chimeric protein including: a first polypeptide that includes a protein transduction domain; and a second polypeptide that includes APOBEC-1 Complementation Factor ("ACF") or a fragment thereof which can bind to apolipoprotein B mRNA to facilitate editing of the mRNA by APOBEC-1.

Third and fourth aspects of the present invention relate to DNA molecules which encode one of the chimeric proteins of the present invention. DNA constructs, expression vectors, and recombinant host cells including such DNA molecules are also disclosed.

A fifth aspect of the present invention relates to a composition which includes: a pharmaceutically acceptable carrier and a chimeric protein of the present invention.

A sixth aspect of the present invention relates to a composition which includes: a first chimeric protein including a first polypeptide that includes a protein transduction domain and a second polypeptide that includes APOBEC-1 or a fragment thereof which can edit mRNA encoding apolipoprotein B; and a second chimeric protein including a first polypeptide that includes a protein transduction domain and a second polypeptide that includes ACF or a fragment thereof which can bind to apolipoprotein B mRNA to facilitate editing of the mRNA by APOBEC-1 or the fragment thereof.

A seventh aspect of the present invention relates to a delivery device which includes either a chimeric protein of the present invention or a composition of the present invention.

An eighth aspect of the present invention relates to a chimeric protein as defined herein before for use in modifying VLDL-apolipoprotein B mRNA editing in vivo, wherein VLDL-apolipoprotein B mRNA in a cell is contacted with said chimeric protein under conditions effective to increase the concentration of VLDL-apolipoprotein B48 which is secreted by the cell, as compared to the concentration of VLDL-apolipoprotein B100 which is secreted by the cell, relative to an untreated cell.

A ninth aspect of the present invention relates to a chimeric protein as herein before defined for use in reducing serum LDL levels in a patient, wherein said protein is delivered into one or more cells of the patient, without genetically modifying the cells, in an amount which is effective to increase the concentration of VLDL-apolipoprotein B48 that is secreted by one or more of the cells into serum.

A tenth aspect of the present invention relates to a chimeric protein as herein before defined for use in treating or preventing an atherogenic disease or disorder, wherein the protein is administered to a patient and wherein upon said administration the protein is taken up by one or more cells of the patient that can synthesize and secrete VDLL-apoliproprotein B under conditions which are effective to increase the concentration of VLDL apolipoprotein B48 that is secreted by the one or more cells into serum, whereby rapid clearing of VLDL-apolipoprotein B48 from serum decreases the serum concentration of LDL.

An eleventh aspect of the present invention relates to a liposome or niosome which is targeted for uptake by a liver cell, the liposome or niosome containing (i) a chimeric protein comprising a first polypeptide comprising a protein transduction domain; and a second polypeptide comprising APOBEC-1 or a fragment thereof which can edit mRNA encoding apolipoprotein B, (ii) a chimeric protein comprising a first polypeptide comprising a protein transduction domain; and a second polypeptide comprising ACF or a fragment thereof which can bind to apolipoprotein B mRNA to facilitate editing of the mRNA by APOBEC-1, or (iii) a combination thereof. Compositions which include the liposome or niosome are also disclosed.

The present invention demonstrates the efficacy of protein-mediated delivery to increase intracellular APOBEC-1 in cells which produce and secrete VLDL-apolipoprotein B. By increasing the extent of apolipoprotein B mRNA editing *in vivo,* it is possible to modify the ratio of VLDL-apolipoprotein 948 to, VLDL-apolipoprotein B 100 which is secreted by such cells, specifically increasing the relative serum concentration of VLDL-apolipoprotein B48 and decreasing the relative serum concentration of VLDL-apolipoprotein B100. Due to the nature of these complexes, the B48 complex is cleared much more rapidly from serum, minimizing the conversion of VLDL into LDL, a major atherogenic disease factor. By minimizing the amount of VLDL-apolipoprotein B100 and increasing the amount of VLDL-apolipoprotein B48, it is possible to both treat and prevent atherogenic diseases or disorders. Moreover, by using protein delivery, it is possible to avoid the apparently unavoidable side effects of gene therapy. These results presented here open new possibilities for the treatment of hyperlipidemia through the induction of precisely controlled hepatic editing activity.

### BRIEF DESCRIPTION OF THE DRAWINGS

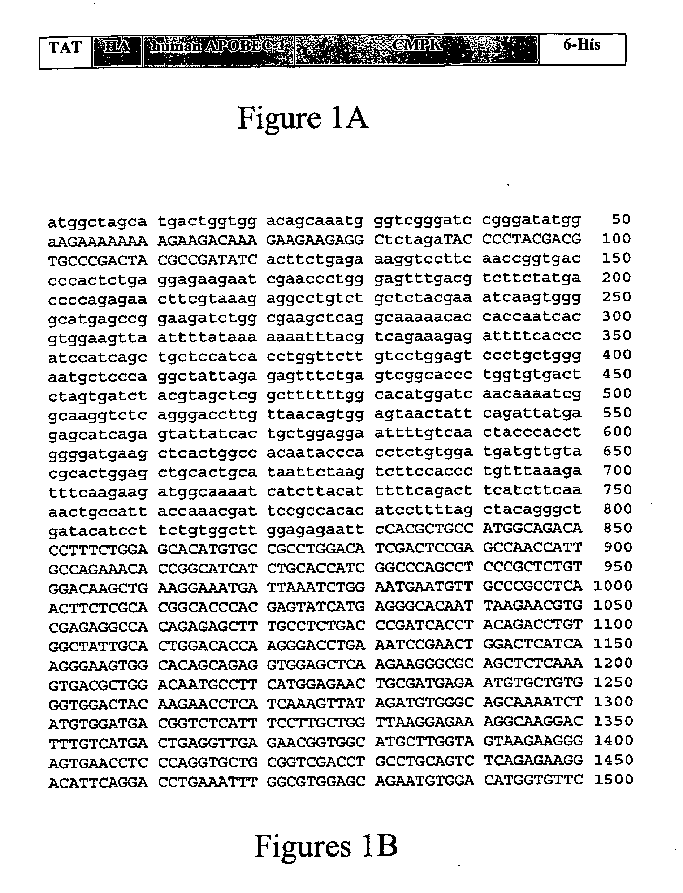

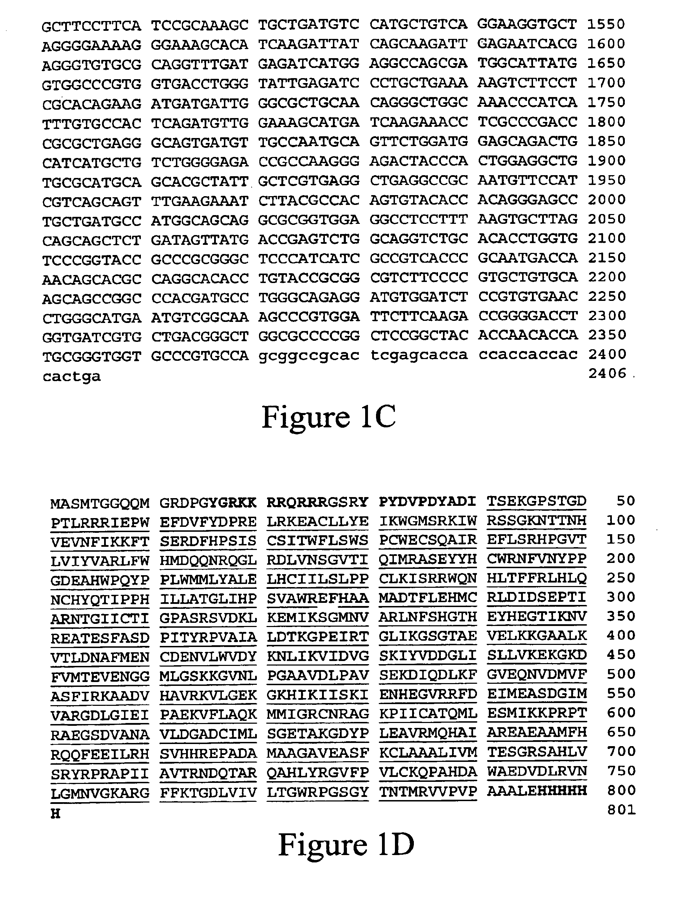

Figures 1A-D illustrate the structure (1A) and both nucleotide (1B-C, SEQ ID No: 1) and amino acid (1D, SEQ ID No: 2) sequences for an exemplary first chimeric protein (designated TAT-hAPOBEC-CMPK) specific for human apolipoprotein B mRNA editing. In Figures 1B-C, the region encoding human APOBEC-1 is shown in lowerease letters and the start codon for this construct is at the beginning of the sequence. The sequences encoding a TAT protein transduction domain and a hemagglutinin domain are shown in uppercase letters near the 5' end (i.e., upstream of the APOBEC-1 sequence). The sequence encoding CMPK is shown 3' of the APOBEC-1 sequence in uppercase letters. At the 3' terminal region and shown in lowercase letters is a sequence encoding a histidine tag. In Figure 1D, beginning from the N-terminal end, the TAT protein transduction domain is shown in bold, followed by the hemagglutinin domain also shown in bold, human APOBEC-1 shown underlined, CMPK also shown underlined, and the histidine tag shown in bold at the C-terminus.

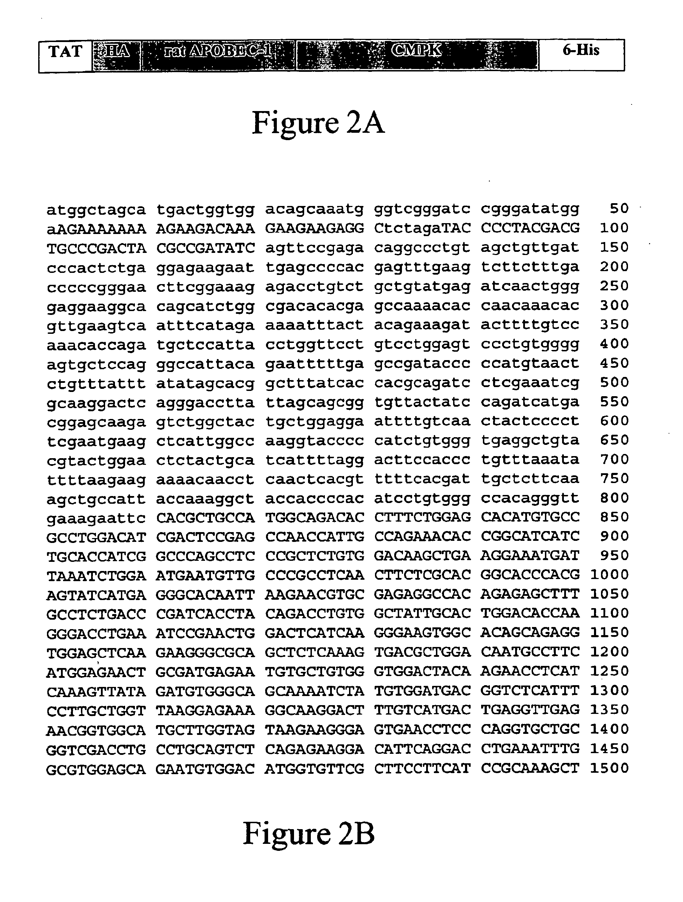

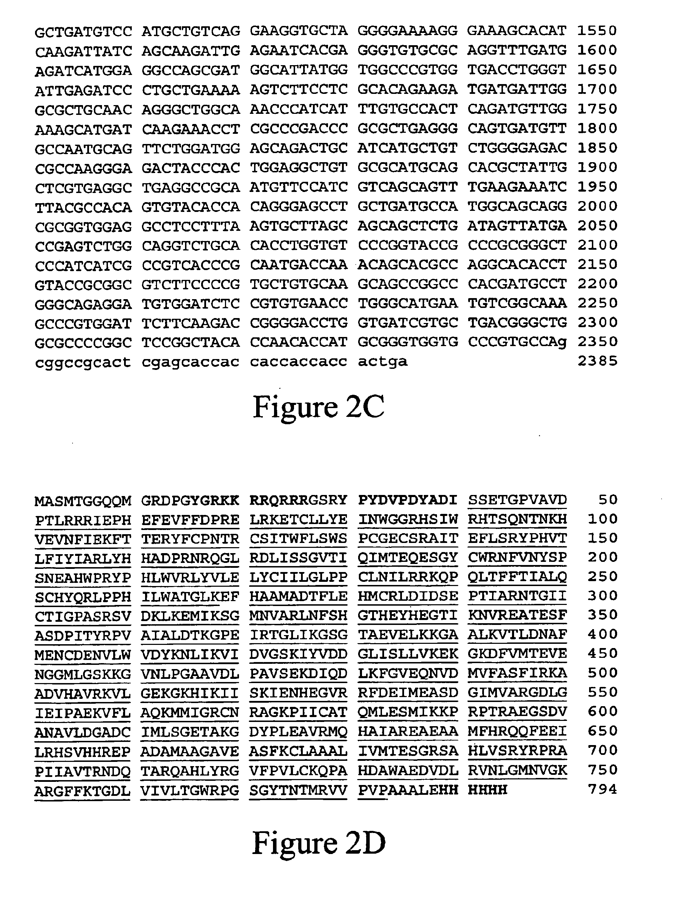

Figures 2A-D illustrate the structure (2A) and both nucleotide (2B-C, SEQ ID No: 3) and amino acid (2D, SEQ ID No: 4) sequences for an exemplary first chimeric protein (designated TAT-rAPOBEC-CMPK) specific for rat apolipoprotein B mRNA editing. In Figures 2B-C, the region encoding rat APOBEC-1 is shown in lowercase letters and the start codon for this construct is at the beginning of the sequence. The sequences encoding a TAT protein transduction domain and a hemagglutinin domain are shown in uppercase letters near the 5' end (i.e., upstream of the APOBEC- sequence). The sequence encoding CMPK is shown 3' of the APOBEC-1 sequence in uppercase letters. At the 3' terminal region and shown in lowercase letters is a sequence encoding a histidine tag. In Figure 2D, beginning from the N-terminal end, the TAT protein transduction domain is shown in bold, followed by the hemagglutinin domain also shown in bold, rat APOBEC-1 shown underlined, CMPK also shown underlined, and the histidine tag shown in bold at the C-terminus.

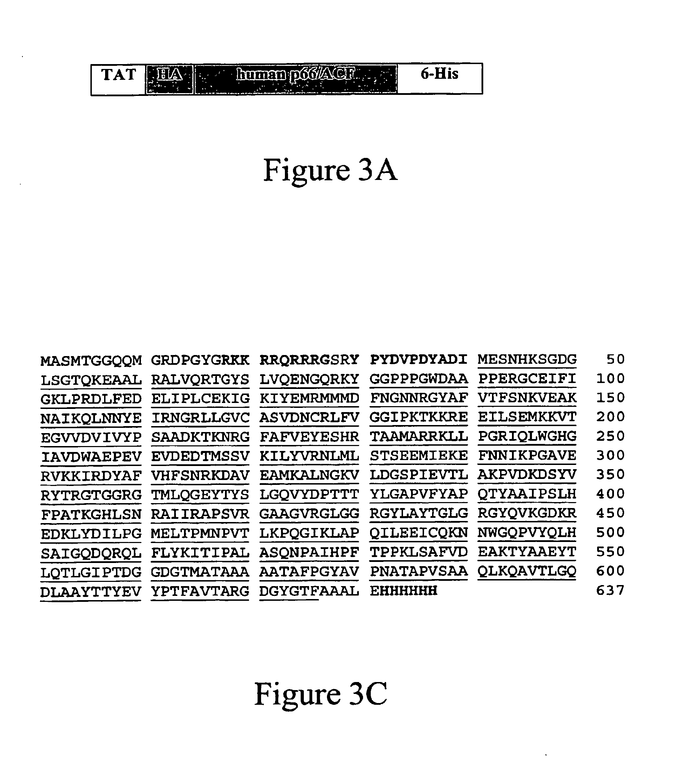

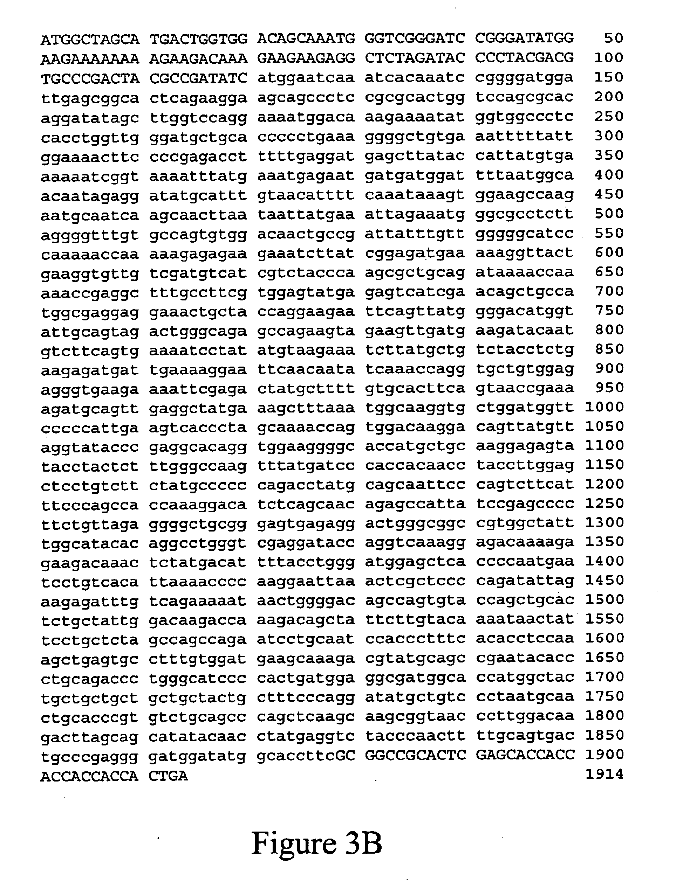

Figures 3A-C illustrate the structure (3A) and both nucleotide (3B, SEQ ID No: 5) and amino acid (3C, SEQ ID No: 6) sequences for an exemplary second chimeric protein (designated TAT-hACF) specific for complementing human APOBEC-1. In Figure 3B, the region encoding human ACF is shown in lowercase letters and the start codon for this construct is at the beginning of the sequence. The sequence encoding a TAT protein transduction domain and a hemagglutinin domain is shown in uppercase letters near the 5' end (i.e., upstream of the ACF sequence). At the 3' terminal region and shown in lowercase letters is a sequence encoding a histidine tag. In Figure 3C, beginning from the N-terminal end, the TAT protein transduction domain is shown in bold, followed by the hemagglutinin domain also shown in bold, human ACF shown underlined, and the histidine tag shown in bold at the C-terminus.

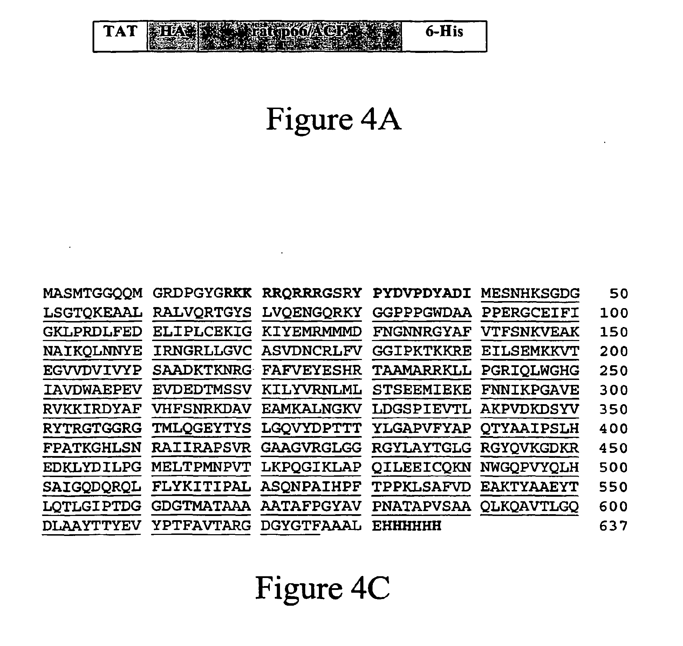

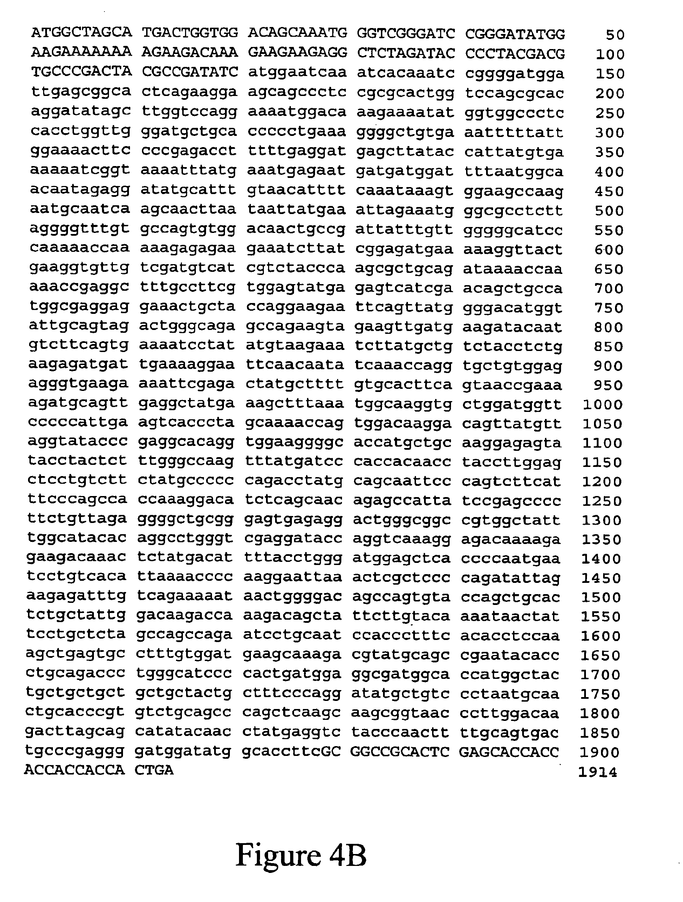

Figures 4A-C illustrate the structure (4A) and both nucleotide (4B, SEQ ID No: 7) and amino acid (4C, SEQ ID No: 8) sequences for an exemplary second chimeric protein (designated TAT-rACF) specific for complementing rat APOBEC-1. In Figure 4B, the region encoding rat ACF is shown in lowercase letters and the start codon for this construct is at the beginning of the sequence. The sequence encoding a TAT protein transduction domain and a hemagglutinin domain is shown in uppercase letters near the 5' end (i.e., upstream of the ACF sequence). At the 3' terminal region and shown in lowercase letters is a sequence encoding a histidine tag. In Figure 4C, beginning from the N-terminal end, the TAT protein transduction domain is shown in bold, followed by the hemagglutinin domain also shown in bold, rat ACF shown underlined, and the histidine tag shown in bold at the C-terminus.

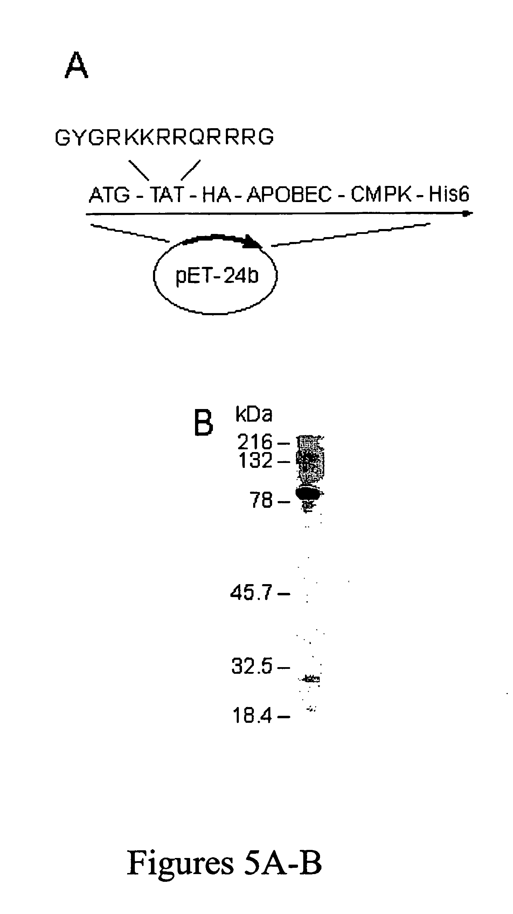

Figures 5A-B illustrate the purification of full-length TAT-rAPOBEC-CMPK protein. In Figure 5A, a schematic image illustrates generally the structure of a prokaryotic expression vector, pET-24b, encoding the TAT fusion protein. Figure 5B illustrates the image of a gel following two-column purification and silver-staining. The TAT fusion protein is the only protein recovered in significant concentrations.

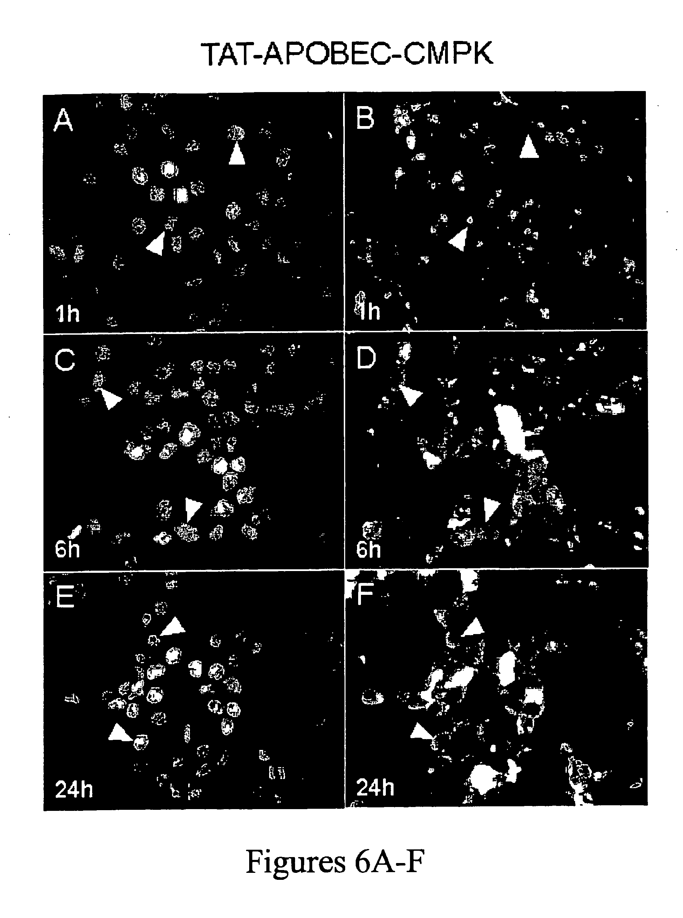

Figures 6A-F are images of immuno-stained cells exposed to the TAT fusion protein TAT-rAPOBEC-CMPK. McArdle cells were treated with 650 nM of recombinant TAT-rAPOBEC-CMPK for the indicated times (1h, 6h, or 24h). Cells were fixed, permeabilized, reacted with antibody to the HA epitope and FITC-conjugated anti-mouse secondary antibody and mounted in DAPI containing buffer as described in the Examples. Arrowheads indicated the position of select nuclei.

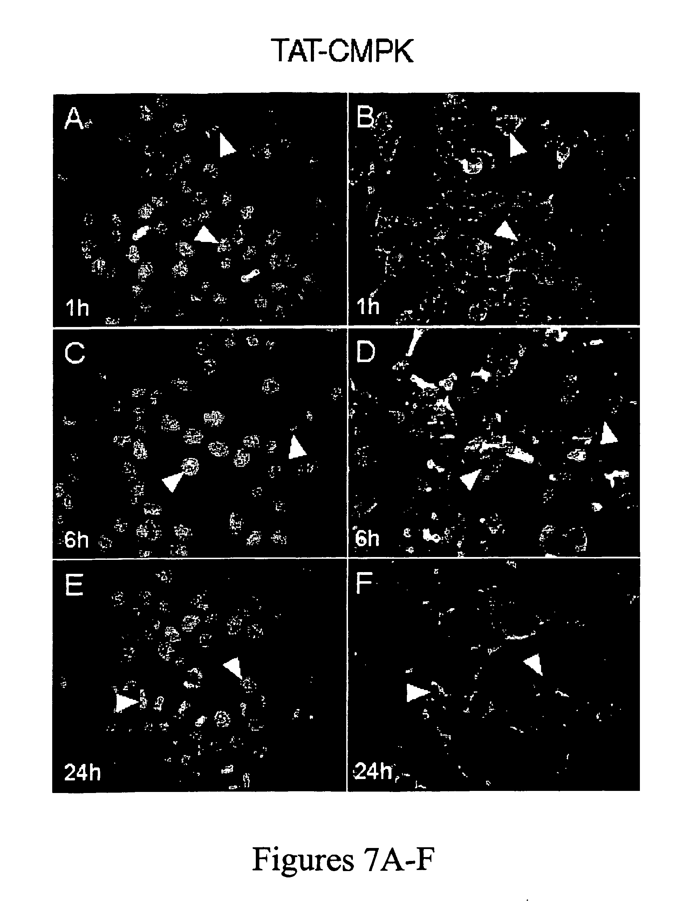

Figures 7A-F are images of immuno-stained cell exposed to TAT-CMPK fusion protein. McArdle cells were treated with 1125 nM of recombinant TAT-CMPK for the indicated times (1h, 6h, or 24h). Cells were fixed, permeabilized, reacted with antibody to the HA epitope and FITC-conjugated anti-mouse secondary antibody and mounted in DAPI containing buffer as described in the Examples. Arrowheads indicated the position of select nuclei.

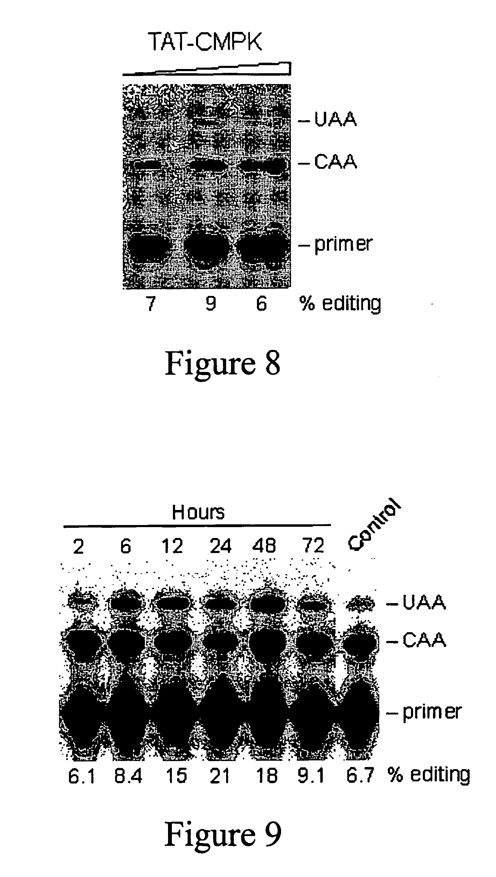

Figure 8 is an image of a gel indicating that TAT-CMPK did not stimulate editing. McArdle cells were treated with 45 nM, 225 nM and 1125 nM of recombinant TAT-CMPK for 24 h. Total cellular RNA was isolated and apolipoprotein B mRNA was selectively amplified by reverse transcription-polymerase chain reaction ("RT-PCR") and the proportion of edited apolipoprotein B RNA determined by poisoned primer extension as described in the Examples. CAA, primer extension product corresponding to unedited RNA; UAA, primer extension product corresponding to edited RNA; P, primer.
Figure 9 is an image of a gel indicating that TAT-rAPOBEC-CMPK increased editing activity in McArdle cells. The TAT fusion protein (360 nM or 62 µg protein/ml media) was added into cell culture media and RNAs were isolated subsequent to treatment from wild type McArdle cells at the indicated time points. Control cells were treated with a corresponding aliquot of buffer B used to dialyze the recombinant protein. The editing efficiency was calculated as described in the Examples. The standard deviations for each of the lanes on the gel, reading left to right, are as follows: 0.9, 2.2, 3.8, 2.1, 1.1, 0.9, 0.2, n=3. CAA, primer extension product corresponding to unedited RNA; UAA, primer extension product corresponding to edited RNA; P, primer.

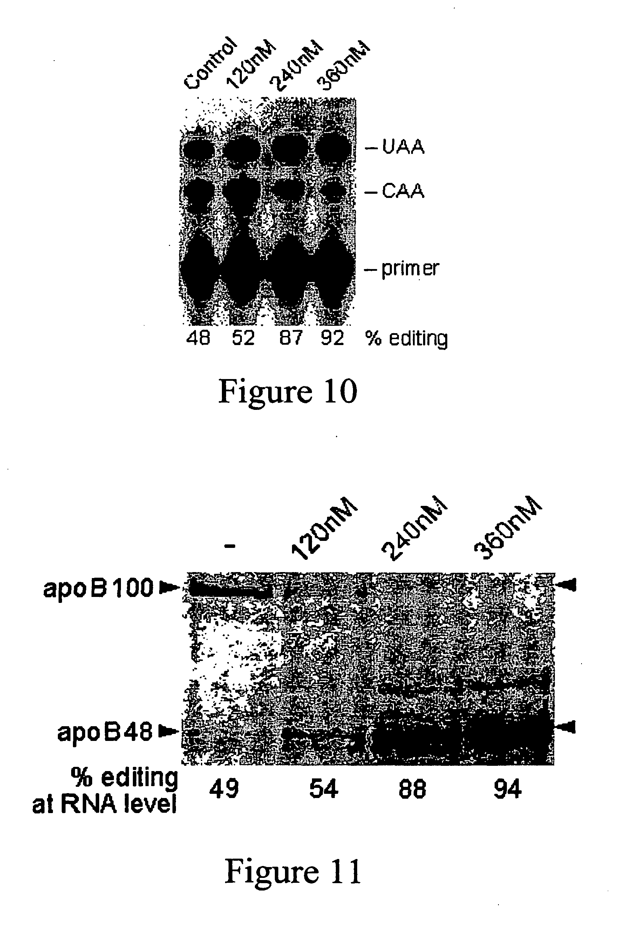

Figure 10 is an image of a gel indicating that TAT fusion protein increased editing activity in primary rat hepatocytes. Hepatocytes were prepared and treated with TAT-rAPOBEC-CMPK as described in the Examples. Control cells were treated with a corresponding aliquot of buffer B used to dialyze the recombinant protein. The increase in editing activity caused by TAT fusion protein was apparent. The standard deviations for each of the lanes on the gel, reading left to right, are as follows: 2.2, 3.6, 2.5, 1.9, n=3.
Figure 11 is an image showing the changes in secreted lipoprotein profile due to TAT-rAPOBEC-CMPK treatment. Primary hepatocytes were treated with TAT fusion protein first, then labeled with [³⁵S]methionine and [³⁵S]cysteine. Control cells (-) were treated with a corresponding aliquot of buffer B used to dialyze the recombinant protein. Cell culture media were collected, apolipoprotein B48 and apolipoprotein B100 were precipitated by anti-apoB antibody and separated by SDS-PAGE. The second band below apolipoprotein B48 might have been due to protein degradation and the band between apolipoprotein B100 and apolipoprotein B48 could be C-3 complement. The editing efficiency of the same cells is shown at the bottom. The results are from a single experiment representative three experiments with similar results.

### DETAILED DESCRIPTION OF THE INVENTION

The present invention relates to protein-mediated approaches for regulating apolipoprotein B mRNA editing and, therefore, regulating the relative concentration of secreted apolipoprotein B derivatives, which offers an approach for controlling the serum levels of atherogenic disease factors such as low density lipoproteins ("LDL") which associates with apolipoprotein B and its derivatives.

According to one aspect of the present invention, a first chimeric protein is provided for such uses. The first chimeric protein includes a first polypeptide that includes a protein transduction domain and a second polypeptide that includes APOBEC-1 or a fragment thereof which can edit mRNA encoding apolipoprotein B.

The first polypeptide can be any protein, or polypeptide fragment thereof, which is suitable for inducing cellular uptake of the chimeric protein.

By way of example, protein transduction domains from several known proteins can be employed, including without limitation, HIV-1 Tat protein, *Drosophila* homeotic transcription factor (ANTP), and HSV-1 VP22 transcription factor (Schwarze et al., "In vivo protein transduction: Intracellular delivery of biologically active proteins, compounds, and DNA," TiPS 21:45-48 (2000).

A preferred protein transduction domain is the protein transduction domain of the human immunodeficiency virus ("HIV") tat protein. An exemplary HIV tat protein transduction domain has an amino acid sequence of SEQ ID No: 9 as follows: This protein transduction domain has also been noted to be a nuclear translocation domain (HIV Sequence Compendium 2000, Kuiken et al. (eds.), Theoretical Biology and Biophysics Group, Los Alamos National Laboratory). One DNA molecule which encodes the HIV tat protein transduction domain has a nucleotide sequence of SEQ ID No: 10 as follows:
agaaaaaaaa gaagacaaag aagaaga 27
Variations of these tat sequences can also be employed. Such sequence variants have been reported in HIV Sequence Compendium 2000, Kuiken et al. (eds.), Theoretical Biology and Biophysics Group, Los Alamos National Laboratory.

Other cellular uptake polypeptides and their use have been described in the literature, including membrane-permeable sequences of the SN50 peptide, the Grb2 SH2 domain, and integrin β₃, β₁, and α_{IIb} cytoplasmic domains (Hawiger, "Noninvasive intracellular delivery of functional peptides and proteins," Curr. Opin. Chem. Biol. 3:89-94 (1999)).

The second polypeptide can be either a full length APOBEC-1 or a fragment thereof which includes the catalytic domain thereof. The APOBEC-1 protein or fragment thereof is a mammalian APOBEC-1 protein or fragment thereof, including without limitation, human, rat, mouse, etc.

The full length human APOBEC-1 has an amino acid sequence according to SEQ ID No: 11 as follows: This human APOBEC-1 sequence is reported at Genbank Accession No. NP_001635. The full length human APOBEC-1 is believed to include a putative bipartite nuclear localization signal between amino acid residues 15-34, a catalytic center between amino acid residues 61-98, and a putative cytoplasmic retention signal between amino acid residues 173-229. A cDNA sequence which encodes the full length human APOBEC-1 is set forth as SEQ ID No: 12 as follows:

The full length rat APOBEC-1 has an amino acid sequence according to SEQ ID No: 13 as follows: This rat APOBEC-1 sequence is reported at Genbank Accession No. P38483. Recombinant studies using rat APOBEC-1 have demonstrated that an N-terminal region, containing the putative nuclear localization signal, is required for nuclear distribution of APOBEC-1 while a C-terminal region, containing a putative cytoplasmic retention signal (Yang et al., "Multiple protein domains determine the cell type-specific nuclear distribution of the catalytic subunit required for apolipoprotein B mRNA editing," Proc. Natl. Acad. Sci. USA 94:13075-13080 (1997)). A cDNA sequence which encodes the full length rat APOBEC-1 is set forth as SEQ ID No: 14 as follows: The cDNA molecule is reported at Genbank Accession No. L07114.

The full length mouse APOBEC-1 has an amino acid sequence according to SEQ ID No: 15 as follows: This mouse APOBEC-1 sequence is reported at Genbank Accession No. NP_112436. A cDNA sequence which encodes the full length mouse APOBEC-1 is set forth as SEQ ID No: 16 as follows: The cDNA molecule is reported at Genbank Accession No. NM_031159.

The first chimeric protein of the present invention can also include one or more other polypeptide sequences, including without limitation: (i) a polypeptide that includes a cytoplasmic localization protein or a fragment thereof which, upon cellular uptake of the first chimeric protein, localizes the first chimeric protein to the cytoplasm; (ii) a polypeptide that includes a plurality of adjacent histidine residues; and (iii) a polypeptide that includes an epitope tag.

The polypeptide that includes a cytoplasmic localization protein or a fragment thereof can be any protein, or fragment thereof, which can effectively retain the first chimeric protein within the cytoplasm of a cell into which the first chimeric protein has been translocated. One such protein is chicken muscle pyruvate kinase ("CMPK"), which has an amino acid sequence of SEQ ID No: 17 as follows: A DNA molecule encoding the full length CMPK has a nucleotide sequence according to SEQ ID No: 18 as follows: The amino acid sequence and nucleotide sequence for the full length CMPK is reported at Genbank Accession Nos. AAA49021 and J00903, respectively.

Fragments of CMPK which afford cytoplasmic retention of the first chimeric protein include, without limitation, polypeptides containing at a minimum residues 1-479 of SEQ ID No: 18.

The polypeptide that includes a plurality of histidine residues preferably contains a sufficient number of histidine residues so as to allow the first chimeric protein containing such histidine residues to be bound by an antibody which recognizes the plurality of histidine residues. One type of DNA molecule encoding Hₙ is (cac)ₙ, where n is greater than 1, but preferably greater than about 5. This His region can be used during immuno-purification, which is described in greater detail below.

The polypeptide that includes an epitope tag can be any epitope tag that is recognized with antibodies raised against the epitope tag. An exemplary epitope tag is a hemagglutinin ("HA") domain. The HA domain is present only when it is desirable to examine, i.e., *in vitro,* localization of the first chimeric protein within cells that have translocated it. One suitable HA domain has an amino acid sequence according to SEQ ID No: 19 as follows: This HA sequence is encoded by a DNA molecule having a nucleotide sequence according to SEQ ID No: 20 as follows:
tacccctacg acgtgcccga ctacgcc 27

An exemplary first chimeric protein of the present invention which is suitable for use in humans, designated TAT-hAPOBEC-CMPK, is set forth in Figure 1A. This first chimeric protein (human) includes: an N-terminal HIV tat protein transduction domain, a hemagglutinin domain, a polypeptide fragment of human APOBEC-1, a CMPK domain, and a C-terminal His tag. The amino acid sequence (SEQ ID No: 2) and encoding nucleotide sequence (SEQ ID No: 1) of this exemplary first chimeric protein (human) is set forth in Figures 1D and 1B-C, respectively.

An exemplary first chimeric protein of the present invention which is suitable for use in rats, designated TAT-rAPOBEC-CMPK, is set forth in Figure 2A. This first chimeric protein (rat) includes: an N-terminal HIV tat protein transduction domain, a hemagglutinin domain, a polypeptide fragment of rat APOBEC-1, a CMPK domain, and a C-terminal His tag. The amino acid sequence (SEQ ID No: 4) and encoding nucleotide sequence (SEQ ID No: 3) of this exemplary first chimeric protein (rat) is set forth in Figures 2D and 2B-C, respectively.

According to a second aspect of the present invention, a second chimeric protein is provided for use in combination with the first chimeric protein described above. The second chimeric protein includes a first polypeptide that includes a protein transduction domain and a second polypeptide the includes ACF or a fragment thereof which can bind to apolipoprotein B mRNA.

The first polypeptide of the second chimeric protein can be a protein transduction domain of the type described above The protein transduction domain of the second chimeric protein can be the same or different from the protein transduction domain of the first chimeric protein.

The second polypeptide of the second chimeric protein, as noted above, includes ACF or a fragment thereof which can bind to apolipoprotein B mRNA. Although it has been proposed that a number of different proteins assist APOBEC-1 in editing apolipoprotein B mRNA, ACF has been identified as the minimal protein complement for editing *in vitro* in the human system (Mehta et al., Molecular cloning of apobec-1 complementation factor, a novel RNA binding protein involved in the editing of apo B mRNA," Mol. Cell. Biol. 20:1846-1854 (2000). In accordance with the present invention, therefore, the second chimeric protein binds apolipoprotein B mRNA at the mooring sequence and through its interactions with the first chimeric protein, sequesters the first chimeric protein to the cytidine of the apolipoprotein B mRNA to be edited (i.e., at position 6666), thereby resulting in its conversion to a uridine. As noted above, this conversion results in a stop codon that contributes to expression of the apolipoprotein B48 derivative.

Recent studies have suggested that APOBEC-1 requires a chaperone for its nuclear localization (Yang et al., "Intracellular trafficking determinants in APOBEC-1, the catalytic subunit for cytidine to uridine editing of apolipoprotein B mRNA," Exp. Cell Res. 267:153-164 (2001). More recently, however, it has been learned that APOBEC-1 is most likely associated with ACF throughout the cell and, therefore, it may import to the nucleus as an APOBEC-1/ACF complex. A bipartite nuclear localization signal is predicted in ACF (see below).

ACF is expressed at sufficient levels within the hepatic cells of rat (Dance et al., "Two proteins essential for apolipoprotein B mRNA editing are expressed from a single gene through alternative splicing," J. Biol. Chem., electronically published as manuscript M 111337200 (2002), such that augmenting of the intracellular ACF concentration is not needed. However, to optimize apolipoprotein B mRNA editing, in some instances it may be desirable to increase the intracellular concentration of ACF.

The full length rat ACF has an amino acid sequence according to SEQ ID No: 21 as follows: A DNA molecule encoding the full length rat ACF has a nucleotide sequence according to SEQ ID No: 22 as follows: The amino acid sequence and nucleotide sequence for the full length rat ACF65 is reported at Genbank Accession Nos. AAK50145 and AY028945, respectively. In addition, it should be noted that a short isoform of rat ACF64 exists, as identified at Genbank Accession No. AF290984.

The full length human ACF has an amino acid sequence according to SEQ ID No: 23 as follows: A DNA molecule encoding the full length human ACF has a nucleotide sequence according to SEQ ID No: 24 as follows: The amino acid sequence and nucleotide sequence for the full length human ACF is reported at Genbank Accession Nos. AAF76221 and AF271789, respectively.

In comparing the human and rat ACF homologs, it is apparent that these proteins share 93.5 percent identity at the amino acid level and, moreover, antibodies raised against the human ACF also recognize rat ACF. Is has been reported that functional complementation of apolipoprotein B mRNA editing by APOBEC-1 involves the N-terminal 380 residues of ACF (Blanc et al., "Mutagenesis of Apobec-1 complementation factor reveals distinct domains that modulate RNA binding, protein-protein interaction with Apobec-1, and complementation of C to U RNA-editing activity," J. Biol. Chem. 276(49): 46386-46393 (2001).

The second chimeric protein of the present invention can also include one or more other polypeptide sequences, including without limitation: (i) a polypeptide that includes a cytoplasmic localization protein or a fragment thereof which, upon cellular uptake of the second chimeric protein, localizes the second chimeric protein to the cytoplasm; (ii) a polypeptide that includes a plurality of adjacent histidine residues; and (iii) a polypeptide that includes a hemagglutinin domain. Each of these has been described above with respect to the first chimeric protein.

An exemplary second chimeric protein of the present invention which is suitable for use in humans, designated TAT-hACF, is set forth in Figure 3A. This second chimeric protein (human) includes: an N-terminal HIV tat protein transduction domain, a hemagglutinin domain, a polypeptide fragment of human ACF, and a C-terminal His tag. The amino acid sequence (SEQ ID No: 6) and encoding nucleotide sequence (SEQ ID No: 5) of this exemplary second chimeric protein (human) is set forth in Figures 3B-C.

An exemplary second chimeric protein of the present invention which is suitable for use in rats, designated TAT-rACF, is set forth in Figure 4A. This second chimeric protein (rat) includes: an N-terminal HIV tat protein transduction domain, a hemagglutinin domain, a polypeptide fragment of rat ACF, and a C-terminal His tag. The amino acid sequence (SEQ ID No: 8) and encoding nucleotide sequence (SEQ ID No: 7) of this exemplary second chimeric protein (rat) is set forth in Figures 4B-C.

DNA molecules encoding the above-identified first and second chimeric proteins can be assembled using conventional molecular genetic manipulation for subcloning gene fragments, such as described by Sambrook et al., Molecular Cloning: A Laboratory Manual, Cold Springs Laboratory, Cold Springs Harbor, New York (1989), and Ausubel et al. (ed.), Current Protocols in Molecular Biology, John Wiley & Sons (New York, NY) (1999 and preceding editions). In conjunction therewith, desired fragments of the APOBEC-1, ACF, or CMPK encoding DNA molecules can be obtained using the PCR technique together with specific sets of primers chosen to represent particular portions of the protein. Erlich et al., Science 252:1643-51 (1991).

Once the desired DNA molecules have been assembled, DNA constructs can be assembled by ligating together the DNA molecule encoding the first or second chimeric protein with appropriate regulatory sequences including, without limitation, a promoter sequence operably connected 5' to the DNA molecule, a 3' regulatory sequence operably connected 3' of the DNA molecule, as well as any enhancer elements, suppressor elements, etc. The DNA construct can then be inserted into an appropriate expression vector. Thereafter, the vector can be used to transform a host cell, typically although not exclusively a prokaryote, and the recombinant host cell can express the first or second chimeric protein of the present invention.

When a prokaryotic host cell is selected for subsequent transformation, the promoter region used to construct the DNA construct (i.e., transgene) should be appropriate for the particular host. The DNA sequences of eukaryotic promoters, as described *infra* for expression in eukaryotic host cells, differ from those of prokaryotic promoters. Eukaryotic promoters and accompanying genetic signals may not be recognized in or may not function in a prokaryotic system and, further, prokaryotic promoters are not recognized and do not function in eukaryotic cells.

Similarly, translation of mRNA in prokaryotes depends upon the presence of the proper prokaryotic signals which differ from those of eukaryotes. Efficient translation of mRNA in prokaryotes requires a ribosome binding site called the Shine-Dalgarno ("SD") sequence on the mRNA. This sequence is a short nucleotide sequence of mRNA that is located before the start codon, usually AUG, which encodes the amino-terminal methionine of the protein. The SD sequences are complementary to the 3'-end of the 16S rRNA (ribosomal RNA) and probably promote binding of mRNA to ribosomes by duplexing with the rRNA to allow correct positioning of the ribosome. For a review on maximizing gene expression, see Roberts and Lauer, Methods in Enzymology, 68:473 (1979).

Promoters vary in their "strength" (i.e., their ability to promote transcription). For the purposes of expressing a cloned gene, it is desirable to use strong promoters in order to obtain a high level of transcription and, hence, expression of the gene. Depending upon the host cell system utilized, any one of a number of suitable promoters may be used. For instance, when cloning in *E. coli,* its bacteriophages, or plasmids, promoters such as the T7 phage promoter, *lac* promoter, *trp* promoter, *rec*A promoter, ribosomal RNA promoter, the P_{R} and P_{L} promoters of coliphage lambda and others, including but not limited, to *lac*UV5, *omp*F*, bla, lpp,* and the like, may be used to direct high levels of transcription of adjacent DNA segments. Additionally, a hybrid *trp-lac*UV5 *(tac)* promoter or other *E. coli* promoters produced by recombinant DNA or other synthetic DNA techniques may be used to provide for transcription of the inserted gene.

Bacterial host cell strains and expression vectors may be chosen which inhibit the action of the promoter unless specifically induced. In certain operons, the addition of specific inducers is necessary for efficient transcription of the inserted DNA. For example, the lac operon is induced by the addition of lactose or IPTG (isopropylthio-beta-D-galactoside). A variety of other operons, such as *trp, pro,* etc., are under different controls.

Specific initiation signals are also required for efficient gene transcription and translation in prokaryotic cells. These transcription and translation initiation signals may vary in "strength" as measured by the quantity of gene specific messenger RNA and protein synthesized, respectively. The DNA expression vector, which contains a promoter, may also contain any combination of various "strong" transcription and/or translation initiation signals. For instance, efficient translation in *E. coli* requires a Shine-Dalgarno ("SD") sequence about 7-9 bases 5' to the initiation codon ("ATG") to provide a ribosome binding site. Thus, any SD-ATG combination that can be utilized by host cell ribosomes may be employed. Such combinations include, but are not limited to, the SD-ATG combination from the *cro* gene or the *N* gene of coliphage lambda, or from the *E. coli* tryptophan E, D, C, B or A genes. Additionally, any SD-ATG combination produced by recombinant DNA or other techniques involving incorporation of synthetic nucleotides may be used.

Mammalian cells can also be used to recombinantly produce the first or second chimeric proteins of the present invention. Suitable mammalian host cells include, without limitation: COS (e.g., ATCC No. CRL 1650 or 1651), BHK (e.g., ATCC No. CRL 6281), CHO (ATCC No. CCL 61), HeLa (e.g., ATCC No. CCL 2), 293 (ATCC No. 1573), CHOP, and NS-1 cells. Suitable expression vectors for directing expression in mammalian cells generally include a promoter, as well as other transcription and translation control sequences known in the art. Common promoters include, without limitation, SV40, MMTV, metallothionein-1, adenovirus Ela, CMV, immediate early, immunoglobulin heavy chain promoter and enhancer, and RSV-LTR.

Regardless of the selection of host cell, once the DNA molecule coding for a first or second chimeric protein has been ligated to its appropriate regulatory regions using well known molecular cloning techniques, it can then be introduced into a suitable vector or otherwise introduced directly into a host cell using transformation protocols well known in the art (Sambrook et al., Molecular Cloning: A Laboratory Manual, Second Edition, Cold Spring Harbor Press, NY (1989).

The recombinant DNA molecule can be introduced into host cells via transformation, particularly transduction, conjugation, mobilization, or electroporation. Suitable host cells include, but are not limited to, bacteria, virus, yeast, mammalian cells, insect, plant, and the like. The host cells, when grown in an appropriate medium, are capable of expressing the chimeric protein, which can then be isolated therefrom and, if necessary, purified. The first or second chimeric protein is preferably produced in purified form (preferably at least about 80%, more preferably 90%, pure) by conventional techniques, including immuno-purification techniques. Immuno-isolation followed by metal-chelating affinity chromatography and cationic exchange chromatography is described in Example 1 *infra.*

A further aspect of the present invention relates to a number of compositions, preferably pharmaceutical compositions, which include the first and/or second chimeric protein of the present invention.

According to one embodiment, a composition includes a pharmaceutically acceptable carrier and the first chimeric protein of the present invention. The first chimeric protein is preferably present in an amount which is effective to modify apolipoprotein B mRNA editing in cells which uptake the first chimeric protein.

According to a second embodiment, a composition includes the first and second chimeric proteins of the present invention. This composition can also include a pharmaceutically acceptable carrier in which the first and second chimeric proteins are dispersed. Preferably, the first chimeric protein is present in an amount which is effective to modify apolipoprotein B mRNA editing in cells which uptake the first chimeric protein and the second chimeric protein is present in an amount which is effective to bind apolipoprotein B mRNA and assist the first chimeric protein in modifying apolipoprotein B mRNA in cells which uptake the first and second chimeric proteins.

The compositions of the present invention can also include suitable excipients, or stabilizers, and can be in solid or liquid form such as, tablets, capsules, powders, solutions, suspensions, or emulsions. Typically, the compositions will contain from about 0.01 to 99 percent, preferably from about 20 to 75 percent of the chimeric protein(s), together with the carrier, excipient, stabilizer, etc.

The solid unit dosage forms can be of the conventional type. The solid form can be a capsule, such as an ordinary gelatin type containing the first and/or second chimeric protein(s) of the present invention and a carrier, for example, lubricants and inert fillers such as, lactose, sucrose, or cornstarch. In another embodiment, these first and/or second chimeric protein(s) are tableted with conventional tablet bases such as lactose, sucrose, or cornstarch in combination with binders like acacia, cornstarch, or gelatin, disintegrating agents, such as cornstarch, potato starch, or alginic acid, and a lubricant, like stearic acid or magnesium stearate.

The first and/or second chimeric protein(s) of the present invention may also be administered in injectable or topically-applied dosages by solution or suspension of these materials in a physiologically acceptable diluent with a pharmaceutical carrier. Such carriers include sterile liquids, such as water and oils, with or without the addition of a surfactant and other pharmaceutically and physiologically acceptable carrier, including adjuvants, excipients or stabilizers. Illustrative oils are those of petroleum, animal, vegetable, or synthetic origin, for example, peanut oil, soybean oil, or mineral oil. In general, water, saline, aqueous dextrose and related sugar solution, and glycols, such as propylene glycol or polyethylene glycol, are preferred liquid carriers, particularly for injectable solutions.

For use as aerosols, the first and/or second chimeric protein(s) of the present invention in solution or suspension may be packaged in a pressurized aerosol container together with suitable propellants, for example, hydrocarbon propellants like propane, butane, or isobutane with conventional adjuvants. The compositions of the present invention also may be administered in a non-pressurized form such as in a nebulizer or atomizer.

Depending upon the treatment being effected, the compounds of the present invention can be administered orally, topically, transdermally, parenterally, subcutaneously, intravenously, intramuscularly, intraperitoneally, by intracavitary or intravesical instillation, intraocularly, intraarterially, intralesionally, or by application to mucous membranes, such as, that of the nose, throat, and bronchial tubes. In most instances, subcutaneous, intravenous, intramuscular, intraperitoneal, and intraarterial routes are preferred.

Compositions within the scope of this invention include all compositions wherein the first and/or second chimeric proteins of the present invention is contained in an amount effective to achieve its intended purpose, noted above. While individual needs vary, determination of optimal ranges of effective amounts of each of the first and second chimeric proteins is within the skill of the art. Typical dosages comprise about 0.01 to about 100 mg/kg-body wt. The preferred dosages comprise about 0.1 to about 100 mg/kg body wt. The most preferred dosages comprise about 1 to about 100 mg/kg body wt.

The amounts of the first and second chimeric proteins can be determined by one of ordinary skill in the art using routine testing to optimize the dosage levels of the first and second chimeric proteins in accordance with the desired degree of apolipoprotein B mRNA editing. Based on May 2001 guidelines by the National Institutes of Health's National Cholesterol Education Program (NCEP), individuals at low risk for a heart attack should have LDL levels under 160 mg/dL, while those at highest risk should aim for LDLs under 100 mg/dL. Treatment regimen for the administration of the first and/or second chimeric proteins of the present invention can also be determined readily by those with ordinary skill in art.

Typically, the first and/or second chimeric proteins (or compositions which contain one or both of the chimeric proteins of the present invention) can be administered via a drug delivery device which includes a chimeric protein or a composition of the present invention. Exemplary delivery devices include, without limitation, liposomes, niosomes, transdermal patches, implants, and syringes.

Liposomes are vesicles comprised of one or more concentrically ordered lipid bilayers which encapsulate an aqueous phase. They are normally not leaky, but can become leaky if a hole or pore occurs in the membrane, if the membrane is dissolved or degrades, or if the membrane temperature is increased to the phase transition temperature. Current methods of drug delivery via liposomes require that the liposome carrier ultimately become permeable and release the encapsulated drug at the target site. This can be accomplished, for example, in a passive manner wherein the liposome bilayer degrades over time through the action of various agents in the body. Every liposome composition will have a characteristic half-life in the circulation or at other sites in the body and, thus, by controlling the half-life of the liposome composition, the rate at which the bilayer degrades can be somewhat regulated.

In contrast to passive drug release, active drug release involves using an agent to induce a permeability change in the liposome vesicle. Liposome membranes can be constructed so that they become destabilized when the environment becomes acidic near the liposome membrane (see, e.g., Proc. Natl. Acad. Sci. USA 84:7851 (1987); Biochemistry 28: 908 (1989). When liposomes are endocytosed by a target cell, for example, they can be routed to acidic endosomes which will destabilize the liposome and result in drug release.

Alternatively, the liposome membrane can be chemically modified such that an enzyme is placed as a coating on the membrane which slowly destabilizes the liposome. Since control of drug release depends on the concentration of enzyme initially placed in the membrane, there is no real effective way to modulate or alter drug release to achieve "on demand" drug delivery. The same problem exists for pH-sensitive liposomes in that as soon as the liposome vesicle comes into contact with a target cell, it will be engulfed and a drop in pH will lead to drug release.

This liposome delivery system can also be made to accumulate at a target organ, tissue, or cell via active targeting. In accordance with the present invention, liposomes can be targeted to liver cells by incorporating into the liposome bilayer a molecule which target hepatocyte receptors. One such molecule is the asialoglycoprotein asialofetuin, which targets the asialoglycoprotein receptor of hepatocytes. The incorporation of asialofetuin into the liposome bilayer can be performed according to the procedures set forth in Wu et al., "Increased liver uptake of liposomes and improved targeting efficacy by labeling with asialofetuin in rodents," Hepatology 27(3):772-778 (1998).

Niosomes are vesicles formed by amphiphilic materials. Non-ionic surfactants were the first materials studied (Iga et al., "Membrane modification by negatively charged stearylpolyoxyethylene derivatives for thermosensitive liposomes: Reduced liposomal aggregation and avoidance of reticuloendothelial system uptake," J. Drug Target 2:259-67 (1994)). and a large number of surfactants have since been found to self assemble into closed bilayer vesicles (Ahl et al., "Enhancement of the in vivo circulation lifetime of L-alpha-distearoylphosphatidylcholine liposomes: Importance of liposomal aggregation versus complement opsonization," Biochim Biophys Acta 1329:370-82 (1997)). These niosomal materials may be used for delivery of the first or second chimeric protein or for delivery of APOBEC-1 or fragments thereof alone or in combination with ACF or fragments thereof.

For example, 200nm doxorubicin niosomes with a polyoxyethylene (molecular weight 1,000) surface have been shown to be rapidly taken up by the liver (Uchegbu et al., "Distribution, metabolism and tumoricidal activity of doxorubicin administered in sorbitan monostearate (Span 60) niosomes in the mouse," Pharm. Res. 12:1019-24 (1995)), allowing polymeric drug conjugates to be formed for delivery of the drug (see Duncan, "Drug polymer conjugates - potential for improved chemotherapy," Anti-Cancer Drugs 3:175-210 (1992)). These techniques can be readily adapted for delivery of the first and second chimeric proteins

Compositions including the liposomes or niosomes in a pharmaceutically acceptable carrier are also contemplated.

Transdermal delivery devices have been employed for delivery of low molecular weight proteins by using lipid-based compositions (i.e., in the form of a patch) in combination with sonophoresis. However, as reported in U.S. Patent No. 6,041,253 to Ellinwood, Jr. et al., transdermal delivery can be further enhanced by the application of an electric field, for example, by iontophoresis or electroporation. Using low frequency ultrasound which induces cavitation of the lipid layers of the stratum corneum, higher transdermal fluxes, rapid control of transdermal fluxes, and drug delivery at lower ultrasound intensities can be achieved. Still further enhancement can be obtained using a combination of chemical enhancers and/or magnetic field along with the electric field and ultrasound.

Implantable or injectable protein depot compositions can also be employed, providing long-term delivery of, e.g., the first and second chimeric proteins. For example, U.S. Patent No. 6,331,31 to Brodbeck et al., reports an injectable depot gel composition which includes a biocompatible polymer, a solvent that dissolves the polymer and forms a viscous gel, and an emulsifying agent in the form of a dispersed droplet phase in the viscous gel. Upon injection, such a gel composition can provide a relatively continuous rate of dispersion of the agent to be delivered, thereby avoiding an initial burst of the agent to be delivered.

Other suitable protein delivery system which are known to those of skill in the art can also be employed to achieve the desired delivery and, thus, modification in the editing of apolipoprotein B mRNA and its concomitant effects.

By virtue of the first chimeric protein being able to edit apolipoprotein B mRNA, the present invention affords the first chimeric protein for use in modifying apolipoprotein BmRNA editing *in vivo.* This aspect of the present invention can be carried out by contacting apolipoprotein B mRNA in a cell with the first chimeric protein of the present invention under conditions effective to increase the concentration of apolipoprotein B48 which is secreted by the cell as compared to the concentration of apolipoprotein B100 which is secreted by the cell, relative to an untreated cell (i.e., which has not taken up the first chimeric protein). Basically, the contacting is carried out by exposing the cell to the first chimeric protein under conditions effective to induce cellular uptake of the first chimeric protein. Because the first chimeric protein includes the first polypeptide (i.e., which includes a protein transduction domain), the first chimeric protein is taken up by the cell. In addition, the same cell can also be contacted with the second chimeric protein of the present invention, causing the second chimeric protein also to be taken up by the cell. As a result, the apolipoprotein B mRNA in the cell is contacted by the second chimeric protein, binding the apolipoprotein mRNA (as described above) so as to facilitate editing thereof by the first chimeric protein. The cell in which the apolipoprotein B mRNA editing is modified can be any cell which can synthesize and secrete VLDL with apolipoprotein B or its derivatives. Exemplary cells of this type include liver cells and intestinal cells, although preferably liver cells. The cell can also be in a mammal, preferably a human.

Likewise, the present invention also affords a chimeric protein as herein before described for use in of reducing serum LDL levels. This aspect of the present invention can be carried out by delivering into one or more cells of a patient, without genetically modifying the cells, an amount of a chimeric protein comprising a first polypeptide comprising a protein transduction domain and a second polypeptide comprising APOBEC-1 or a fragment thereof which can edit mRNA encoding apolipoprotein B, which amount is effective to increase the concentration of VLDL-apolipoprotein B48 that is secreted by the one or more cells into serum and, consequently, reduce the serum concentration of LDL. In accordance with this aspect of the present invention, the patient is a mammal, preferably a human, and the one or more cells are preferably liver cells, intestinal cells, or a combination thereof.

To sustain the reduced serum LDL levels, delivery of the chimeric protein into the one or more cells is preferably repeated periodically (i.e., following a delay of from about 1 to about 7 days).

Delivery of the chimeric protein into the one or more cells can be carried out by exposing the one or more cells to the protein under conditions effective to cause cellular uptake of the protein. Preferably, the protein transduction domain induces cellular uptake by the one or more cells. In addition to delivering the first chimeric protein, a second chimeric protein can also be delivered simultaneously into the one or more cells of the patient, without genetically modifying the cells, where the second chimeric protein comprises a first polypeptide comprising a protein transduction domain and a second polypeptide comprising APOBEC-1 Complementation Factor (AFC) or a fragment thereof which can bind to apolipoprotien B mRNA to facilitate editing of the mRNA by APOBEC-1. Preferably, the protein transduction domain induces cellular uptake by the one or more cells.

APOBEC-1 can be delivered directly into one or more liver cells by contacting each of them with liposomes including a molecule which binds to a hepatocyte receptor (e.g., asialofetuin), thereby inducing uptake of the liposomes and degradation thereof intracellularly to empty their contents into the one or more liver cells. In addition, ACF or a fragment thereof which can bind to apolipoprotein B mRNA can also be delivered via the liposomes.

By increasing the ratio of apolipoprotein B48 to apolipoprotein B100 which is secreted by the one or more cells, the present invention also relates to a chimeric protein as herein before described for use in treating or preventing an atherogenic disease or disorder. This aspect of the present invention can be carried out by administering to a patient an effective amount of a first chimeric protein as herein before described, wherein upon said administering the protein is taken up by one or more cells of the patient that can synthesize and secrete VLDL-apolipoprotein under conditions which are effective to increase the concentration of VLDL-apolipoprotein B48 that is secreted by the one or more cells into serum, whereby rapid clearing of VLDL-apolipoprotein B48 from serum decreases the serum concentration of LDL, to treat or prevent the atherogenic disease or disorder. In accordance with this aspect of the present invention, the patient is a mammal, preferably a human, and the one or more cells are preferably liver cells.

Administration of the protein can be carried out according to any of the above identified approaches. Continued preventative or therapeutic treatment can be effected by repeatedly administering the first chimeric protein periodically (i.e., following a delay of from about 1 to about 7 days).

Preferably, the protein transduction domain induces cellular uptake by the one or more cells. As with the above-described methods, a second chimeric protein as herein before described can also be delivered simultaneously. Preferably, the protein transduction domain induces cellular uptake by the one or more cells.

Using a liposome delivery vehicle, APOBEC-1 and optionally ACF can be delivered directly into one or more liver cells by contacting each of them with a liposome including a molecule which binds to a hepatocyte receptor, thereby inducing uptake of the liposomes and degradation thereof intracellularly to empty their contents into the one or more liver cells.

### EXAMPLES

The following examples are intended to illustrate, but by no means are intended to limit, the scope of the present invention as set forth in the appended claims.

### Example 1 - Generation of TAT Fusion Protein

The induction of hepatic apolipoprotein B mRNA editing was sought through TAT mediated APOBEC-1 protein transduction into liver cells. It has been shown that linking an 11-amino-acid protein transduction domain (PTD) of HIV-1 TAT protein to heterologous protein conferred the ability to transduce into cells (Nagahara et al., "Transduction of full-length TAT fusion proteins into mammalian cells: TAT-p27Kip1 induces cell migration," Nature Med. 4:1449-1452 (1998); Schwarze et al., "In vivo protein transduction: delivery of a biologically active protein into the mouse," Science 285:1569-1572 (1999); Vocero-Akbani et al., "Killing HIV-infected cells by transduction with an HIV protease-activated caspase-3 protein," Nature Med. 5:29-33 (1999)). PTD-linked protein transduced into ~ 100% of cells and the transduction process occurred in a rapid and concentrationdependent but receptor- and transporter-independent manner (Schwarze et al., "Protein transduction: unrestricted delivery into all cells," Trends Cell Biol. 10:290-295 (2000). Liver cells have been shown to be susceptible to transduction (Nagahara et al., "Transduction of full-length TAT fusion proteins into mammalian cells: TAT-p27Kip1 induces cell migration," Nature Med. 4:1449-1452 (1998)). In order to produce in-frame TAT fusion protein from *E*. *coli,* a prokaryotic expression vector was constructed that has an N-terminal PTD flanked by glycine residues for free bond rotation of the domain (Schwarze et al., "In vivo protein transduction: delivery of a biologically active protein into the mouse," Science 285:1569-1572 (1999)). an hemagglutinin (HA) tag and a C-terminal 6-histidine tag. Using this vector as a backbone, a plasmid was constructed to encode full-length TAT-rAPOBEC-CMPK protein, SEQ ID No: 4 (Figures 2A, 2D, and 5A). APOBEC-1 conjugated to CMPK was used in this study because it showed a less robust editing activity *in vitro* and targeted primarily cytoplasmic mRNAs (Yang et al., "Induction of cytidine to uridine editing on cytoplasmic apolipoprotein B mRNA by overexpressing APOBEC-1," J. Biol. Chem. 275:22663-22669 (2000). *In vitro* studies demonstrated that APOBEC-1 retained catalytic activity when conjugated to various lengths of nonspecific proteins (Siddiqui et al., "Disproportionate relationship between APOBEC-1 expression and apoB mRNA editing activity," Exp. Cell Res. 252:154-164 (1999)); Yang et al., "Induction of cytidine to uridine editing on cytoplasmic apolipoprotein B mRNA by overexpressing APOBEC-1," J. Biol. Chem. 275:22663-22669 (2000).

A double-stranded oligomeric nucleotide encoding the 9-amino acid TAT domain flanked by glycine residues (sense strand shown below, SEQ ID No: 25)
catatgggaa gaaaaaaaag aagacaaaga agaagaggcc tcgag 45
and a PCR product encoding HA-rAPOBEC-CMPK (SEQ ID No: 26 as set forth below) or HA-CMPK (SEQ ID No: 27 as set forth below) (Yang et al., "Induction of cytidine to uridine editing on cytoplasmic apolipoprotein B mRNA by overexpressing APOBEC-1," J. Biol. Chem. 275:22663-22669 (2000), were inserted into *Nde*I/*Xho*I digested p*PROEX* vector (Life, Gaithersburg, Maryland). The entire constructs (TAT-rAPOBEC-CMPK (SEQ ID No: 3) or TAT-CMPK (SEQ ID No: 28 as set forth below) were inserted into p*ET*-24b (Novagen, Madison, Wisconsin) vector to take advantage of the C-terminal His₆ tag. TAT fusion proteins (referred to as TAT-CMPK, the expression product of SEQ ID No: 28, and TAT-rAPOBEC-CMPK, SEQ ID No: 4) were purified from BL-21(DE3) codon plus cells (Stratagene, La Jolla, California). Two to four 1-liter cultures were inoculated with a 10 ml overnight culture each and induced by 0.1 mM IPTG at 30°C for 1 hour. Soluble proteins were obtained by French press in 25 ml of buffer A (8M urea, 10 mM Tris pH 8, 100 mM NaH₂PO₄). Cellular lysates were cleared by centrifugation, loaded onto a 5-ml Ni-NTA column (Qiagen, Valencia, California) in buffer A with 10-20 mM imidazole, washed and eluted with imidazole in buffer A 'stepwise' (100, 175 and 250 mM) and loaded onto a HiTrap SP column (Amersham Pharmacia, Piscataway, New Jersey). The column was washed and eluted with 1 M NaCI in buffer A. The urea and high salt were removed from the relevant fractions by rapid dialysis against buffer B (30 mM Tris pH=8.5, 50 mM NaCI, 10µM zinc acetate, 5% glycerol). The elution profile was analyzed by SDS-PAGE. Gels were stained with silver according to manufacture's recommendations (Bio-Rad, Hercules, California).

Recombinant proteins were solubilized in 8M urea buffer from bacterial cells so as to maximize their yield from inclusion bodies. Previous studies have shown that denatured proteins could transduce as well as native proteins (Schwarze et al., "In vivo protein transduction: delivery of a biologically active protein into the mouse," Science 285:1569-1572 (1999)). The proteins were purified through metal-chelating affinity chromatography followed by cationic exchange chromatography. The urea was removed by rapid dialysis and the purity of full-length 86 kDa TAT-rAPOBEC-CMPK, SEQ ID No: 4, was apparent as shown by silver staining (Figure 5B). The purification of full-length protein was also confirmed by western blot using anti-His₆ antibody.

### Example 2 - In vitro Introduction of TAT-rAPOBEC-CMPK into McArdle Cells

The uptake of TAT-rAPOBEC-CMPK, SEQ ID No: 4, into McArdle cells was evaluated using an antibody reactive with the HA epitope and fluorescence microscopy.

McArdle RH7777 cells were obtained from ATCC (Manassas, Virginia) and cultured as described previously (Yang et al., "Partial characterization of the auxiliary factors involved in apo B mRNA editing through APOBEC-1 affinity chromatography," J. Biol. Chem. 272:27700-27706 (1997)). McArdle cells, grown on six well cluster plates were treated with either TAT-rAPOBEC-CMPK or TAT-CMPK for the indicated times. Cells were then washed extensively with PBS and subsequently fixed with 2% paraformaldehyde, permeabilized with 0.4% Triton X 100, blocked with 1% BSA and reacted with affinity purified anti-HA (Babco, Berkeley, CA) and affinity purified FITC conjugated goat anti-mouse secondary antibody (Organon Teknika, West Chester, PA), each at 1:1000 dilution. Fluorescence was observed and electronic images captured on an inverted, fluorescence Olympus microscope.

Recombinant APOBEC-1 has a tendency to aggregate, a property which persists in TAT-rAPOBEC-CMPK, apparent as aggregates of HA antibody-reactive material attached to the surface of cells 1h following the addition of the protein to the media (Figures 6A-B). Aggregation was not a property of the TAT motif or CMPK as control protein (TAT-CMPK) at a higher molar concentration appeared as an array of speckles attached to the surface of McArdle cells 1 h following its addition to the media (Figures 7A and B).

Within 6 h following treatment, both TAT-rAPOBEC-CMPK (Figures 6C-D) and TAT-CMPK (Figures 7C-D) were apparent inside the cells and the cell surface-attached aggregates appeared to be more disperse. Following 24 h of treatment, many of the cells treated with TAT-rAPOBEC-CMPK demonstrated bright perinuclear fluorescence and also a low intensity of fluorescence throughout the nucleus and cytoplasm (Figures 6E-F). Cells treated for 24 h with TAT-CMPK demonstrated bright fluorescent speckles in the cytoplasm and fainter homogenous nuclear fluorescence (Figure 7E-F). The nuclear distribution of the recombinant protein might have been facilitated by the embedded nuclear localization signal (NLS) in TAT sequence (Schwarze et al., "In vivo protein transduction: delivery of a biologically active protein into the mouse," Science 285:1569-1572 (1999)) as APOBEC-1 alone does not have a functional NLS (Yang et al., "Multiple protein domains determine the cell type-specific nuclear distribution of the catalytic subunit required for apo B mRNA editing," Proc. Natl. Acad. Sci. USA 94:13075-13080 (1997)). and 6His-HA-APOBEC-CMPK was excluded from the nucleus (Yang et al., "Induction of cytidine to uridine editing on cytoplasmic apolipoprotein B mRNA by overexpressing APOBEC-1," J. Biol. Chem. 275:22663-22669 (2000). The data suggested that both TAT-rAPOBEC-CMPK and TAT-CMPK were taken up by McArdle cells. Comparatively, the efficiency of TAT-rAPOBEC-CMPK uptake was poorer than that for TAT-CMPK, and the distribution of these proteins within the cells appeared different.

### Example 3 - Measurement of Apolipoprotein B mRNA Editing in TAT-rAPOBEC-CMPK Transduced McArdle Cells

Given that TAT-CMPK entered McArdle cells, as demonstrated in Example 2, an evaluation was made as to whether this would affect apolipoprotein B mRNA editing activity (Figure 8). Cells were treated with the indicated amounts of TAT-CMPK (using the same preparation of protein as in Figure 7) and total cellular RNA was isolated following 24 h and the proportion of edited apolipoprotein B mRNA measured.

Total cellular RNA was isolated from cells with Tri-Reagent (Molecular Research Center, Cincinnati, Ohio) according to manufacture's recommendations. Purified RNAs were digested with RQ-DNase I (Promega, Madison, Wisconsin) and with RsaI (Promega) restriction enzyme that has a recognition site between the PCR annealing sites of target substrates to ensure the removal of the contaminating genomic DNA.

Editing activity was determined by the reverse transcriptase-polymerase chain reaction (RT-PCR) methodology described previously (Smith et al. "In vitro apolipoprotein B mRNA editing: Identification of a 27S editing complex," Proc. Natl. Acad. Sci. USA 88:1489-1493 (1991)). First strand cDNA was generated using oligo dT-primed total cellular RNA. Specific PCR amplification of rat apolipoprotein B sequence surrounding the editing site was accomplished using ND1/ND2 primer pairs set forth below:
ND1 (SEQ ID No: 29)
   atctgactgg gagagacaag tag 23
ND2 (SEQ ID No: 30)
   gttcttttta agtcctgtgc atc 23

PCR products were gel isolated and the editing efficiency was determined by poisoned primer extension assay using ³²P ATP (NEN, Boston, Massachusetts) end-labeled DD3 primer (SEQ ID No: 31) as follows:
aatcatgtaa atcataacta tctttaatat actga 35
under high concentration of dideoxy GTP as described previously (Smith et al. "In vitro apolipoprotein B mRNA editing: Identification of a 27S editing complex," Proc. Natl. Acad. Sci. USA 88:1489-1493 (1991); Sowden et al., "Overexpression of APOBEC-1 results in mooring-sequence-dependent promiscuous RNA editing," J. Biol. Chem. 271:3011-3017 (1996)). Primer extension products were resolved on a 10% denaturing polyacrylamide gel, autoradiographed, and then quantified by a laser densitometric scanning (Molecular Dynamics, Sunnyvale, California). Percent editing was calculated as the counts in the UAA (edited) band divided by the sum of the counts in UAA and those in the CAA (unedited) bands and multiplied by 100.

No change in the percent editing of apolipoprotein B mRNA relative to untreated cells (see Figure 9) was observed with TAT-CMPK concentrations ranging from 45 to 1125 nM (5 to 133 µg protein/ml of media) (Figure 8).

In contrast, editing activity increased in McArdle cells with 360 nM (62 µg protein/ml media) TAT-rAPOBEC-CMPK following 6 h and continued to a peak by 24 h, a more than 3-fold increase over the level of editing observed in control cells (Figure 9). The proportion of edited RNA remained elevated up to 48 h after treatment (Figure 9) and approached baseline by 72 h. It has been reported that the enzymatic activity lagged the appearance of the transduced protein inside the cells, probably due to a slow refolding of the transduced protein (Schwarze et al., "In vivo protein transduction: delivery of a biologically active protein into the mouse," Science 285:1569-1572 (1999)). Taken together, the results demonstrated that TAT-rAPOBEC-CMPK transduced into McArdle cells, refolded into an enzymatically active conformation over the first 6 hr and then edited apolipoprotein B mRNA. The reduction in the proportion of edited apolipoprotein B mRNA after 48 hr was likely due to enzyme inactivation and apolipoprotein B mRNA turnover. This characteristic was important as it demonstrated the transient and reversible nature of the protein transduction system.

### Example 4 - In vitro Introduction of TAT-rAPOBEC-CMPK into Primary Hepatocytes

To determine if the results obtained using McArdle cells would be applicable in primary liver cells, cultured rat primary hepatocytes were prepared and then treated with TAT-rAPOBEC-CMPK. The rat primary hepatocytes were prepared from unfasted, male Sprague-Dawley rats (250-275 g body weight, Taconic Farm) fed *ad libitum* normal rat chow as described previously (Van Mater et a]., "Ethanol increases apolipoprotein B mRNA editing in rat primary hepatocytes and McArdle cells," Biochem. Biophys. Res. Comm. 252:334-339 (1998)). Recombinant TAT fusion protein was added directly to the cell culture media after dialysis.

It has been shown that the editing efficiency in primary rat hepatocytes decreased as a result of proliferation after 72 hours in culture (Van Mater et al., "Ethanol increases apolipoprotein B mRNA editing in rat primary hepatocytes and McArdle cells," Biochem. Biophys. Res. Comm. 252:334-339 (1998)). Together with the fact that TAT-rAPOBEC-CMPK maximally increased editing 24 hours after treatment in McArdle cells, a decision was made to evaluate dose response for a fixed time rather than study kinetics. Primary hepatocytes were treated with the indicated amounts of TAT-rAPOBEC-CMPK and analyzed for edited apolipoprotein B mRNA 24 hours afterwards. Analysis of apolipoprotein B mRNA was carried out a described in Example 3 above.

The editing activity of hepatocytes increased in proportion to the amount of TAT-rAPOBEC-CMPK added to the cell culture media relative to cells treated with buffer alone (Figure 10) or treated with TAT-CMPK (Figure 8). Given that the primary hepatocytes were seeded at the same cell number as McArdle cells, a comparison of the data in Figures 9 and 10 suggested that TAT-rAPOBEC-CMPK was more effective in inducing editing activity in the primary cell culture. This was true for several preparations of recombinant protein and primary cells and, therefore, the difference may be due to the fact that the primary hepatocytes have a higher baseline of editing than McArdle cells (48% versus 7%) and/or may be "primed" with more auxiliary factors.

Promiscuous editing of additional cytidines in rat apolipoprotein B mRNA oftransfected cells (Sowden et al., "Overexpression of APOBEC-1 results in mooring-sequence-dependent promiscuous RNA editing," J. Biol. Chem. 271:3011-3017 (1996); Yamanaka et al., "Hyperediting of multiple cytidines of apolipoprotein B mRNA by APOBEC-1 requires auxiliary protein(s) but not a mooring sequence motif," J. Biol. Chem. 271:11506-11510 (1996); Sowden et al., "Apolipoprotein B RNA Sequence 3' of the mooring sequence and cellular sources of auxiliary factors determine the location and extent of promiscuous editing," Nucleic Acids Res. 26:1644-1652 (1998). or hyper-editing of other mRNAs in transgenic mice and rabbits (Yamanaka et al., "Hyperediting of multiple cytidines of apolipoprotein B mRNA by APOBEC-1 requires auxiliary protein(s) but not a mooring sequence motif," J. Biol. Chem. 271:11506-11510 (1996); Yamanaka et al., "A novel translational repressor mRNA is edited extensively in livers containing tumors caused by the transgene expression of the apoB mRNA editing enzyme," Genes & Dev. 11:321-333 (1997)) has been observed in response to very high levels of APOBEC-1 expression. Editing of cytidines 5' of the wild type editing site (C6666) was a bellwether for the loss of editing site fidelity in rat cells and could be used to monitor the induction of promiscuous editing in relation to changes in APOBEC-1 expression (Sowden et al., "Apolipoprotein B RNA Sequence 3' of the mooring sequence and cellular sources of auxiliary factors determine the location and extent of promiscuous editing," Nucleic Acids Res. 26:1644-1652 (1998); Siddiqui et al., "Disproportionate relationship between APOBEC-1 expression and apoB mRNA editing activity," Exp. Cell Res. 252 : 154-164 (1999)). Promiscuous editing of cytidine 3' C6666 in apolipoprotein B mRNA did not occur to a significant extent in rat cells and hyperediting of mRNAs other than apolipoprotein B was not a characteristic of APOBEC-1 overexpression in rat cells (Sowden et al., "Apolipoprotein B RNA Sequence 3' of the mooring sequence and cellular sources of auxiliary factors determine the location and extent of promiscuous editing," Nucleic Acids Res. 26 : 1644-1652 (1998)).

Despite the high level of editing activity in treated primary hepatocytes, promiscuous editing (evident as additional primer extension products above UAA (Sowden et al., "Determinants involved in regulating the proportion of edited apolipoprotein B RNAs," RNA 2:274-288 (1996); Sowden et al., "Apolipoprotein B RNA Sequence 3' of the mooring sequence and cellular sources of auxiliary factors determine the location and extent of promiscuous editing," Nucleic Acids Res. 26:1644-1652 (1998). was not observed (Figure 10). Given that our detection limit for promiscuous editing was 0.3% (Sowden et al., "Determinants involved in regulating the proportion of edited apolipoprotein B RNAs," RNA 2:274-288 (1996)) the data suggested that TAT-rAPOBEC-CMPK could be used to substantially increase site-specific editing of apolipoprotein B mRNA without significant loss of fidelity of the reaction.

### Example 5 - Analysis of Secreted Lipoprotein Products by Transduced Primary Hepatocytes

To further confirm the efficacy of this method, secreted apolipoprotein B protein was evaluated in primary rat hepatocytes that were long-term metabolically labeled with [³⁵S]-methionine and [³⁵S]-cysteine after TAT-rAPOBEC-CMPK treatment.

Twelve to eighteen hour rat primary hepatocytes grown in Waymouth's 752/1 media (Sigma, St. Louis, MO) were treated for 11 hours with TAT-rAPOBEC-CMPK and then incubated for 1 hour in DMEM deficient medium (without methionine, cysteine and L-glutamine) (Sigma, St. Louis, MO) containing 0.2% (w/v) BSA, 0.1 nM insulin, 100 µg/ml streptomycin and 50 µg/ml gentamicin. The medium was replaced with fresh labeling medium containing 0.7µCi/ml L-[³⁵S]-Methionine and L-[³⁵S]-Cysteine using EXPRE³⁵S³⁵S protein labeling mix (NEN, Boston, Massachusetts). Cells were incubated in the labeling medium for 30 minutes. One volume of Waymouth's medium with cold cysteine and methionine was added to cells and the labeling continued for an additional 12 hours, after which cell culture medium was collected for the isolation and analysis of secreted apolipoprotein B protein and RNAs. (RNA analysis was conducted as in Example 3 above.)

Immunoprecipitation of apolipoprotein B from cell culture medium was performed as described previously (Sparks et al., "Insulin-mediated inhibition of apolipoprotein B secretion requires an intracellular trafficking event and phosphatidylinositol 3-kinase activation: studies with brefeldin A and wortmannin in primary cultures of rat hepatocytes," Biochem. J. 313:567-574 (1996)). A rabbit polyclonal antibody raised against rat apolipoprotein B and reactive with the N-terminus of apolipoprotein B100 and apolipoprotein B48 (obtained from Drs. J.D. Sparks and C.E. Sparks, University of Rochester) was used to precipitate apolipoprotein B. The immunoprecipitants were separated by SDS-PAGE on 5% gel. The gel was dried and exposed to film to reveal the secreted apolipoprotein B containing lipoprotein profile which represents the secreted apolipoprotein B48 and apolipoprotein B 100 during the 12 hour labeling period.

The secreted [³⁵S]-labeled apolipoprotein B lipoproteins were isolated from the cell culture media exposed to cells for 12 hours followed by immunoprecipitation, and analyzed by autoradiography after SDS-PAGE separation. The signal on the gel was in direct proportion to the number of cysteine and methionine residues in apolipoprotein B100 and apolipoprotein B48. Since apolipoprotein B48 was the N-terminal 48% of apolipoprotein B 100, stronger signal was expected from apolipoprotein B100 in control cells. However, as the editing efficiency approached 90% due to TAT-rAPOBEC-CMPK treatment, an increasing amount of apolipoprotein B48 was secreted, and apolipoprotein B100 became almost undetectable (Figure 11). Thus, lowering apolipoprotein B100 associated atherogenic risk factors through precisely controlled hepatic apolipoprotein B mRNA editing was achievable by protein transduction with TAT-rAPOBEC-CMPK.

### Discussion of Examples 1-5

It is believed that the present invention offers a novel approach to curtail hepatic output of apolipoprotein B100 associated atherogenic factors through up-regulating apolipoprotein B mRNA editing by using protein transduction into target (e.g., liver) cells. The PTD, amino acid residues 49-57, of HIV-1 TAT protein has been used in other systems to deliver functional full-length protein molecules into cells (Nagahara et al., "Transduction of full-length TAT fusion proteins into mammalian cells: TAT-p27Kip1 induces cell migration," Nature Med. 4:1449-1452 (1998); Schwarze et al., "In vivo protein transduction: delivery of a biologically active protein into the mouse," Science 285:1569-1572 (1999)); Vocero-Akbani et al., "Killing HIV-infected cells by transduction with an HIV protease-activated caspase-3 protein," . Nature Med. 5:29-33 (1999)). Some of these fusion molecules, when introduced into mice, entered all tissue cells, even crossing the blood brain barrier (Schwarze et al., "In vivo protein transduction: delivery of a biologically active protein into the mouse," Science 285:1569-1572 (1999)). Although the detailed mechanism for the cellular uptake of the fusions remains unknown, denaturing of the protein during membrane transduction is thought to be a rapid process and the rate limiting event is the renaturing of the transduced protein once inside of cells (Schwarze et al., "Protein transduction: unrestricted delivery into all cells," Trends Cell Biol. 10:290-295 (2000).

In this regard, the protein transduction method may have limitations in that some proteins may not be able successfully to adopt an active conformation after they have been unfolded. It is significant, therefore, that the above Examples demonstrate that both TAT-CMPK (expression product of SEQ ID No: 28) and TAT-rAPOBEC-CMPK (SEQ ID No: 4) had the capacity to enter hepatocytes and that TAT-rAPOBEC-CMPK activated editing within 6 hours of its addition to the media. Similar kinetics have been observed with TAT-rAPOBEC-CMPK prepared under native conditions.

Importantly, TAT-CMPK could not stimulate editing activity, demonstrating that the observed changes in editing were specific to APOBEC-1 containing recombinant proteins. Considering the tendency for APOBEC-1 containing proteins to aggregate, part of the lag in entering cells could have been due to the inability of these multimeric complexes to cross the plasma membrane and the time it took for TAT-rAPOBEC-CMPK monomers to dissociate from the aggregates and cross the membrane. This is supported by the finding that TAT-CMPK, which did not appear to form large aggregates, appeared to accumulate within the cells with more rapid kinetics than that observed for TAT-rAPOBEC-CMPK. The six hour lag before an increase in editing activity could be measured may have also been due to the time required for the transduced protein to refold and assemble editosomes.

Apolipoprotein B mRNA editing occurs in the cell nucleus despite the fact that editing factors can also be demonstrated in the cytoplasm (Yang et al., "Induction of cytidine to uridine editing on cytoplasmic apolipoprotein B mRNA by overexpressing APOBEC-1," J. Biol. Chem. 275:22663-22669 (2000). The mechanism responsible for APOBEC-1's distribution in the nucleus is not understood (Yang et al., "Intracellular Trafficking Determinants in APOBEC-1, the Catalytic Subunit for Cytidine to Uridine Editing of ApoB mRNA," Exp. Cell Res. 267:163-184 (2001)), however its mass appeared to be important as the chimeric protein APOBEC-CMPK was excluded from the nucleus (Yang et al., "Multiple protein domains determine the cell type-specific nuclear distribution of the catalytic subunit required for apo B mRNA editing," Proc. Natl. Acad. Sci. USA 94:13075-13080 (1997); Yang et al., "Induction of cytidine to uridine editing on cytoplasmic apolipoprotein B mRNA by overexpressing APOBEC-1," J. Biol. Chem. 275:22663-22669 (2000). TAT-rAPOBEC-CMPK's ability to distribute in both the cytoplasm and the nucleus was consistent with the proposed ability of the TAT PTD to act also as a nuclear localization signal (Schwarze et al., "In vivo protein transduction: delivery of a biologically active protein into the mouse," Science 285:1569-1572 (1999)). Although TAT-rAPOBEC-CMPK's distribution mimicked that of the wild type enzyme's distribution (Yang et al., "Multiple protein domains determine the cell type-specific nuclear distribution of the catalytic subunit required for apo B mRNA editing," Proc. Natl. Acad. Sci. USA 94:13075-13080 (1997)), uncertainty remains as to whether all of the transduced TAT-rAPOBEC-CMPK molecules were active in editing, as well as whether cytoplasmic or nuclear transcripts were edited. Nonetheless, regardless of the degree of activity or its localization within the cell, a positive reduction in apolipoprotein B100 lipoprotein was demonstrated.

Enhancement of editing activity by overexpression of APOBEC-1 through gene transfer has been shown to be associated with promiscuous editing on both nuclear and cytoplasmic transcripts (Sowden et al., "Overexpression of APOBEC-1 results in mooring-sequence-dependent promiscuous RNA editing," J. Biol. Chem. 271:3011-3017 (1996); Yang et al., "Induction of cytidine to uridine editing on cytoplasmic apolipoprotein B mRNA by overexpressing APOBEC-1," J. Biol. Chem. 275:22663-22669 (2000). Metabolic stimulation of apolipoprotein B mRNA editing always retained fidelity (Wu et al., "ApoB mRNA editing: validation of a sensitive assay and developmental biology of RNA editing in the rat," J. Biol. Chem. 265:12312-12316 (1990); Greeve et al., "Apolipoprotein B mRNA editing in 12 different mammalian species: hepatic expression is reflected in low concentrations of apoB-containing plasma lipoproteins," J. Lipid Res. 34:1367-1383 (1993); Phung et al., "Regulation of hepatic apoB RNA editing in the genetically obese Zucker rat," Metabolism 45:1056-1058 (1996); von Wronski et al., "Insulin increases expression of apobec-1, the catalytic subunit of the apoB B mRNA editing complex in rat hepatocytes," Metabolism Clinical & Exp. 7:869-873 (1998)). It is highly significant, therefore, that the fidelity of the editing activity was retained with TAT-rAPOBEC-CMPK even when editing was enhanced to >90%. This level of high fidelity editing could not be achieved without hyper-editing in *apobec-I* transgenic animals (Yamanaka et al., "Hyperediting of multiple cytidines of apolipoprotein B mRNA by APOBEC-1 I requires auxiliary protein(s) but not a mooring sequence motif," J. Biol. Chem. 271:11506-11510 (1996); Yamanaka et al., "A novel translational repressor mRNA is edited extensively in livers containing tumors caused by the transgene expression of the apoB mRNA editing enzyme," Genes & Dev. 11:321-333 (1997); Sowden et al., "Overexpression of APOBEC-1 results in mooring-sequence-dependent promiscuous RNA editing," J. Biol. Chem. 271:3011-3017 (1996); Sowden et al., "Apolipoprotein B RNA Sequence 3' of the mooring sequence and cellular sources of auxiliary factors determine the location and extent of promiscuous editing," Nucleic Acids Res. 26:1644-1652 (1998)). There was no pathology in transgenic animals in which induction of hepatic apolipoprotein B mRNA editing was achieved at a low level of *apobec-1* expression and these animals had a markedly lower serum apolipoprotein B100 and significantly reduced serum LDL compared to controls (Teng et al., "Adenovirus-mediated gene transfer of rat apolipoprotein B mRNA editing protein in mice virtually eliminates apolipoprotein B-100 and normal low density lipoprotein production," J. Biol. Chem. 269:29395-29404 (1994); Hughs et al., "Gene transfer of cytidine deaminase APOBEC-1 lowers lipoprotein(a) in transgenic mice and induces apolipoprotein B mRNA editing in rabbits," Hum. Gene Ther. 7:39-49 (1996); Kozarsky et al., "Hepatic expression of the catalytic subunit of the apolipoprotein B mRNA editing enzyme ameliorates hypercholesterolemia in LDL receptor-deficient rabbits," Hum. Gene Ther. 7:943-957 (1996); Farese et al., "Phenotypic analysis of mice expressing exclusively apolipoprotein B48 or apolipoprotein B 100," Proc. Natl. Acad. Sci. USA 93:6393-6398 (1996); Qian et al., "Low expression of the apolipoprotein B mRNA editing transgene in mice reduces LDL but does not cause liver dysplasia or tumors," Arteriosc. Thromb. Vasc. Biol. 18:1013-1020 (1998); Wu et al., "Normal perinatal rise in serum cholesterol is inhibited by hepatic delivery of adenoviral vector expressing apolipoprotein B mRNA editing enzyme in rabbits," J. Surg. Res. 85:148-157 (1999)), Interestingly, *apobec-I* gene transfer into apobec- gene knockout mice restored editing and reduced serum LDL levels (Nakamuta et al., "Complete phenotypic characterization of the apobec-1 knockout mice with a wild-type genetic background and a human apolipoprotein B transgenic background, and restoration of apolipoprotein B mRNA editing by somatic gene transfer of Apobec-1," J. Biol. Chem. 271:25981-25988 (1996)), demonstrating that APOBEC-1 has therapeutic potential in livers with no prior editing activity. The induction of hepatic editing of apolipoprotein B mRNA in *apobec-1* transgenic rabbits with an LDL receptor deficiency also ameliorated hypercholesterolemia (Kozarsky et al., "Hepatic expression of the catalytic subunit of the apolipoprotein B mRNA editing enzyme ameliorates hypercholesterolemia in LDL receptor-deficient rabbits," Hum. Gene Ther. 7:943-957 (1996). Taken together, these studies suggested that apolipoprotein B mRNA editing could be safely targeted as a mechanism for reducing serum LDL and the risk of atherogenic diseases.

However, controlling a low level of *apobec-1* expression using gene therapy is difficult and, quite often, unpredictable. For all of these reasons, despite the limited success of gene therapy approaches, gene therapy using *apobec-1* does not appear to be a promising avenue which can be pursued for preventative or therapeutic control over atherogenic disease factors The advantage of protein transduction therapy is that the dose can be modulated relative to the desired response and that the effect on editing can be terminated by withdrawing therapy.

The PTD should allow protein to enter all cells of the body, even if the protein is delivery intravenously (Schwarze et al., "In vivo protein transduction: delivery of a biologically active protein into the mouse," Science 285:1569-1572 (1999)). Ideally the liver should be specifically targeted with TAT-rAPOBEC-CMPK and an intraperitoneal injection can be utilized to accomplish a first pass clearance, transducing most of the protein into hepatocytes. Even though APOBEC-1 is not widely expressed in tissues (Teng et al., "Molecular cloning of an apo B messenger RNA editing protein," Science 260:18116-1819 (1993)), its generalized expression in transgenic animals did not induce pathology (Teng et al., "Adenovirus-mediated gene transfer of rat apolipoprotein B mRNA editing protein in mice virtually eliminates apolipoprotein B-100 and normal low density lipoprotein production," J. Biol. Chem. 269:29395-29404 (1994); Hughs et al., "Gene transfer of cytidine deaminase APOBEC-1 lowers lipoprotein(a) in transgenic mice and induces apolipoprotein B mRNA editing in rabbits," Hum. Gene Ther. 7:39-49 (1996); Kozarsky et al., "Hepatic expression of the catalytic subunit of the apolipoprotein B mRNA editing enzyme ameliorates hypercholesterolemia in LDL receptor-deficient rabbits," Hum. Gene Ther. 7:943-957 (1996); Farese et al., "Phenotypic analysis of mice expressing exclusively apolipoprotein B48 or apolipoprotein B100," Proc. Natl. Acad. Sci. USA 93:6393-6398 (1996); Qian et al., "Low expression of the apolipoprotein B mRNA editing transgene in mice reduces LDL but does not cause liver dysplasia or tumors," Arteriosc. Thromb. Vasc. Biol. 18:1013-1020 (1998); Wu et al., "Normal perinatal rise in serum cholesterol is inhibited by hepatic delivery of adenoviral vector expressing apolipoprotein B mRNA editing enzyme in rabbits," J. Surg. Res. 85:148-157 (1999)).

Uptake of TAT-rAPOBEC-CMPK or TAT-hAPOBEC-CMPK is unlikely to induce any side effects. Aside from one study suggesting that overexpression of APOBEC-1 in liver can lead to editing of mRNAs other than apolipoprotein B (Yamanaka et al., "A novel translational repressor mRNA is edited extensively in livers containing tumors caused by the transgene expression of the apoB mRNA editing enzyme," Genes & Dev. 11:321-333 (1997), which is hereby incorporated by reference in its entirety) no other mRNA substrates for APOBEC-1 have been found (Skuse et al., "Neurofibromatosis type I mRNA undergoes base-modification RNA editing," Nucleic Acids Res. 24:478-486 (1996); Sowden et al., "Apolipoprotein B RNA Sequence 3' of the mooring sequence and cellular sources of auxiliary factors determine the location and extent of promiscuous editing," Nucleic Acids Res. 26:1644-1652 (1998)). Furthermore, *apobec-1* gene knock out studies have shown that there were no other editing enzymes capable of editing apolipoprotein B mRNA and that APOBEC-1 was not required for life (Hirano et al., "Targeted disruption of the mouse apobec-1 gene abolishes apolipoprotein B mRNA editing and eliminates apolipoprotein B48," J. Biol. Chem. 271:9887-9890 (1996); Nakamuta et al., "Complete phenotypic characterization of the apobec-1 knockout mice with a wild-type genetic background and a human apolipoprotein B transgenic background, and restoration of apolipoprotein B mRNA editing by somatic gene transfer of Apobec-1," J. Biol. Chem. 271:25981-25988 (1996)). Taken together the data suggest that mRNA editing by APOBEC is self-limited due to its specificity for apolipoprotein B mRNA and, therefore, neither TAT-rAPOBEC-CMPK nor TAT-hAPOBEC-CMPK is likely to have effects in tissues other than those which express apolipoprotein B mRNA and auxiliary proteins.

Current cholesterol-lowering therapies target circulating cholesterol at the level of enhanced elimination or reduced production. A sector of the population remains at risk for atherosclerosis due to side effects from current therapies in some of these patients and the inability of others with defects in apolipoprotein B and/or the LDL receptor mediated uptake pathway to completely benefit from conventional cholesterol lowering therapies. Hypercholesterolemia is an early onset disease yet the restricted usage of conventional therapies among children due to the potential of interfering with pubertal development has not been resolved. Protein based therapies such as insulin or growth hormone have been extensively used among children to treat Type I diabetes or pituitary dwarfism, respectively. To the patient or the parent of the patient, the reversible nature of protein based therapy may be more appealing than gene therapy. To this end, the above results illustrate an alternative to conventional or gene therapy approaches for reducing the risk of atherosclerosis in the sectors of population at risk.

Although preferred embodiments have been depicted and described in detail herein, it will be apparent to those skilled in the relevant art that various modifications, additions, substitutions, and the like can be made.

## Claims

1. A chimeric protein comprising:
a first polypeptide comprising a protein transduction domain; and
a second polypeptide comprising APOBEC-1 or a fragment thereof which can edit mRNA. encoding apolipoprotein B.

2. The chimeric protein according to claim 1 wherein the APOBEC-1 or fragment thereof comprises an amino acid sequence of SEQ ID No: 11, SEQ ID No: 13, or SEQ ID No: 15, or fragments thereof.

3. The chimeric protein according to claim 1 or 2 further comprising:
a third polypeptide comprising a cytoplasmic localization protein or a fragment thereof which, upon cellular uptake of the chimeric protein, enhances localization of the chimeric protein to the cytoplasm.

4. The chimeric protein according to claim 3 wherein the cytoplasmic localization protein or fragment thereof is chicken muscle pyruvate kinase or a fragment thereof.

5. The chimeric protein according to claim 4 wherein the chicken muscle pyruvate kinase or a fragment thereof comprises an amino acid sequence of SEQ ID No: 17 or fragments thereof.

6. The chimeric protein according to any one of claims 3 -5 wherein, within the chimeric protein, the third polypeptide is C-terminal of the second polypeptide.

7. The chimeric protein according to any one of claims 1 - 6, wherein the chimeric protein comprises an amino acid sequence of SEQ ID No: 2 or SEQ ID No: 4.

8. A chimeric protein comprising:
a first polypeptide comprising a protein transduction domain; and
a second polypeptide comprising APOBEC-1 Complementation Factor (ACF) or a fragment thereof which can bind to apolipoprotein B mRNA to facilitate editing of the mRNA by APOBEC-1.

9. The chimeric protein according to claim 8 wherein the ACF or fragment thereof comprises an amino acid sequence of SEQ ID No: 21 or SEQ ID No: 23 or fragments thereof.

10. The chimeric protein according to claim 8 wherein the chimeric protein comprises an amino acid sequence of SEQ ID No: 6 or SEQ ID No: 8.

11. The chimeric protein according to any of claims 1-10 wherein the protein transduction domain is an HIV tat protein transduction domain.

12. The chimeric protein according to claim 11, wherein the HIV tat protein transduction domain comprises an amino acid sequence of SEQ ID No: 9.

13. The chimeric protein according to any one of claims 1, 2 or 8 - 12 further comprising:
a third polypeptide comprising a plurality of adjacent histidine residues.

14. The chimeric protein according to any one of claims 1, 2 or 8-12 further comprising:
a third polypeptide comprising a hemagglutinin domain.

15. The chimeric protein according to any one of claims 1-14 wherein, within the chimeric protein, the first polypeptide is N-terminal of the second polypeptide.

16. The chimeric protein according to any one of claims 1-15 wherein the chimeric protein is in isolated form.

17. A composition comprising:
a pharmaceutically acceptable carrier and
the chimeric protein according to any one of claims 1-7.

18. The composition according to claim 17, wherein the chimeric protein is present in an amount which is effective to modify apolipoprotein B mRNA editing in liver cells which uptake the chimeric protein.

19. A composition comprising:
a first chimeric protein according to any one of claims 1 to 7; and
a second chimeric protein according to any of claims 8 - 16.

20. The composition according to claim 19 wherein
the first chimeric protein is present in an amount effective to modify apolipoprotein B mRNA editing in cells which uptake the first chimeric protein and
the second chimeric protein is present in an amount which is effective to bind apolipoprotein B mRNA and assist the first chimeric protein in modifying apolipoprotein B mRNA in cells which uptake the first and second chimeric proteins.

21. The composition according to claim 19 or 20 further comprising:
a pharmaceutically acceptable carrier in which the First and second chimeric proteins are dispersed.

22. The composition according to any one of claims 17, 18 or 19-21 wherein the composition is in the form of a tablet, capsule, powder, solution, suspension, or emulsion.

23. A DNA molecule encoding a chimeric protein as defined in any one of claims 1-16.

24. The DNA molecule according to claim 23 comprising a nucleotide sequence of SEQ ID No: 1 or SEQ ID No: 3.

25. The DNA molecule according to claim 23 comprising a nucleotide sequence of SEQ ID No: 5 or SEQ ID No: 7.

26. A DNA construct comprising:
the DNA molecule according to any one of claims 23-25; a promoter sequence operably connected 5' to the DNA molecule; and a 3' regulatory sequence operably , connected 3' of the DNA molecule.

27. An expression vector comprising a DNA molecule according to any one of claims 23-25.

28. A recombinant host cell transformed with a DNA molecule according to any one of claims 23-25.

29. A delivery device comprising a chimeric protein as defined in any one of claims 1-16.

30. A delivery device comprising a composition as defined in any one of claims 17-20.

31. The delivery device according to claims 29 or 30, wherein the delivery device is in the form of a liposome, a niosome, a transdermal patch, an implant, or a syringe.

32. A chimeric protein as defined in any one of claims 1-7 for use in modifying VLDL-apolipoprotein B mRNA editing *in vivo,* wherein VLDL-apolipoprotein B mRNA in a cell is contacted with said chimeric protein under conditions effective to increase the concentration of VLDL-apolipoprotein B48 which is secreted by the cell, as compared to the concentration of VLDL-apolipoprotein B100 which is secreted by the cell, relative to an untreated cell.

33. The chimeric protein according to claim 32 **characterised in that** the cell is a liver cell.

34. The chimeric protein according to claim 32 or 33 **characterised in that** the cell is present in a mammal.

35. The chimeric protein according to any one of claims 32-34 wherein the cell is exposed to said chimeric protein under conditions effective to induce cellular uptake of said chimeric protein prior to contacting the cell with said chimeric protein under the conditions defined in claim 32.

36. The chimeric protein of any one of claims 33-35, wherein the apolipoprotein B mRNA in the cell is further
contacted with a second chimeric protein according to any one of claims 8-16.

37. The chimeric protein of claim 36 wherein the second chimeric protein comprises an amino acid sequence of SEQ ID No.6 or SEQ ID No.8.

38. The chimeric protein of claim 1 for use in reducing serum LDL levels in a patient, wherein said protein is delivered into one or more cells of the patient, without genetically modifying the cells, in an amount which is effective to increase the concentration of VLDL-apolipoprotein B48 that is secreted by one or more of the cells into serum.

39. The chimeric protein of claim 38 wherein the one or more cells are liver cells, intestinal cells, or a combination thereof.

40. The chimeric protein of claims 38 or 39 wherein the patient is a mammal.

41. The chimeric protein of claim 40 wherein the mammal is a human.

42. The chimeric protein according to any one of claims 38-41 wherein said protein is delivered by exposing the one or more cells to the protein under conditions effective to cause cellular uptake of the protein.

43. The chimeric protein according to any one of claims 38-42 wherein the protein is present in a liposome or noisome which is taken up by liver cells.

44. The chimeric protein of any one of claims 38-42 or 43 which is simultaneously delivered into the one or more cells of the patient with an amount of a second protein comprising ACF or a fragment thereof which can bind to apolipoprotein B mRNA, also without genetically modifying the cells.

45. The chimeric protein of claim 44 wherein the proteins are simultaneously delivered by exposing the one or more cells to the second protein under conditions effective to cause cellular uptake of the second protein.

46. The chimeric protein of claim 44 wherein the second protein is a chimeric protein which further comprises a polypeptide comprising a protein transduction domain.

47. The chimeric protein according to claim 46 wherein the second chimeric protein comprises an amino acid sequence of SEQ ID No: 6 or SEQ ID No: 8.

48. The chimeric protein according to any one of claims 38-42 and 44-48 wherein delivering is repeated following a delay.

49. The protein according to claim 48 wherein the delay is from about 1 to about 7 days.

50. The chimeric protein of claim 1 for use in treating or preventing an atherogenic disease or disorder, wherein the protein is administered to a patient and wherein upon said administration the protein is taken up by one or more cells of the patient that can synthesize and secrete VLDL-apolipoprotein B under conditions which are effective to increase the concentration of VLDL-apolipoprotein B48 that is secreted by the one or more cells into serum, whereby rapid clearing of VLDL-apolipoprotein B48 from serum decreases the serum concentration of LDL.

51. The chimeric protein according to claim 50 wherein the patient is a mammal.

52. The chimeric protein according to claim 51 wherein the mammal is a human.

53. The chimeric protein according to any one of claims 50-52 wherein said administration is carried out orally, topically, transdermally, parenterally, subcutaneously, intravenously, intramuscularly, intraperitoneally, by intracavitary or intravesical instillation, intraocularly, intraarterially, intralesionally, by application to mucous membranes, or by implantation.

54. The chimeric protein according to any one of claims 50-53 wherein the chimeric protein comprises an amino acid sequence of SEQ ID No: 2 or SEQ ID No: 4.

55. The chimeric protein according to any of claims 51-56 wherein the protein is present in a liposome or noisome which is taken up by liver cells.

56. The chimeric protein according to any one of claims 50-55 wherein an effective amount of a second protein comprising ACF or a fragment thereof which can bind to apolipoprotein B mRNA is also administered to the patient.

57. The chimeric protein according to claim 56 wherein both proteins are administered simultaneously.

58. The chimeric protein according to claim 56 or 57 wherein the second protein is a chimeric protein which further comprises a protein transduction domain.

59. The chimeric protein according to claim 58 wherein the second chimeric protein comprises an amino acid sequence of SEQ ID No: 6 or SEQ ID No: 8.

60. The chimeric protein according to any one of claims 50-59 wherein the administration is repeated following a delay.

61. The protein according to claim 60 wherein the delay is from about 1 to about 7 days.

62. A liposome or niosome which is targeted for uptake by a liver cell, the liposome or niosome containing (i) the chimeric protein of claim 1, or (ii) the chimeric protein of claim 8, or (iii) a combination thereof.

63. The liposome or niosome according to claim 62 in the form of a liposome comprising asialofetuin incorporated into a lipid bilayer.

64. The liposome or niosome according to claim 62 or 63, in the form of a niosome comprising doxorubicin with a polyoxyethylene surface.

65. The liposome or niosome according to any one of claims 62-64, wherein the liposome or niosome contains the chimeric protein of claim 1.

66. The liposome or niosome according to any one of claims 62-64, wherein the liposome or niosome contains the chimeric protein of claim 8.

67. The liposome or niosome according to any one of claim 62-66, wherein the liposome or niosome contains a combination of the chimeric protein of claim 1 and the chimeric protein of claim 8.

68. A composition comprising:
a pharmaceutically acceptable carrier and the liposome or niosome according to any one of claims 62-67.

## Patentansprüche

1. Chimäres Protein umfassend:
ein erstes Polypeptid, das eine Proteintransduktionsdomäne umfasst; und
ein zweites Polypeptid, das APOBEC-1 oder ein Fragment davon umfasst, das mRNA editieren kann, die Apolipoprotein B codiert.

2. Chimäres Protein nach Anspruch 1, worin das APOBEC-1 oder das Fragment davon eine Aminosäuresequenz gemäß SEQ ID Nr. 11, SEQ ID Nr. 13 oder SEQ ID Nr. 15 oder Fragmente davon umfasst.

3. Chimäres Protein nach Anspruch 1 oder 2, ferner umfassend:
ein drittes Polypeptid, das ein zytoplasmatisches Transportprotein oder ein Fragment davon umfasst, das bei zellulärer Aufnahme des chimären Proteins den Transport des chimären Proteins in das Zytoplasma verstärkt.

4. Chimäres Protein nach Anspruch 3, worin das zytoplasmatische Transportprotein oder das Fragment davon Pyruvatkinase aus Hühnermuskeln oder ein Fragment davon ist.

5. Chimäres Protein nach Anspruch 4, worin die Pyruvatkinase aus Hühnermuskeln oder ein Fragment davon eine Aminosäuresequenz gemäß SEQ ID Nr. 17 oder Fragmente davon umfasst.

6. Chimäres Protein nach einem der Ansprüche 3 bis 5, worin innerhalb des chimären Proteins das dritte Polypeptid C-terminal zum zweiten Polypeptid angeordnet ist.

7. Chimäres Protein nach einem der Ansprüche 1 bis 6, worin das chimäre Protein eine Aminosäuresequenz gemäß SEQ ID Nr. 2 oder SEQ ID Nr. 4 umfasst.

8. Chimäres Protein umfassend:
ein erstes Polypeptid, das eine Proteintransduktionsdomäne umfasst; und
ein zweites Polypeptid, das APOBEC-1 Complementation Factor (ACF) oder ein Fragment davon umfasst, das an Apolipoprotein B mRNA binden kann, um das Editieren der mRNA durch APOBEC-1 zu erleichtern.

9. Chimäres Protein nach Anspruch 8, worin das ACF oder das Fragment davon eine Aminosäuresequenz gemäß SEQ ID Nr. 21 oder SEQ ID Nr. 23 oder Fragmente davon umfasst.

10. Chimäres Protein nach Anspruch 8, worin das chimäre Protein eine Aminosäuresequenz gemäß SEQ ID Nr. 6 oder SEQ ID Nr. 8 umfasst.

11. Chimäres Protein nach einem der Ansprüche 1 bis 10, worin die Proteintransduktionsdomäne eine HIV tat Proteintransduktionsdomäne ist.

12. Chimäres Protein nach Anspruch 11, worin die HIV tat Proteintransduktionsdomäne eine Aminosäuresequenz gemäß SEQ 1D Nr. 9 umfasst.

13. Chimäres Protein nach einem der Ansprüche 1, 2 oder 8 bis 12, ferner umfassend:
ein drittes Polypeptid, das mehrere benachbarte Histidinreste umfasst.

14. Chimäres Protein nach einem der Ansprüche 1, 2 oder 8 bis 12, ferner umfassend:
ein drittes Polypeptid, das eine Hämagglutinin-Domäne umfasst.

15. Chimäres Protein nach einem der Ansprüche 1 bis 14, worin innerhalb des chimären Proteins das erste Polypeptid N-terminal zum zweiten Polypeptid angeordnet ist.

16. Chimäres Protein nach einem der Ansprüche 1 bis 15, worin das chimäre Protein in isolierter Form vorliegt

17. Zusammensetzung umfassend:
einen unter pharmazeutischen Gesichtspunkten annehmbaren Träger und
das chimäre Protein nach einem der Ansprüche 1 bis 7.

18. Zusammensetzung nach Anspruch 17, worin das chimäre Protein in einer Menge vorliegt, die wirksam ist, um das Editieren von Apolipoprotein B mRNA in Leberzellen, die das chimäre Protein aufnehmen, zu verändern.

19. Zusammensetzung umfassend:
ein erstes chimäres Protein nach einem der Ansprüche 1 bis 7; und
ein zweites chimäres Protein nach einem der Ansprüche 8 bis 16.

20. Zusammensetzung nach Anspruch 19, worin
das erste chimäre Protein in einer Menge vorliegt, die wirksam ist, um das Editieren von Apolipoprotein B mRNA in Zellen, die das erste chimäre Protein aufnehmen, zu verändern und
das zweite chimäre Protein in einer Menge vorliegt, die wirksam ist, um Apolipoprotein B mRNA zu binden und das erste chimäre Protein dabei zu unterstützen, Apolipoprotein B mRNA in Zellen, die das erste und zweite chimäre Protein aufnehmen, zu verändern.

21. Zusammensetzung nach Anspruch 19 oder 20, ferner umfassend:
einen unter pharmazeutischen Gesichtspunkten annehmbaren Träger, in dem das erste und zweite Protein dispergiert sind.

22. Zusammensetzung nach einem der Ansprüche 17, 18 oder 19 bis 21, worin die Zusammensetzung in Form einer Tablette, einer Kapsel, eines Pulvers, einer Lösung, einer Suspension oder einer Emulsion vorliegt.

23. DNA-Molekül, das ein chimäres Protein kodiert, wie es in einem der Ansprüche 1 bis 16 definiert ist.

24. DNA-Molekül nach Anspruch 23, das eine Nukleotidsequenz gemäß SEQ ID Nr. 1 oder SEQ ID Nr. 3 umfasst.

25. DNA-Molekül nach Anspruch 23, das eine Nukleotidsequenz gemäß SEQ ID Nr. 5 oder SEQ ID Nr. 7 umfasst.

26. Ein DNA-Konstrukt umfassend:
das DNA-Molekül nach einem der Ansprüche 23 bis 25; eine Promotorsequenz, die in funktionsfähiger Weise 5 an das DNA-Molekül gebunden ist; und eine 3' Regulationssequenz, die in funktionsfähiger Weise 3' an das DNA-Molekül gebunden ist.

27. Expressionsvektor umfassend ein DNA-Molekül nach einem der Ansprüche 23 bis 25.

28. Rekombinante Wirtszelle, die mit einem DNA-Molekül nach einem der Ansprüche 23 bis 25 transformiert wurde.

29. Zufuhrmittel, umfassend ein chimäres Protein, wie es in einem der Ansprüche 1 bis 16 definiert ist.

30. Zufuhrmittel, umfassend eine Zusammensetzung, wie sie in einem der Ansprüche 17 bis 20 definiert ist.

31. Zufuhrmittel nach Anspruch 29 oder 30, worin das zufuhrmittel in Form eines Liposoms, eines Niosoms, eines transdermalen Pflasters, eines Implantats oder einer Injektionsspritze vorliegt.

32. Chimäres Protein wie in einem der Ansprüche 1 bis 7 definiert zur Verwendung bei der Veränderung des Editierens von VLDL-Apolipoprotein B mRNA in vivo, worin VLDL-Apolipoprotein B mRNA in einer Zelle mit dem chimären Protein in Kontakt gebracht wird unter Bedingungen, die wirksam dazu sind, bezogen auf eine unbehandelte Zelle die Konzentration von VLDL-Apolipoprotein B48, das von der Zelle abgesondert wird, im Vergleich zu der Konzentration von VLDL-Apolipoprotein B100, das von der Zelle abgesondert wird, zu erhöhen.

33. Chimäres Protein nach Anspruch 32, **dadurch gekennzeichnet, dass** die Zelle eine Leberzelle ist.

34. Chimäres Protein nach Anspruch 32 oder 33, **dadurch gekennzeichnet, dass** die Zelle in einem Säugetier vorliegt.

35. Chimäres Protein nach einem der Ansprüche 32 bis 34, worin die Zelle dem chimären Protein unter Bedingungen ausgesetzt wird, die wirksam dazu sind, die zelluläre Aufnahme des chimären Proteins einzuleiten bzw. herbeizuführen, bevor die Zelle mit dem chimären Protein unter den in Anspruch 32 definierten Bedingungen in Kontakt gebracht wird.

36. Chimäres Protein nach einem der Ansprüche 33 bis 35, worin die Apolipoprotein B mRNA in der Zelle ferner mit einem zweiten chimären Protein nach einem der Ansprüche 8 bis 16 in Kontakt gebracht wird.

37. Chimäres Protein nach Anspruch 36, worin das zweite chimäre Protein eine Aminosäuresequenz gemäß SEQ ID Nr. 6 oder SEQ ID NR. 8 umfasst.

38. Chimäres Protein nach Anspruch 1 zur Verwendung zur Verringerung der LDL Konzentration im Serum eines Patienten, worin das Protein in eine oder mehrere Zellen des Patienten eingebracht wird, ohne die Zellen genetisch zu verändern, in einer Menge, die wirksam dafür ist, die Konzentration von VL17L-Apolipoprotein B48, das von einer oder mehreren Zellen in das Serum abgesondert wird, zu erhöhen.

39. chimäres Protein nach Anspruch 38, worin die eine oder mehreren Zellen Leberzellen, Darmzellen oder eine Kombination davon sind.

40. Chimäres Protein nach Anspruch 38 oder 39, worin der Patient ein Säugetier ist.

41. Chimäres Protein nach Anspruch 40, worin das Säugetier ein Mensch ist.

42. Chimäres Protein nach einem der Ansprüche 38 bis 41, worin das Protein zugeführt wird, indem die eine oder mehreren Zellen dem Protein unter Bedingungen ausgesetzt werden, die wirksam dazu sind, eine zelluläre Aufnahme des Proteins zu bewirken.

43. Chimäres Protein nach einem der Ansprüche 38 bis 42, worin das Protein in einem Liposom oder Niosom vorliegt, das von Leberzellen aufgenommen wird.

44. Chimäres Protein nach einem der Ansprüche 38 bis 42 oder 43, das gleichzeitig mit einer Menge eines zweiten Proteins, das ACF oder ein Fragment davon, das an Apolipoprotein B mRNA binden kann, umfasst, in die eine oder mehreren Zellen des Patienten eingebracht wird, ebenfalls ohne die Zellen genetisch zu verändern.

45. Chimäres Protein nach Anspruch 44, worin die Proteine gleichzeitig eingebracht werden, indem die eine oder mehreren Zellen dem zweiten Protein unter Bedingungen ausgesetzt werden, die wirksam sind, um eine zelluläre Aufnahme des zweiten Proteins zu bewirken.

46. Chimäres Protein nach Anspruch 44, worin das zweite Protein ein chimäres Protein ist, das ferner ein Polypeptid umfasst, das eine Proteintransduktionsdomäne umfasst,

47. Chimäres Protein nach Anspruch 46, worin das zweite Protein eine Aminosäuresequenz gemäß SEQ ID Nr. 6 oder SEQ ID NR. 8 umfasst.

48. Chimäres Protein nach einem der Ansprüche 38 bis 42 und 44 bis 48, worin das Einbringen nach Ablauf einer Wartezeit wiederholt wird.

49. Chimäres Protein nach Anspruch 48, worin die Wartezeit von etwa 1 bis etwa 7 Tagen beträgt.

50. Chimäres Protein nach Anspruch 1 zur Verwendung zur Behandlung oder Vorbeugung einer atherogenen Krankheit oder Störung, worin das Protein einem Patienten verabreicht wird und worin nach der Verabreichung das Protein von einer oder mehreren Zellen des Patienten aufgenommen wird, die VLDL-Apolipoprotein B synthetisieren und absondern können unter Bedingungen, die wirksam sind, um die Konzentration von VLDL-Apolipoprotein B48, das von den ein oder mehreren Zellen in das Serum abgesondert wird, zu erhöhen, wodurch schnelle Entfernung von VLDL-Apolipoprotein B48 aus dem Serum die Konzentration von LDL im Serum verringert.

51. Chimäres Protein nach Anspruch 50, worin der Patient ein Säugetier ist.

52. Chimäres Protein nach Anspruch 51, worin das Säugetier ein Mensch ist.

53. Chimäres Protein nach einem der Ansprüche 50 bis 52, worin die Verabreichung oral, topisch, transdermal, parenteral, subkutan, intravenös, intramuskulär, intraperitoneal, durch intrakavitäre oder intravesikale Instillation, intraokular, intraarteriell, intraläsional, durch Aufbringen auf Schleimhäute oder durch Implantation durchgeführt wird.

54. Chimäres Protein nach einem der Ansprüche 50 bis 53, worin das chimäre Protein eine Aminosäuresequenz gemäß SEQ ID Nr. 2 oder SEQ ID NR. 4 umfasst.

55. Chimäres Protein nach einem der Ansprüche 51 bis 56, worin das chimäre Protein in einem Liposom oder Niosom vorliegt, das von Leberzellen aufgenommen wird.

56. Chimäres Protein nach einem der Ansprüche 50 bis 55, worin eine wirksame Menge eines zweiten Proteins, das ACF oder ein Fragment davon, das an Apolipoprotein B mRNA binden kann, umfasst, ebenfalls dem Patienten verabreicht wird.

57. Chimäres Protein nach Anspruch 56, worin beide Proteine gleichzeitig verabreicht werden.

58. Chimäres Protein nach Anspruch 56 oder 57, worin das zweite Protein ein chimäres Protein ist, das ferner eine Proteintransduktionsdomäne umfasst.

59. Chimäres Protein nach Anspruch 58, worin das zweite chimäre Protein eine Aminosäuresequenz gemäß SEQ ID Nr. 6 oder SEQ ID NR. 8 umfasst,

60. Chimäres Protein nach einem der Ansprüche 50 bis 59, worin die Verabreichung nach Ablauf einer Wartezeit wiederholt wird.

61. Protein nach Anspruch 60, worin die Wartezeit von etwa 1 bis etwa 7 Tagen beträgt.

62. Liposom oder Niosom, das für die Aufnahme durch eine Leberzelle vorgesehen ist, wobei das Liposom oder Niosom (i) das chimäre Protein nach Anspruch 1, oder (ii) das chimäre Protein nach Anspruch 8, oder (iii) eine Kombination davon enthält.

63. Liposom oder Niosom nach Anspruch 62 in Form eines Liposoms, das in eine Lipiddoppelschicht eingebautes Asialofetuin umfasst.

64. Liposom oder Niosom nach Anspruch 62 oder 63 in Form eines Doxorubicin umfassenden Niosoms mit einer Polyoxyethylenoberfläche.

65. Liposom oder Niosom nach einem der Ansprüche 62 bis 64, worin das Liposom oder Niosom das chimäre Protein nach Anspruch 1 enthält.

66. Liposom oder Niosom nach einem der Ansprüche 62 bis 64, worin das Liposom oder Niosom das chimäre Protein nach Anspruch 8 enthält.

67. Liposom oder Niosom nach einem der Ansprüche 62 bis 66, worin das Liposom oder Niosom eine Kombination des chimären Proteins nach Anspruch 1 und des chimären Proteins nach Anspruch 8 enthält.

68. Eine Zusammensetzung, umfassend:
einen unter pharmazeutischen Gesichtspunkten annehmbaren Träger und das Liposom oder Niosom nach einem der Ansprüche 62 bis 67.

## Revendications

1. Protéine chimérique comprenant :
un premier polypeptide comprenant un domaine de transduction de protéine ; et
un second polypeptide comprenant APOBEC-1 ou un fragment de celle-ci qui peut réguler post-transcriptionnellement l'ARNm codant pour l'apolipoprotéine B.

2. Protéine chimérique selon la revendication 1, dans laquelle APOBEC-1 ou un fragment de celle-ci comprend une séquence d'acide aminé de SEQ ID N° :11, de SEQ ID N°:13 ou de SEQ ID N°:15 ou des fragments de celles-ci.

3. Protéine chimérique selon la revendication 1 ou 2, comprenant en outre :
un troisième polypeptide comprenant une protéine de localisation cytoplasmique ou un fragment de celle-ci qui, par absorption cellulaire de la protéine chimérique, augmente la localisation de la protéine chimérique dans le cytoplasme.

4. Protéine chimérique selon la revendication 3, dans laquelle la protéine de localisation cytoplasmique ou un fragment de celle-ci consiste en la pyruvate kinase du muscle du poulet ou un fragment de celle-ci.

5. Protéine chimérique selon la revendication 4, dans laquelle la pyruvate kinase du muscle du poulet ou un fragment de celle-ci comprend une séquence d'acide aminé de SEQ ID N°: 17 ou des fragments de celle-ci.

6. Protéine chimérique selon l'une quelconque des revendications 3 à 5, dans laquelle, au sein de la protéine chimérique, le troisième polypeptide consiste en la partie C-terminale du second polypeptide.

7. Protéine chimérique selon l'une quelconque des revendications 1 à 6, dans laquelle la protéine chimérique comprend une séquence d'acide aminé de SEQ ID N°;2 ou de SEQ ID N°:4.

8. Protéine chimérique comprenant :
un premier polypeptide comprenant un domaine de transduction de protéine ; et
un second polypeptide comprenant le facteur de complémentation (ACF) de APOBEC-1 ou un fragment de celui-ci qui peut se lier à l'ARNm de l'apolipoprotéine B afin de faciliter la régulation post-transcriptionnelle de l'ARNm par APOBEC-1.

9. Protéine chimérique selon la revendication 8, dans laquelle le facteur ACF ou un fragment de celui-ci comprend une séquence d'acide aminé de SEQ ID N°:21 ou de SEQ ID N°:23 ou des fragments de celles-ci.

10. Protéine chimérique selon la revendication 8, dans laquelle la protéine chimérique comprend une séquence d'acide aminé de SEQ ID N°:6 ou de SEQ ID N°:8.

11. Protéine chimérique selon l'une quelconque des revendications 1 à 10, dans laquelle le domaine de transduction de protéine consiste en le domaine de transduction de la protéine tat du VIH.

12. Protéine chimérique selon la revendication 11, dans laquelle le domaine de transduction de la protéine tat du VIH comprend une séquence d'acide aminé de SEQ ID N°:9.

13. Protéine chimérique selon l'une quelconque des revendications 1, 2 ou 8 à 12 comprenant en outre:
un troisième polypeptide comprenant une pluralité de résidus histidine adjacents.

14. Protéine chimérique selon l'une quelconque des revendications 1, 2 ou 8 à 12 comprenant en outre :
un troisième polypeptide comprenant un domaine de l'hémagglutinine.

15. Protéine chimérique selon l'une quelconque des revendications 1 à 14, dans laquelle, au sein de la protéine chimérique, le premier polypeptide consiste en la partie N-terminale du second polypeptide.

16. Protéine chimérique selon l'une quelconque des revendications 1 à 15, dans laquelle la protéine chimérique est sous forme isolée.

17. Composition comprenant:
un support pharmaceutiquement acceptable et
la protéine chimérique selon l'une quelconque des revendications 1 à 7.

18. Composition selon la revendication 17, dans laquelle la protéine chimérique est présente en une quantité qui est efficace pour modifier la régulation post-transcriptionnelle de l'ARNm de l'apolipoprotéine B dans les cellules hépatiques qui absorbent la protéine chimérique.

19. Composition comprenant:
une première protéine chimérique selon l'une quelconque des revendications 1 à 7 ; et
une seconde protéine chimérique selon l'une quelconque des revendications 8 à 16.

20. Composition selon la revendication 19, dans laquelle :
la première protéine chimérique est présente en une quantité qui est efficace pour modifier la régulation post-transcriptionnelle de l'ARNm de l'apolipoprotéine B dans les cellules qui absorbent la première protéine chimérique ; et
la seconde protéine chimérique est présente en une quantité qui est efficace pour lier l'ARNm de l'apolipoprotéine B et aide la première protéine chimérique lors de la modification de l'ARNm de l'apolipoprotéine B dans les cellules qui absorbent la première et la seconde protéines chimériques.

21. Composition selon la revendication 19 ou 20 comprenant en outre:
un support pharmaceutiquement acceptable dans lequel la première et la seconde protéines chimériques sont dispersées.

22. Composition selon l'une quelconque des revendications 17, 18 ou 19 à 21, dans laquelle la composition est sous la forme de comprimé, de capsule, de poudre, de solution, de suspension ou d'émulsion.

23. Molécule d'ADN codant pour une protéine chimérique telle que définie dans l'une quelconque des revendications 1 à 16.

24. Molécule d'ADN selon la revendication 23, comprenant une séquence nucléotidique de SEQ ID N°:1 ou de SEQ ID N°:3.

25. Molécule d'ADN selon la revendication 23, comprenant une séquence nucléotidique de SEQ ID N°:5 ou de SEQ ID N°:7.

26. Produit de synthèse d'ADN comprenant:
la molécule d'ADN selon l'une quelconque des revendications 23 à 25 ; une séquence promotrice liée, de manière fonctionnelle, en 5' à la molécule d'ADN ; et une séquence régulatrice 3' liée, de manière fonctionnelle, en 3' de la molécule d'ADN.

27. vecteur d'expression comprenant une molécule d'ADN selon l'une quelconque des revendications 23 à 25.

28. Cellule hôte recombinante transformée à l'aide d'une molécule d'ADN selon l'une quelconque des revendications 23 à 25.

29. Dispositif de délivrance comprenant une protéine chimérique telle que définie dans l'une quelconque des revendications 1 à 16.

30. Dispositif de délivrance comprenant une composition telle que définie dans l'une quelconque des revendications 17 à 20.

31. Dispositif de délivrance selon la revendication 29 ou 30, dans laquelle le dispositif de délivrance est sous la forme d'un liposome, un niosome, un patch transcutané, un implant ou une seringue.

32. Protéine chimérique telle que définie dans l'une quelconque des revendication 1 à 7, pour une utilisation dans la modification de la régulation post-transcriptionnelle *in vivo* de l'ARNm de VLDL-apolipoprotéine B, dans laquelle l'ARNm de VLDL-apolipoprotéine B dans une cellule est mis en contact avec ladite protéine chimérique dans des conditions efficaces pour augmenter la concentration de VLDL-apolipoprotéine B48 qui est sécrétée par la cellule, en comparaison avec la concentration de VLDL-apolipoprotéine B100 qui est sécrétée par la cellule, par rapport à une cellule non traitée.

33. Protéine chimérique selon la revendication 32, **caractérisée par le fait que** la cellule est une cellule hépatique.

34. Protéine chimérique selon la revendication 32 ou 33, **caractérisée par le fait que** la cellule est présente chez un mammifère.

35. Protéine chimérique selon l'une quelconque des revendications 32 à 34, dans laquelle la cellule est présentée à ladite protéine chimérique dans des conditions efficaces pour induire l'absorption cellulaire de ladite protéine chimérique avant la mise en contact de la cellule avec la ladite protéine chimérique dans des conditions définies dans la revendication 32.

36. Protéine chimérique selon l'une quelconque des revendications 33 à 35, dans laquelle l'ARNm de l'apolipoprotéine B dans la cellule est, en outre, mis en contact avec une seconde protéine chimérique selon l'une quelconque des revendications 8 à 16.

37. Protéine chimérique selon la revendication 36, dans laquelle la seconde protéine chimérique comprend une séquence d'acide aminé de SEQ ID N°:6 ou de SEQ ID N°:8.

38. Protéine chimérique selon la revendication 1, pour une utilisation dans la réduction des taux de LDL sérique chez un patient, dans laquelle ladite protéine est délivrée dans une ou plusieurs cellules du patient, sans modifier génétiquement les cellules, en une quantité qui est efficace pour augmenter la concentration de VLDL-apolipoprotéine B48 qui est sécrétée par une ou plusieurs des cellules dans le sérum.

39. Protéine chimérique selon la revendication 38, dans laquelle une ou plusieurs cellules sont des cellules hépatiques, des cellules intestinales ou une combinaison de celles-ci.

40. Protéine chimérique selon la revendication 38 ou 39, dans laquelle le patient est un mammifère.

41. Protéine chimérique selon la revendication 40, dans laquelle le mammifère est un être humain.

42. Protéine chimérique selon l'une quelconque des revendications 38 à 41, dans laquelle ladite protéine est délivrée par présentation d'une ou de plusieurs cellules à la protéine dans des conditions efficaces pour permettre l'absorption cellulaire de la protéine.

43. Protéine chimérique selon l'une quelconque des revendications 38 à 42, dans laquelle la protéine est présente dans un liposome ou un niosome qui est capturé par les cellules hépatiques.

44. Protéine chimérique de l'une quelconque des revendications 38 à 42 ou 43, qui est simultanément délivrée dans une ou plusieurs cellules du patient avec une quantité d'une seconde protéine comprenant le facteur ACF ou un fragment de celui-ci qui peut se lier à l'ARNm de l'apolipoprotéine B, également sans modifier génétiquement les cellules.

45. Protéine chimérique selon la revendication 44, dans laquelle les protéines sont simultanément délivrées par présentation d'une ou de plusieurs cellules à la seconde protéine dans des conditions efficaces pour permettre l'absorption cellulaire de la seconde protéine.

46. Protéine chimérique selon la revendication 44, dans laquelle la seconde protéine est une protéine chimérique qui comprend, en outre, un polypeptide comprenant un domaine de transduction de protéine.

47. Protéine chimérique selon la revendication 46, dans laquelle la seconde protéine chimérique comprend une séquence d'acide aminé de SEQ ID N°:6 ou de SEQ ID N°:8.

48. Protéine chimérique selon l'une quelconque des revendication 38 à 42 et 44 à 48, dans laquelle la délivrance est répétée à la suite d'un décalage.

49. Protéine selon la revendication 48, dans laquelle le décalage est compris entre environ 1 jour à environ 7 jours.

50. Protéine chimérique de la revendication 1, pour une utilisation dans le traitement ou la prévention d'une maladie ou d'un trouble athérogène, dans laquelle la protéine est administrée à un patient et dans laquelle par ladite administration la protéine est capturée par une ou plusieurs cellules du patient qui peuvent synthétiser et secréter la VLDL-apolipoprotéine B dans des conditions qui sont efficaces pour augmenter la concentration de la VLDL-apolipoprotéine B48 qui est sécrétée par une ou plusieurs cellules dans le sérum, à la suite de quoi l'élimination rapide de VLDL-apolipoprotéine B48 du sérum diminue la concentration sérique en LDL.

51. Protéine chimérique selon la revendication 50, dans laquelle le patient est un mammifère.

52. Protéine chimérique selon la revendication 51, dans laquelle le mammifère est un être humain.

53. Protéine chimérique selon l'une quelconque des revendication 50 à 52, dans laquelle ladite administration est réalisée par voie orale, locale, transdermique, parentérale, sous-cutanée, intraveineuse, intramusculaire, intrapéritonéale, par instillation intracavitaire ou intravésiculaire, intra-oculaire, intra-artérielle, intra-lésionnelle, par application à des membranes muqueuses ou par implantation.

54. Protéine chimérique selon l'une quelconque des revendication 50 à 53, dans laquelle la protéine chimérique comprend une séquence d'acide aminé de SEQ ID N°:2 ou de SEQ ID N°:4.

55. Protéine chimérique selon l'une quelconque des revendication 51 à 56, dans laquelle la protéine est présente dans un liposome ou un niosome qui est capturé par les cellules hépatiques.

56. Protéine chimérique selon l'une quelconque des revendication 50 à 55, dans laquelle une quantité efficace d'une seconde protéine comprenant le facteur ACF ou un fragment de celui-ci qui peut se lier à l'ARNm de l'apolipoprotéine B est également administrée au patient.

57. Protéine chimérique selon la revendication 56, dans laquelle les deux protéines sont administrées de manière simultanée.

58. Protéine chimérique selon la revendication 56 ou 57, dans laquelle la seconde protéine est une protéine chimérique qui comprend en outre un domaine de transduction de protéine.

59. Protéine chimérique selon la revendication 58, dans laquelle la seconde protéine chimérique comprend une séquence d'acide aminé de SEQ ID N°:6 ou de SEQ ID N°:8.

60. Protéine chimérique selon l'une quelconque des revendication 50 à 59, dans laquelle l'administration est répétée à la suite d'un décalage.

61. Protéine selon la revendication 60, dans laquelle le décalage est compris entre environ 1 jour à environ 7 jours.

62. Liposome ou niosome qui est la cible d'une absorption par une cellule hépatique, le liposome ou le niosome contenant (i) la protéine chimérique de la revendication 1, ou (ii) la protéine chimérique de la revendication 8 ou (iii) une combinaison de celles-ci.

63. Liposome ou niosome selon la revendication 62, sous la forme d'un liposome comprenant l'asialofétuine incorporée dans une bicouche lipidique

64. Liposome ou niosome selon la revendication 62 ou 63, sous la forme d'un niosome comprenant la doxorubicine avec une surface de polyoxyéthylène.

65. Liposome ou niosome selon l'une quelconque des revendications 62 à 64, dans laquelle le liposome ou le niosome contient la protéine chimérique de la revendication 1.

66. Liposome ou niosome selon l'une quelconque des revendications 62 à 64, dans laquelle le liposome ou le niosome contient la protéine chimérique de la revendication 8.

67. Liposome ou niosome selon l'une quelconque des revendications 62 à 66, dans laquelle le liposome ou le niosome contient une combinaison de la protéine chimérique de la revendication 1 et de la protéine chimérique de la revendication 8.

68. Composition comprenant :
un support pharmaceutiquement acceptable et le liposome ou le niosome selon l'une quelconque des revendications 62 à 67.


### SEQUENCE LISTING

<110> University of Rochester
   Smith, Harold C.
   Yang, Yan
   Sowden, Mark P.
<120> METHODS AND COMPOSITIONS FOR MODIFYING APOLIPOPROTEIN B mRNA EDITING
<130> 59.68.82107
<140> European Patent Application No. 02731102.6
   <141> 2002-02-26
<150> PCT/US02/05824
   <151> 2002-02-26
<150> 60/271,856
   <151> 2001-02-27
<160> 31
<170> Patent In Ver. 2.1
<210> 1
   <211> 2406
   <212> DNA
   <213> Artificial Sequence
<220>
   <223> Description of Artificial Sequence:
   TAT-hAPOBEC-CMPK
<400> 1
<210> 2
   <211> 801
   <212> PRT
   <213> Artificial Sequence
<220>
<223> Description of Artificial Sequence:
   TAT-hAPOBEC-CMPK
<400> 2
   Met Ala Ser Met Thr Gly Gly Gln Gln Met Gly Arg Asp Pro Gly Tyr
   1               5                   10                  15
   Gly Arg Lys Lys Arg Arg Gln Arg Arg Arg Gly Ser Arg Tyr Pro Tyr
               20                  25                  30
   Asp Val Pro Asp Tyr Ala Asp Ile Thr Ser Glu Lys Gly Pro Ser Thr
           35                  40                  45
   Gly Asp Pro Thr Leu Arg Arg Arg Ile Glu Pro Trp Glu Phe Asp Val
       50                  55                  60
   Phe Tyr Asp Pro Arg Glu Leu Arg Lys Glu Ala Cys Leu Leu Tyr Glu
   65                  70                  75                  80
   Ile Lys Trp Gly Met Ser Arg Lys Ile Trp Arg Ser Ser Gly Lys Asn
                   85                  90                  95
   Thr Thr Asn His Val Glu Val Asn Phe Ile Lys Lys Phe Thr Ser Glu
               100                 105                 110
   Arg Asp Phe His Pro Ser Ile Ser Cys Ser Ile Thr Trp Phe Leu Ser
           115                 120                 125
   Trp Ser Pro Cys Trp Glu Cys Ser Gln Ala Ile Arg Glu Phe Leu Ser
       130                 135                 140
   Arg His Pro Gly Val Thr Leu Val Ile Tyr Val Ala Arg Leu Phe Trp
   145                 150                 155                 160
   His Met Asp Gln Gln Asn Arg Gln Gly Leu Arg Asp Leu Val Asn Ser
                   165                 170                 175
   Gly Val Thr Ile Gln Ile Met Arg Ala Ser Glu Tyr Tyr His Cys Trp
               180                     185                     190
   Arg Asn Phe Val Asn Tyr Pro Pro Gly Asp Glu Ala His Trp Pro Gln
           195                     200                     205
   Tyr Pro Pro Leu Trp Met Met Leu Tyr Ala Leu Glu Leu His Cys Ile
       210                     215                     220
   Ile Leu Ser Leu Pro Pro Cys Leu Lys Ile Ser Arg Arg Trp Gln Asn
   225                     230                     235                     240
   His Leu Thr Phe Phe Arg Leu His Leu Gln Asn Cys His Tyr Gln Thr
                   245                     250                     255
   Ile Pro Pro His Ile Leu Leu Ala Thr Gly Leu Ile His Pro Ser Val
               260                     265                     270
   Ala Trp Arg Glu Phe His Ala Ala Met Ala Asp Thr Phe Leu Glu His
           275                     280                     285
   Met Cys Arg Leu Asp Ile Asp Ser Glu Pro Thr Ile Ala Arg Asn Thr
       290                     295                     300
   Gly Ile Ile Cys Thr Ile Gly Pro Ala Ser Arg Ser Val Asp Lys Leu
   305                     310                     315                     320
   Lys Glu Met Ile Lys Ser Gly Met Asn Val Ala Arg Leu Asn Phe Ser
                   325                     330                     335
   His Gly Thr His Glu Tyr His Glu Gly Thr Ile Lys Asn Val Arg Glu
               340                     345                     350
   Ala Thr Glu Ser Phe Ala Ser Asp Pro Ile Thr Tyr Arg Pro Val Ala
           355                     360                     365
   Ile Ala Leu Asp Thr Lys Gly Pro Glu Ile Arg Thr Gly Leu Ile Lys
       370                     375                     380
   Gly Ser Gly Thr Ala Glu Val Glu Leu Lys Lys Gly Ala Ala Leu Lys
   385                     390                     395                     400
   Val Thr Leu Asp Asn Ala Phe Met Glu Asn Cys Asp Glu Asn Val Leu
                   405                     410                     415
   Trp Val Asp Tyr Lys Asn Leu Ile Lys Val Ile Asp Val Gly Ser Lys
               420                     425                     430
   Ile Tyr Val Asp Asp Gly Leu Ile Ser Leu Leu Val Lys Glu Lys Gly
           435                     440                     445
   Lys Asp Phe Val Met Thr Glu Val Glu Asn Gly Gly Met Leu Gly Ser
       450                     455                     460
   Lys Lys Gly Val Asn Leu Pro Gly Ala Ala Val Asp Leu Pro Ala Val
   465                     470                     475                     480
   Ser Glu Lys Asp Ile Gln Asp Leu Lys Phe Gly Val Glu Gln Asn Val
                   485                     490                     495
   Asp Met Val Phe Ala Ser Phe Ile Arg Lys Ala Ala Asp Val His Ala
   500 505 510
   Val Arg Lys Val Leu Gly Glu Lys Gly Lys His Ile Lys Ile Ile Ser
   515 520 525
   Lys Ile Glu Asn His Glu Gly Val Arg Arg Phe Asp Glu Ile Met Glu
   530 535 540
   Ala Ser Asp Gly Ile Met Val Ala Arg Gly Asp Leu Gly Ile Glu Ile
   545 550 555 560
   Pro Ala Glu Lys Val Phe Leu Ala Gln Lys Met Met Ile Gly Arg Cys
   565 570 575
   Asn Arg Ala Gly Lys Pro Ile Ile Cys Ala Thr Gln Met Leu Glu Ser
   580 585 590
   Met Ile Lys Lys Pro Arg Pro Thr Arg Ala Glu Gly Ser Asp Val Ala
   595 600 605
   Asn Ala Val Leu Asp Gly Ala Asp Cys Ile Met Leu Ser Gly Glu Thr
   610 615 620
   Ala Lys Gly Asp Tyr Pro Leu Glu Ala Val Arg Met Gln His Ala Ile
   625 630 635 640
   Ala Arg Glu Ala Glu Ala Ala Met Phe His Arg Gln Gln Phe Glu Glu
   645 650 655
   Ile Leu Arg His Ser Val His His Arg Glu Pro Ala Asp Ala Met Ala
   660 665 670
   Ala Gly Ala Val Glu Ala Ser Phe Lys Cys Leu Ala Ala Ala Leu Ile
   675 680 685
   Val Met Thr Glu Ser Gly Arg Ser Ala His Leu Val Ser Arg Tyr Arg
   690 695 700
   Pro Arg Ala Pro Ile Ile Ala Val Thr Arg Asn Asp Gln Thr Ala Arg
   705 710 715 720
   Gln Ala His Leu Tyr Arg Gly Val Phe Pro Val Leu Cys Lys Gln Pro
   725 730 735
   Ala His Asp Ala Trp Ala Glu Asp Val Asp Leu Arg Val Asn Leu Gly
   740 745 750
   Met Asn Val Gly Lys Ala Arg Gly Phe Phe Lys Thr Gly Asp Leu Val
   755 760 765
   Ile Val Leu Thr Gly Trp Arg Pro Gly Ser Gly Tyr Thr Asn Thr Met
   770 775 780
   Arg Val Val Pro Val Pro Ala Ala Ala Leu Glu His His His His His
   785 790 795 800
   His
<210> 3
   <211> 2385
   <212> DNA
   <213> Artificial Sequence
<220>
<223> Description of Artificial Sequence:
   TAT-rAPOBEC-CMPK
<400> 3
   atggctagca tgactggtgg acagcaaatg ggtcgggatc cgggatatgg aagaaaaaaa 60
   agaagacaaa gaagaagagg ctctagatac ccctacgacg tgcccgacta cgccgatatc 120
   agttccgaga caggccctgt agctgttgat cccactctga ggagaagaat tgagccccac 180
   gagtttgaag tcttctttga cccccgggaa cttcggaaag agacctgtct gctgtatgag 240
   atcaactggg gaggaaggca cagcatctgg cgacacacga gccaaaacac caacaaacac 300
   gttgaagtca atttcataga aaaatttact acagaaagat acttttgtcc aaacaccaga 360
   tgctccatta cctggttcct gtcctggagt ccctgtgggg agtgctccag ggccattaca 420
   gaatttttga gccgataccc ccatgtaact ctgtttattt atatagcacg gctttatcac 480
   cacgcagatc ctcgaaatcg gcaaggactc agggacctta ttagcagcgg tgttactatc 540
   cagatcatga cggagcaaga gtctggctac tgctggagga attttgtcaa ctactcccct 600
   tcgaatgaag ctcattggcc aaggtacccc catctgtggg tgaggctgta cgtactggaa 660
   ctctactgca tcattttagg acttccaccc tgtttaaata ttttaagaag aaaacaacct 720
   caactcacgt ttttcacgat tgctcttcaa agctgccatt accaaaggct accaccccac 780
   atcctgtggg ccacagggtt gaaagaattc cacgctgcca tggcagacac ctttctggag 840
   cacatgtgcc gcctggacat cgactccgag ccaaccattg ccagaaacac cggcatcatc 900
   tgcaccatcg gcccagcctc ccgctctgtg gacaagctga aggaaatgat taaatctgga 960
   atgaatgttg cccgcctcaa cttctcgcac ggcacccacg agtatcatga gggcacaatt 1020
   aagaacgtgc gagaggccac agagagcttt gcctctgacc cgatcaccta cagacctgtg 1080
   gctattgcac tggacaccaa gggacctgaa atccgaactg gactcatcaa gggaagtggc 1140
   acagcagagg tggagctcaa gaagggcgca gctctcaaag tgacgctgga caatgccttc 1200
   atggagaact gcgatgagaa tgtgctgtgg gtggactaca agaacctcat caaagttata 1260
   gatgtgggca gcaaaatcta tgtggatgac ggtctcattt ccttgctggt taaggagaaa 1320
   ggcaaggact ttgtcatgac tgaggttgag aacggtggca tgcttggtag taagaaggga 1380
   gtgaacctcc caggtgctgc ggtcgacctg cctgcagtct cagagaagga cattcaggac 1440
   ctgaaatttg gcgtggagca gaatgtggac atggtgttcg cttccttcat ccgcaaagct 1500
   gctgatgtcc atgctgtcag gaaggtgcta ggggaaaagg gaaagcacat caagattatc 1560
   agcaagattg agaatcacga gggtgtgcgc aggtttgatg agatcatgga ggccagcgat 1620
   ggcattatgg tggcccgtgg tgacctgggt attgagatcc ctgctgaaaa agtcttcctc 1680
   gcacagaaga tgatgattgg gcgctgcaac agggctggca aacccatcat ttgtgccact 1740
   cagatgttgg aaagcatgat caagaaacct cgcccgaccc gcgctgaggg cagtgatgtt 1800
   gccaatgcag ttctggatgg agcagactgc atcatgctgt ctggggagac cgccaaggga 1860
   gactacccac tggaggctgt gcgcatgcag cacgctattg ctcgtgaggc tgaggccgca 1920
   atgttccatc gtcagcagtt tgaagaaatc ttacgccaca gtgtacacca cagggagcct 1980
   gctgatgcca tggcagcagg cgcggtggag gcctccttta agtgcttagc agcagctctg 2040
   atagttatga ccgagtctgg caggtctgca cacctggtgt cccggtaccg cccgcgggct 2100
   cccatcatcg ccgtcacccg caatgaccaa acagcacgcc aggcacacct gtaccgcggc 2160
   gtcttccccg tgctgtgcaa gcagccggcc cacgatgcct gggcagagga tgtggatctc 2220
   cgtgtgaacc tgggcatgaa tgtcggcaaa gcccgtggat tcttcaagac cggggacctg 2280
   gtgatcgtgc tgacgggctg gcgccccggc tccggctaca ccaacaccat gcgggtggtg 2340
   cccgtgccag cggccgcact cgagcaccac caccaccacc actga                 2385
<210> 4
   <211> 794
   <212> PRT
   <213> Artificial Sequence
<220>
   <223> Description of Artificial Sequence:
   TAT-rAPOBEC-CMPK
<400> 4
   Met Ala Ser Met Thr Gly Gly Gln Gln Met Gly Arg Asp Pro Gly Tyr
    1               5                  10                  15
   Gly Arg Lys Lys Arg Arg Gln Arg Arg Arg Gly Ser Arg Tyr Pro Tyr
               20                  25                  30
   Asp Val Pro Asp Tyr Ala Asp Ile Ser Ser Glu Thr Gly Pro Val Ala
           35                  40                  45
   Val Asp Pro Thr Leu Arg Arg Arg Ile Glu Pro His Glu Phe Glu Val
       50                  55                  60
   Phe Phe Asp Pro Arg Glu Leu Arg Lys Glu Thr Cys Leu Leu Tyr Glu
   65                  70                  75                  80
   Ile Asn Trp Gly Gly Arg His Ser Ile Trp Arg His Thr Ser Gln Asn
                   85                  90                  95
   Thr Asn Lys His Val Glu Val Asn Phe Ile Glu Lys Phe Thr Thr Glu
               100                 105                 110
   Arg Tyr Phe Cys Pro Asn Thr Arg Cys Ser Ile Thr Trp Phe Leu Ser
           115                 120                 125
   Trp Ser Pro Cys Gly Glu Cys Ser Arg Ala Ile Thr Glu Phe Leu Ser
       130                 135                 140
   Arg Tyr Pro His Val Thr Leu Phe Ile Tyr Ile Ala Arg Leu Tyr His
   145                 150                 155                 160
   His Ala Asp Pro Arg Asn Arg Gln Gly Leu Arg Asp Leu Ile Ser Ser
                   165                 170                 175
   Gly Val Thr Ile Gln Ile Met Thr Glu Gln Glu Ser Gly Tyr Cys Trp
               180                 185                 190
   Arg Asn Phe Val Asn Tyr Ser Pro Ser Asn Glu Ala His Trp Pro Arg
           195                 200                 205
   Tyr Pro His Leu Trp Val Arg Leu Tyr Val Leu Glu Leu Tyr Cys Ile
       210                 215                 220
   Ile Leu Gly Leu Pro Pro Cys Leu Asn Ile Leu Arg Arg Lys Gln Pro
   225                 230                 235                 240
   Gln Leu Thr Phe Phe Thr Ile Ala Leu Gln Ser Cys His Tyr Gln Arg
                   245                 250                 255
   Leu Pro Pro His Ile Leu Trp Ala Thr Gly Leu Lys Glu Phe His Ala
               260                 265                 270
   Ala Met Ala Asp Thr Phe Leu Glu His Met Cys Arg Leu Asp Ile Asp
           275                 280                 285
   Ser Glu Pro Thr Ile Ala Arg Asn Thr Gly Ile Ile Cys Thr Ile Gly
       290                 295                 300
   Pro Ala Ser Arg Ser Val Asp Lys Leu Lys Glu Met Ile Lys Ser Gly
   305                 310                 315                 320
   Met Asn Val Ala Arg Leu Asn Phe Ser His Gly Thr His Glu Tyr His
   325 330 335
   Glu Gly Thr Ile Lys Asn Val Arg Glu Ala Thr Glu Ser Phe Ala Ser
   340 345 350
   Asp Pro Ile Thr Tyr Arg Pro Val Ala Ile Ala Leu Asp Thr Lys Gly
   355 360 365
   Pro Glu Ile Arg Thr Gly Leu Ile Lys Gly Ser Gly Thr Ala Glu Val
   370 375 380
   Glu Leu Lys Lys Gly Ala Ala Leu Lys Val Thr Leu Asp Asn Ala Phe
   385 390 395 400
   Met Glu Asn Cys Asp Glu Asn Val Leu Trp Val Asp Tyr Lys Asn Leu
   405 410 415
   Ile Lys Val Ile Asp Val Gly Ser Lys Ile Tyr Val Asp Asp Gly Leu
   420 425 430
   Ile Ser Leu Leu Val Lys Glu Lys Gly Lys Asp Phe Val Met Thr Glu
   435 440 445
   Val Glu Asn Gly Gly Met Leu Gly Ser Lys Lys Gly Val Asn Leu Pro
   450 455 460
   Gly Ala Ala Val Asp Leu Pro Ala Val Ser Glu Lys Asp Ile Gln Asp
   465 470 475 480
   Leu Lys Phe Gly Val Glu Gln Asn Val Asp Met Val Phe Ala Ser Phe
   485 490 495
   Ile Arg Lys Ala Ala Asp Val His Ala Val Arg Lys Val Leu Gly Glu
   500 505 510
   Lys Gly Lys His Ile Lys Ile Ile Ser Lys Ile Glu Asn His Glu Gly
   515 520 525
   Val Arg Arg Phe Asp Glu Ile Met Glu Ala Ser Asp Gly Ile Met Val
   530 535 540
   Ala Arg Gly Asp Leu Gly Ile Glu Ile Pro Ala Glu Lys Val Phe Leu
   545 550 555 560
   Ala Gln Lys Met Met Ile Gly Arg Cys Asn Arg Ala Gly Lys Pro Ile
   565 570 575
   Ile Cys Ala Thr Gln Met Leu Glu Ser Met Ile Lys Lys Pro Arg Pro
   580 585 590
   Thr Arg Ala Glu Gly Ser Asp Val Ala Asn Ala Val Leu Asp Gly Ala
   595 600 605
   Asp Cys Ile Met Leu Ser Gly Glu Thr Ala Lys Gly Asp Tyr Pro Leu
   610 615 620
   Glu Ala Val Arg Met Gln His Ala Ile Ala Arg Glu Ala Glu Ala Ala
   625 630 635 640
   Met Phe His Arg Gln Gln Phe Glu Glu Ile Leu Arg His Ser Val His
   645 650 655
   His Arg Glu Pro Ala Asp Ala Met Ala Ala Gly Ala Val Glu Ala Ser
               660                 665                 670
   Phe Lys Cys Leu Ala Ala Ala Leu Ile Val Met Thr Glu Ser Gly Arg
           675                 680                 685
   Ser Ala His Leu Val Ser Arg Tyr Arg Pro Arg Ala Pro Ile Ile Ala
       690                 695                 700
   Val Thr Arg Asn Asp Gln Thr Ala Arg Gln Ala His Leu Tyr Arg Gly
   705                 710                 715                 720
   Val Phe Pro Val Leu Cys Lys Gln Pro Ala His Asp Ala Trp Ala Glu
                   725                 730                 735
   Asp Val Asp Leu Arg Val Asn Leu Gly Met Asn Val Gly Lys Ala Arg
               740                 745                 750
   Gly Phe Phe Lys Thr Gly Asp Leu Val Ile Val Leu Thr Gly Trp Arg
           755                 760                 765
   Pro Gly Ser Gly Tyr Thr Asn Thr Met Arg Val Val Pro Val Pro Ala
       770                 775                 780
   Ala Ala Leu Glu His His His His His His
   785                 790
<210> 5
   <211> 1914
   <212> DNA
   <213> Artificial Sequence
<220>
   <223> Description of Artificial Sequence: TAT-hACF
<400> 5
   atggctagca tgactggtgg acagcaaatg ggtcgggatc cgggatatgg aagaaaaaaa 60
   agaagacaaa gaagaagagg ctctagatac ccctacgacg tgcccgacta cgccgatatc 120
   atggaatcaa atcacaaatc cggggatgga ttgagcggca ctcagaagga agcagccctc 180
   cgcgcactgg tccagcgcac aggatatagc ttggtccagg aaaatggaca aagaaaatat 240
   ggtggccctc cacctggttg ggatgctgca ccccctgaaa ggggctgtga aatttttatt 300
   ggaaaacttc cccgagacct ttttgaggat gagcttatac cattatgtga aaaaatcggt 360
   aaaatttatg aaatgagaat gatgatggat tttaatggca acaatagagg atatgcattt 420
   gtaacatttt caaataaagt ggaagccaag aatgcaatca agcaacttaa taattatgaa 480
   attagaaatg ggcgcctctt aggggtttgt gccagtgtgg acaactgccg attatttgtt 540
   gggggcatcc caaaaaccaa aaagagagaa gaaatcttat cggagatgaa aaaggttact 600
   gaaggtgttg tcgatgtcat cgtctaccca agcgctgcag ataaaaccaa aaaccgaggc 660
   tttgccttcg tggagtatga gagtcatcga acagctgcca tggcgaggag gaaactgcta 720
   ccaggaagaa ttcagttatg gggacatggt attgcagtag actgggcaga gccagaagta 780
   gaagttgatg aagatacaat gtcttcagtg aaaatcctat atgtaagaaa tcttatgctg 840
   tctacctctg aagagatgat tgaaaaggaa ttcaacaata tcaaaccagg tgctgtggag 900
   agggtgaaga aaattcgaga ctatgctttt gtgcacttca gtaaccgaaa agatgcagtt 960
   gaggctatga aagctttaaa tggcaaggtg ctggatggtt cccccattga agtcacccta 1020
   gcaaaaccag tggacaagga cagttatgtt aggtataccc gaggcacagg tggaaggggc 1080
   accatgctgc aaggagagta tacctactct ttgggccaag tttatgatcc caccacaacc 1140
   taccttggag ctcctgtctt ctatgccccc cagacctatg cagcaattcc cagtcttcat 1200
   ttcccagcca ccaaaggaca tctcagcaac agagccatta tccgagcccc ttctgttaga 1260
   ggggctgcgg gagtgagagg actgggcggc cgtggctatt tggcatacac aggcctgggt 1320
   cgaggatacc aggtcaaagg agacaaaaga gaagacaaac tctatgacat tttacctggg 1380
   atggagctca ccccaatgaa tcctgtcaca ttaaaacccc aaggaattaa actcgctccc 1440
   cagatattag aagagatttg tcagaaaaat aactggggac agccagtgta ccagctgcac 1500
   tctgctattg gacaagacca aagacagcta ttcttgtaca aaataactat tcctgctcta 1560
   gccagccaga atcctgcaat ccaccctttc acacctccaa agctgagtgc ctttgtggat 1620
   gaagcaaaga cgtatgcagc cgaatacacc ctgcagaccc tgggcatccc cactgatgga 1680
   ggcgatggca ccatggctac tgctgctgct gctgctactg ctttcccagg atatgctgtc 1740
   cctaatgcaa ctgcacccgt gtctgcagcc cagctcaagc aagcggtaac ccttggacaa 1800
   gacttagcag catatacaac ctatgaggtc tacccaactt ttgcagtgac tgcccgaggg 1860
   gatggatatg gcaccttcgc ggccgcactc gagcaccacc accaccacca ctga       1914
<210> 6
   <211> 637
   <212> PRT
   <213> Artificial Sequence
<220>
   <223> Description of Artificial Sequence: TAT-hACF
<400> 6
   Met Ala Ser Met Thr Gly Gly Gln Gln Met Gly Arg Asp Pro Gly Tyr
    1               5                  10                  15
   Gly Arg Lys Lys Arg Arg Gln Arg Arg Arg Gly Ser Arg Tyr Pro Tyr
               20                  25                  30
   Asp Val Pro Asp Tyr Ala Asp Ile Met Glu Ser Asn His Lys Ser Gly
           35                  40                  45
   Asp Gly Leu Ser Gly Thr Gln Lys Glu Ala Ala Leu Arg Ala Leu Val
       50                  55                  60
   Gln Arg Thr Gly Tyr Ser Leu Val Gln Glu Asn Gly Gln Arg Lys Tyr
   65                  70                  75                  80
   Gly Gly Pro Pro Pro Gly Trp Asp Ala Ala Pro Pro Glu Arg Gly Cys
                   85                  90                  95
   Glu Ile Phe Ile Gly Lys Leu Pro Arg Asp Leu Phe Glu Asp Glu Leu
               100                 105                 110
   Ile Pro Leu Cys Glu Lys Ile Gly Lys Ile Tyr Glu Met Arg Met Met
           115                 120                 125
   Met Asp Phe Asn Gly Asn Asn Arg Gly Tyr Ala Phe Val Thr Phe Ser
       130                 135                 140
   Asn Lys Val Glu Ala Lys Asn Ala Ile Lys Gln Leu Asn Asn Tyr Glu
   145                 150                 155                 160
   Ile Arg Asn Gly Arg Leu Leu Gly Val Cys Ala Ser Val Asp Asn Cys
                   165                 170                 175
   Arg Leu Phe Val Gly Gly Ile Pro Lys Thr Lys Lys Arg Glu Glu Ile
               180                 185                 190
   Leu Ser Glu Met Lys Lys Val Thr Glu Gly Val Val Asp Val Ile Val
           195                 200                 205
   Tyr Pro Ser Ala Ala Asp Lys Thr Lys Asn Arg Gly Phe Ala Phe Val
       210                 215                 220
   Glu Tyr Glu Ser His Arg Thr Ala Ala Met Ala Arg Arg Lys Leu Leu
   225                 230                 235                 240
   Pro Gly Arg Ile Gln Leu Trp Gly His Gly Ile Ala Val Asp Trp Ala
                   245                 250                 255
   Glu Pro Glu Val Glu Val Asp Glu Asp Thr Met Ser Ser Val Lys Ile
               260                 265                 270
   Leu Tyr Val Arg Asn Leu Met Leu Ser Thr Ser Glu Glu Met Ile Glu
           275                 280                 285
   Lys Glu Phe Asn Asn Ile Lys Pro Gly Ala Val Glu Arg Val Lys Lys
       290                 295                 300
   Ile Arg Asp Tyr Ala Phe Val His Phe Ser Asn Arg Lys Asp Ala Val
   305                 310                 315                 320
   Glu Ala Met Lys Ala Leu Asn Gly Lys Val Leu Asp Gly Ser Pro Ile
                   325                 330                 335
   Glu Val Thr Leu Ala Lys Pro Val Asp Lys Asp Ser Tyr Val Arg Tyr
               340                 345                 350
   Thr Arg Gly Thr Gly Gly Arg Gly Thr Met Leu Gln Gly Glu Tyr Thr
           355                 360                 365
   Tyr Ser Leu Gly Gln Val Tyr Asp Pro Thr Thr Thr Tyr Leu Gly Ala
       370                 375                 380
   Pro Val Phe Tyr Ala Pro Gln Thr Tyr Ala Ala Ile Pro Ser Leu His
   385                 390                 395                 400
   Phe Pro Ala Thr Lys Gly His Leu Ser Asn Arg Ala Ile Ile Arg Ala
                   405                 410                 415
   Pro Ser Val Arg Gly Ala Ala Gly Val Arg Gly Leu Gly Gly Arg Gly
               420                 425                 430
   Tyr Leu Ala Tyr Thr Gly Leu Gly Arg Gly Tyr Gln Val Lys Gly Asp
           435                 440                 445
   Lys Arg Glu Asp Lys Leu Tyr Asp Ile Leu Pro Gly Met Glu Leu Thr
       450                 455                 460
   Pro Met Asn Pro Val Thr Leu Lys Pro Gln Gly Ile Lys Leu Ala Pro
   465                 470                 475                 480
   Gln Ile Leu Glu Glu Ile Cys Gln Lys Asn Asn Trp Gly Gln Pro Val
                   485                 490                 495
   Tyr Gln Leu His Ser Ala Ile Gly Gln Asp Gln Arg Gln Leu Phe Leu
               500                 505                 510
   Tyr Lys Ile Thr Ile Pro Ala Leu Ala Ser Gln Asn Pro Ala Ile His
           515                 520                 525
   Pro Phe Thr Pro Pro Lys Leu Ser Ala Phe Val Asp Glu Ala Lys Thr
       530                 535                 540
   Tyr Ala Ala Glu Tyr Thr Leu Gln Thr Leu Gly Ile Pro Thr Asp Gly
   545                 550                 555                 560
   Gly Asp Gly Thr Met Ala Thr Ala Ala Ala Ala Ala Thr Ala Phe Pro
                   565                 570                 575
   Gly Tyr Ala Val Pro Asn Ala Thr Ala Pro Val Ser Ala Ala Gln Leu
               580                 585                 590
   Lys Gln Ala Val Thr Leu Gly Gln Asp Leu Ala Ala Tyr Thr Thr Tyr
           595                 600                 605
   Glu Val Tyr Pro Thr Phe Ala Val Thr Ala Arg Gly Asp Gly Tyr Gly
       610                 615                 620
   Thr Phe Ala Ala Ala Leu Glu His His His His His His
   625                 630                 635
<210> 7
   <211> 1914
   <212> DNA
   <213> Artificial Sequence
<220>
   <223> Description of Artificial Sequence: TAT-rACF
<400> 7
   atggctagca tgactggtgg acagcaaatg ggtcgggatc cgggatatgg aagaaaaaaa 60
   agaagacaaa gaagaagagg ctctagatac ccctacgacg tgcccgacta cgccgatatc 120
   atggaatcaa atcacaaatc cggggatgga ttgagcggca cccagaagga agcagcactc 180
   cgcgcactgg tccagcgcac aggatatagc ttggtccagg aaaatggaca aagaaaatat 240
   ggtggtcctc caccaggctg ggatactaca cccccagaaa ggggctgcga gattttcatt 300
   gggaaacttc cccgggacct ttttgaggat gaactcatac cattgtgtga aaaaattggt 360
   aaaatttatg aaatgagaat gatgatggat ttcaatggga acaacagagg ctatgcattt 420
   gtaaccttct caaataagca ggaagccaag aatgcaatca agcaacttaa taattatgaa 480
   attcggaatg gccgtctcct gggcgtctgt gccagtgtgg acaactgccg gttgtttgtg 540
   gggggaatcc ccaaaaccaa aaagagagaa gaaatcttgt cagagatgaa aaaggtcact 600
   gaaggagttg ttgatgtcat tgtctaccca agcgctgccg ataaaaccaa aaaccggggg 660
   tttgcctttg tggaatatga gagtcaccgc gcagccgcca tggctaggcg gaggctgctg 720
   ccaggaagaa ttcagttgtg gggacatcct atcgcagtag actgggcaga gccagaagtc 780
   gaagttgacg aagacacaat gtcttccgtg aaaatcctgt acgtaaggaa ccttatgctg 840
   tctacctcgg aagagatgat tgagaaggaa ttcaacagta ttaaaccagg tgctgtggaa 900
   cgggtgaaga agatccgaga ctatgctttt gtgcatttca gtaaccgaga agatgcagtt 960
   gaagccatga aggctttgaa tggcaaggtg ctggatggtt ccccaataga agtgaccttg 1020
   gccaagccag tggacaagga cagttacgtt aggtacaccc ggggcaccgg gggcaggaac 1080
   accatgctgc aagaatacac ctaccctctg agccatgttt atgaccctac cacaacctac 1140
   cttggagctc ctgtcttcta tactccccaa gcctacgcag ccattccaag tcttcatttc 1200
   ccagctacca aaggacatct cagcaacaga gctctcatcc ggaccccttc tgtcagaggg 1260
   gctgcgggcg tgagaggact gggcggccgt gggtatttgg catatacagg cctgggtcga 1320
   ggataccagg tcaaaggaga caagagacaa gacaaactct atgaccttct gcctgggatg 1380
   gagctcaccc cgatgaatac tatctcttta aaaccacaag gagttaaact tgctcctcag 1440
   atattagaag aaatctgtca gaaaaataac tggggacagc cagtgtacca gctgcactct 1500
   gccattggac aagaccaaag acagttattc ctatacaaag taactatccc agcgctggcc 1560
   agccagaatc ctgcgatcca ccctttcaca cccccaaagc taagcgccta cgtggatgaa 1620
   gcaaagaggt acgccgcaga gcacacccta cagacactag gcatccccac agaaggaggg 1680
   gacgctggga ctacagcacc cactgccaca tccgccactg tgtttccagg atacgctgtc 1740
   cccagtgcca ccgctcctgt gtctacagcc cagctcaagc aagcagtgac acttggacaa 1800
   gacttagcag catatacaac ctatgaggtc taccctactt ttgcagtgac cacccgaggt 1860
   gatggatatg gcaccttcgc ggccgcactc gagcaccacc accaccacca ctga       1914
<210> 8
   <211> 637
   <212> PRT
   <213> Artificial Sequence
<220>
   <223> Description of Artificial Sequence: TAT-rACF
<400> 8
   Met Ala Ser Met Thr Gly Gly Gln Gln Met Gly Arg Asp Pro Gly Tyr
    1               5                  10                  15
   Gly Arg Lys Lys Arg Arg Gln Arg Arg Arg Gly Ser Arg Tyr Pro Tyr
               20                  25                  30
   Asp Val Pro Asp Tyr Ala Asp Ile Met Glu Ser Asn His Lys Ser Gly
           35                  40                  45
   Asp Gly Leu Ser Gly Thr Gln Lys Glu Ala Ala Leu Arg Ala Leu Val
       50                  55                  60
   Gln Arg Thr Gly Tyr Ser Leu Val Gln Glu Asn Gly Gln Arg Lys Tyr
   65                  70                  75                  80
   Gly Gly Pro Pro Pro Gly Trp Asp Thr Thr Pro Pro Glu Arg Gly Cys
                   85                  90                  95
   Glu Ile Phe Ile Gly Lys Leu Pro Arg Asp Leu Phe Glu Asp Glu Leu
               100                 105                 110
   Ile Pro Leu Cys Glu Lys Ile Gly Lys Ile Tyr Glu Met Arg Met Met
           115                 120                 125
   Met Asp Phe Asn Gly Asn Asn Arg Gly Tyr Ala Phe Val Thr Phe Ser
       130                 135                 140
   Asn Lys Gln Glu Ala Lys Asn Ala Ile Lys Gln Leu Asn Asn Tyr Glu
   145                 150                 155                 160
   Ile Arg Asn Gly Arg Leu Leu Gly Val Cys Ala Ser Val Asp Asn Cys
                   165                 170                 175
   Arg Leu Phe Val Gly Gly Ile Pro Lys Thr Lys Lys Arg Glu Glu Ile
               180                 185                 190
   Leu Ser Glu Met Lys Lys Val Thr Glu Gly Val Val Asp Val Ile Val
           195                 200                 205
   Tyr Pro Ser Ala Ala Asp Lys Thr Lys Asn Arg Gly Phe Ala Phe Val
       210                 215                 220
   Glu Tyr Glu Ser His Arg Ala Ala Ala Met Ala Arg Arg Arg Leu Leu
   225                 230                 235                 240
   Pro Gly Arg Ile Gln Leu Trp Gly His Pro Ile Ala Val Asp Trp Ala
                   245                 250                 255
   Glu Pro Glu Val Glu Val Asp Glu Asp Thr Met Ser Ser Val Lys Ile
               260                 265                 270
   Leu Tyr Val Arg Asn Leu Met Leu Ser Thr Ser Glu Glu Met Ile Glu
           275                 280                 285
   Lys Glu Phe Asn Ser Ile Lys Pro Gly Ala Val Glu Arg Val Lys Lys
       290                 295                 300
   Ile Arg Asp Tyr Ala Phe Val His Phe Ser Asn Arg Glu Asp Ala Val
   305                 310                 315                 320
   Glu Ala Met Lys Ala Leu Asn Gly Lys Val Leu Asp Gly Ser Pro Ile
                   325                 330                 335
   Glu Val Thr Leu Ala Lys Pro Val Asp Lys Asp Ser Tyr Val Arg Tyr
               340                 345                 350
   Thr Arg Gly Thr Gly Gly Arg Asn Thr Met Leu Gln Glu Tyr Thr Tyr
           355                 360                 365
   Pro Leu Ser His Val Tyr Asp Pro Thr Thr Thr Tyr Leu Gly Ala Pro
       370                 375                 380
   Val Phe Tyr Thr Pro Gln Ala Tyr Ala Ala Ile Pro Ser Leu His Phe
   385                 390                 395                 400
   Pro Ala Thr Lys Gly His Leu Ser Asn Arg Ala Leu Ile Arg Thr Pro
                   405                 410                 415
   Ser Val Arg Gly Ala Ala Gly Val Arg Gly Leu Gly Gly Arg Gly Tyr
               420                 425                 430
   Leu Ala Tyr Thr Gly Leu Gly Arg Gly Tyr Gln Val Lys Gly Asp Lys
           435                 440                 445
   Arg Gln Asp Lys Leu Tyr Asp Leu Leu Pro Gly Met Glu Leu Thr Pro
       450                 455                 460
   Met Asn Thr Ile Ser Leu Lys Pro Gln Gly Val Lys Leu Ala Pro Gln
   465                 470                 475                 480
   Ile Leu Glu Glu Ile Cys Gln Lys Asn Asn Trp Gly Gln Pro Val Tyr
                   485                 490                 495
   Gln Leu His Ser Ala Ile Gly Gln Asp Gln Arg Gln Leu Phe Leu Tyr
               500                 505                 510
   Lys Val Thr Ile Pro Ala Leu Ala Ser Gln Asn Pro Ala Ile His Pro
           515                 520                 525
   Phe Thr Pro Pro Lys Leu Ser Ala Tyr Val Asp Glu Ala Lys Arg Tyr
       530                 535                 540
   Ala Ala Glu His Thr Leu Gln Thr Leu Gly Ile Pro Thr Glu Gly Gly
   545                 550                 555                 560
   Asp Ala Gly Thr Thr Ala Pro Thr Ala Thr Ser Ala Thr Val Phe Pro
                   565                 570                 575
   Gly Tyr Ala Val Pro Ser Ala Thr Ala Pro Val Ser Thr Ala Gln Leu
               580                 585                 590
   Lys Gln Ala Val Thr Leu Gly Gln Asp Leu Ala Ala Tyr Thr Thr Tyr
   595 600 605
   Glu Val Tyr Pro Thr Phe Ala Val Thr Thr Arg Gly Asp Gly Tyr Gly
   610 615 620
   Thr Phe Ala Ala Ala Leu Glu His His His His His His
   625 630 635
<210> 9
   <211> 9
   <212> PRT
   <213> Artificial Sequence
<220>
   <223> Description of Artificial Sequence: protein transduction domain of HIV-1
<400> 9
   Arg Lys Lys Arg Arg Gln Arg Arg Arg
   1 5
<210> 10
   <211> 27
   <212> DNA
   <213> Artificial Sequence
<220>
   <223> Description of Artificial Sequence: encodes protein transduction domain of HIV-1
<400> 10
   agaaaaaaaa gaagacaaag aagaaga 27
<210> 11
   <211> 236
   <212> PRT
   <213> Homo sapiens
<400> 11
   Met Thr Ser Glu Lys Gly Pro Ser Thr Gly Asp Pro Thr Leu Arg Arg
   1               5                   10                  15
   Arg Ile Glu Pro Trp Glu Phe Asp Val Phe Tyr Asp Pro Arg Glu Leu
               20                  25                  30
   Arg Lys Glu Ala Cys Leu Leu Tyr Glu Ile Lys Trp Gly Met Ser Arg
           35                  40                  45
   Lys Ile Trp Arg Ser Ser Gly Lys Asn Thr Thr Asn His Val Glu Val
       50                  55                  60
   Asn Phe Ile Lys Lys Phe Thr Ser Glu Arg Asp Phe His Pro Ser Ile
   65                  70                  75                  80
   Ser Cys Ser Ile Thr Trp Phe Leu Ser Trp Ser Pro Cys Trp Glu Cys
                   85                  90                  95
   Ser Gln Ala Ile Arg Glu Phe Leu Ser Arg His Pro Gly Val Thr Leu
               100                 105                 110
   Val Ile Tyr Val Ala Arg Leu Phe Trp His Met Asp Gln Gln Asn Arg
           115                 120                 125
   Gln Gly Leu Arg Asp Leu Val Asn Ser Gly Val Thr Ile Gln Ile Met
       130                 135                 140
   Arg Ala Ser Glu Tyr Tyr His Cys Trp Arg Asn Phe Val Asn Tyr Pro
   145                 150                 155                 160
   Pro Gly Asp Glu Ala His Trp Pro Gln Tyr Pro Pro Leu Trp Met Met
                   165                 170                 175
   Leu Tyr Ala Leu Glu Leu His Cys Ile Ile Leu Ser Leu Pro Pro Cys
               180                 185                 190
   Leu Lys Ile Ser Arg Arg Trp Gln Asn His Leu Thr Phe Phe Arg Leu
           195                 200                 205
   His Leu Gln Asn Cys His Tyr Gln Thr Ile Pro Pro His Ile Leu Leu
       210                 215                 220
   Ala Thr Gly Leu Ile His Pro Ser Val Ala Trp Arg
   225                 230                 235
<210> 12
   <211> 711
   <212> DNA
   <213> Homo sapiens
<400> 12
   atgacttctg agaaaggtcc ttcaaccggt gaccccactc tgaggagaag aatcgaaccc 60
   tgggagtttg acgtcttcta tgaccccaga gaacttcgta aagaggcctg tctgctctac 120
   gaaatcaagt ggggcatgag ccggaagatc tggcgaagct caggcaaaaa caccaccaat 180
   cacgtggaag ttaattttat aaaaaaattt acgtcagaaa gagattttca cccatccatc 240
   agctgctcca tcacctggtt cttgtcctgg agtccctgct gggaatgctc ccaggctatt 300
   agagagtttc tgagtcggca ccctggtgtg actctagtga tctacgtagc tcggcttttt 360
   tggcacatgg atcaacaaaa tcggcaaggt ctcagggacc ttgttaacag tggagtaact 420
   attcagatta tgagagcatc agagtattat cactgctgga ggaattttgt caactaccca 480
   cctggggatg aagctcactg gccacaatac ccacctctgt ggatgatgtt gtacgcactg 540
   gagctgcact gcataattct aagtcttcca ccctgtttaa agatttcaag aagatggcaa 600
   aatcatctta catttttcag acttcatctt caaaactgcc attaccaaac gattccgcca 660
   cacatccttt tagctacagg gctgatacat ccttctgtgg cttggagatg a          711
<210> 13
   <211> 229
   <212> PRT
   <213> Rattus norvegicus
<400> 13
<210> 14
   <211> 690
   <212> DNA
   <213> Rattus norvegicus
<400> 14
   atgagttccg agacaggccc tgtagctgtt gatcccactc tgaggagaag aattgagccc 60
   cacgagtttg aagtcttctt tgacccccgg gaacttcgga aagagacctg tctgctgtat 120
   gagatcaact ggggaggaag gcacagcatc tggcgacaca cgagccaaaa caccaacaaa 180
   cacgttgaag tcaatttcat agaaaaattt actacagaaa gatacttttg tccaaacacc 240
   agatgctcca ttacctggtt cctgtcctgg agtccctgtg gggagtgctc cagggccatt 300
   acagaatttt tgagccgata cccccatgta actctgttta tttatatagc acggctttat 360
   caccacgcag atcctcgaaa tcggcaagga ctcagggacc ttattagcag cggtgttact 420
   atccagatca tgacggagca agagtctggc tactgctgga ggaattttgt caactactcc 480
   ccttcgaatg aagctcattg gccaaggtac ccccatctgt gggtgaggct gtacgtactg 540
   gaactctact gcatcatttt aggacttcca ccctgtttaa atattttaag aagaaaacaa 600
   cctcaactca cgtttttcac gattgctctt caaagctgcc attaccaaag gctaccaccc 660
   cacatcctgt gggccacagg gttgaaatga                                  690
<210> 15
   <211> 229
   <212> PRT
   <213> Mus musculus
<400> 15
   Met Ser Ser Glu Thr Gly Pro Val Ala Val Asp Pro Thr Leu Arg Arg
    1               5                  10                  15
   Arg Ile Glu Pro His Glu Phe Glu Val Phe Phe Asp Pro Arg Glu Leu
               20                  25                  30
   Arg Lys Glu Thr Cys Leu Leu Tyr Glu Ile Asn Trp Gly Gly Arg His
           35                  40                  45
   Ser Val Trp Arg His Thr Ser Gln Asn Thr Ser Asn His Val Glu Val
       50                  55                  60
   Asn Phe Leu Glu Lys Phe Thr Thr Glu Arg Tyr Phe Arg Pro Asn Thr
   65                  70                  75                  80
   Arg Cys Ser Ile Thr Trp Phe Leu Ser Trp Ser Pro Cys Gly Glu Cys
                   85                  90                  95
   Ser Arg Ala Ile Thr Glu Phe Leu Ser Arg His Pro Tyr Val Thr Leu
               100                 105                 110
   Phe Ile Tyr Ile Ala Arg Leu Tyr His His Thr Asp Gln Arg Asn Arg
           115                 120                 125
   Gln Gly Leu Arg Asp Leu Ile Ser Ser Gly Val Thr Ile Gln Ile Met
       130                 135                 140
   Thr Glu Gln Glu Tyr Cys Tyr Cys Trp Arg Asn Phe Val Asn Tyr Pro
   145                 150                 155                 160
   Pro Ser Asn Glu Ala Tyr Trp Pro Arg Tyr Pro His Leu Trp Val Lys
                   165                 170                 175
   Leu Tyr Val Leu Glu Leu Tyr Cys Ile Ile Leu Gly Leu Pro Pro Cys
               180                 185                 190
   Leu Lys Ile Leu Arg Arg Lys Gln Pro Gln Leu Thr Phe Phe Thr Ile
           195                 200                 205
   Thr Leu Gln Thr Cys His Tyr Gln Arg Ile Pro Pro His Leu Leu Trp
       210                 215                 220
   Ala Thr Gly Leu Lys
   225
<210> 16
   <211> 690
   <212> DNA
   <213> Mus musculus
<400> 16
   atgagttccg agacaggccc tgtagctgtt gatcccactc tgaggagaag aattgagccc 60
   cacgagtttg aagtcttctt tgacccccgg gagcttcgga aagagacctg tctgctgtat 120
   gagatcaact ggggtggaag gcacagtgtc tggcgacaca cgagccaaaa caccagcaac 180
   cacgttgaag tcaacttctt agaaaaattt actacagaaa gatactttcg tccgaacacc 240
   agatgctcca ttacctggtt cctgtcctgg agtccctgcg gggagtgctc cagggccatt 300
   acagagtttc tgagccgaca cccctatgta actctgttta tttacatagc acggctttat 360
   caccacacgg atcagcgaaa ccgccaagga ctcagggacc ttattagcag cggtgtgact 420
   atccagatca tgacagagca agagtattgt tactgctgga ggaatttcgt caactacccc 480
   ccttcaaacg aagcttattg gccaaggtac ccccatctgt gggtgaaact gtatgtattg 540
   gagctctact gcatcatttt aggacttcca ccctgtttaa aaattttaag aagaaagcaa 600
   cctcaactca cgtttttcac aattactctt caaacctgcc attaccaaag gataccaccc 660
   catctccttt gggctacagg gttgaaatga                                  690
<210> 17
   <211> 530
   <212> PRT
   <213> Gallus gallus
<400> 17
   Met Ser Lys His His Asp Ala Gly Thr Ala Phe Ile Gln Thr Gln Gln
    1               5                  10                  15
   Leu His Ala Ala Met Ala Asp Thr Phe Leu Glu His Met Cys Arg Leu
               20                  25                  30
   Asp Ile Asp Ser Glu Pro Thr Ile Ala Arg Asn Thr Gly Ile Ile Cys
           35                  40                  45
   Thr Ile Gly Pro Ala Ser Arg Ser Val Asp Lys Leu Lys Glu Met Ile
       50                  55                  60
   Lys Ser Gly Met Asn Val Ala Arg Leu Asn Phe Ser His Gly Thr His
   65                  70                  75                  80
   Glu Tyr His Glu Gly Thr Ile Lys Asn Val Arg Glu Ala Thr Glu Ser
                   85                  90                  95
   Phe Ala Ser Asp Pro Ile Thr Tyr Arg Pro Val Ala Ile Ala Leu Asp
               100                 105                 110
   Thr Lys Gly Pro Glu Ile Arg Thr Gly Leu Ile Lys Gly Ser Gly Thr
           115                 120                 125
   Ala Glu Val Glu Leu Lys Lys Gly Ala Ala Leu Lys Val Thr Leu Asp
       130                 135                 140
   Asn Ala Phe Met Glu Asn Cys Asp Glu Asn Val Leu Trp Val Asp Tyr
   145                 150                 155                 160
   Lys Asn Leu Ile Lys Val Ile Asp Val Gly Ser Lys Ile Tyr Val Asp
                   165                 170                 175
   Asp Gly Leu Ile Ser Leu Leu Val Lys Glu Lys Gly Lys Asp Phe Val
               180                 185                 190
   Met Thr Glu Val Glu Asn Gly Gly Met Leu Gly Ser Lys Lys Gly Val
           195                 200                 205
   Asn Leu Pro Gly Ala Ala Val Asp Leu Pro Ala Val Ser Glu Lys Asp
       210                 215                 220
   Ile Gln Asp Leu Lys Phe Gly Val Glu Gln Asn Val Asp Met Val Phe
   225                 230                 235                 240
   Ala Ser Phe Ile Arg Lys Ala Ala Asp Val His Ala Val Arg Lys Val
                   245                 250                 255
   Leu Gly Glu Lys Gly Lys His Ile Lys Ile Ile Ser Lys Ile Glu Asn
               260                 265                 270
   His Glu Gly Val Arg Arg Phe Asp Glu Ile Met Glu Ala Ser Asp Gly
           275                 280                 285
   Ile Met Val Ala Arg Gly Asp Leu Gly Ile Glu Ile Pro Ala Glu Lys
       290                 295                 300
   Val Phe Leu Ala Gln Lys Met Met Ile Gly Arg Cys Asn Arg Ala Gly
   305                 310                 315                 320
   Lys Pro Ile Ile Cys Ala Thr Gln Met Leu Glu Ser Met Ile Lys Lys
                   325                 330                 335
   Pro Arg Pro Thr Arg Ala Glu Gly Ser Asp Val Ala Asn Ala Val Leu
               340                 345                 350
   Asp Gly Ala Asp Cys Ile Met Leu Ser Gly Glu Thr Ala Lys Gly Asp
           355                 360                 365
   Tyr Pro Leu Glu Ala Val Arg Met Gln His Ala Ile Ala Arg Glu Ala
       370                 375                 380
   Glu Ala Ala Met Phe His Arg Gln Gln Phe Glu Glu Ile Leu Arg His
   385                 390                 395                 400
   Ser Val His His Arg Glu Pro Ala Asp Ala Met Ala Ala Gly Ala Val
                   405                 410                 415
   Glu Ala Ser Phe Lys Cys Leu Ala Ala Ala Leu Ile Val Met Thr Glu
               420                 425                 430
   Ser Gly Arg Ser Ala His Leu Val Ser Arg Tyr Arg Pro Arg Ala Pro
           435                 440                 445
   Ile Ile Ala Val Thr Arg Asn Asp Gln Thr Ala Arg Gln Ala His Leu
       450                 455                 460
   Tyr Arg Gly Val Phe Pro Val Leu Cys Lys Gln Pro Ala His Asp Ala
   465                 470                 475                 480
   Trp Ala Glu Asp Val Asp Leu Arg Val Asn Leu Gly Met Asn Val Gly
                   485                 490                 495
   Lys Ala Arg Gly Phe Phe Lys Thr Gly Asp Leu Val Ile Val Leu Thr
               500                 505                 510
   Gly Trp Arg Pro Gly Ser Gly Tyr Thr Asn Thr Met Arg Val Val Pro
           515                 520                 525
   Val Pro
       530
<210> 18
   <211> 1593
   <212> DNA
   <213> Gallus gallus
<400> 18
   atgtcgaagc accacgatgc agggaccgct ttcatccaga cccagcagct gcacgctgcc 60
   atggcagaca cctttctgga gcacatgtgc cgcctggaca tcgactccga gccaaccatt 120
   gccagaaaca ccggcatcat ctgcaccatc ggcccagcct cccgctctgt ggacaagctg 180
   aaggaaatga ttaaatctgg aatgaatgtt gcccgcctca acttctcgca cggcacccac 240
   gagtatcatg agggcacaat taagaacgtg cgagaggcca cagagagctt tgcctctgac 300
   ccgatcacct acagacctgt ggctattgca ctggacacca agggacctga aatccgaact 360
   ggactcatca agggaagtgg cacagcagag gtggagctca agaagggcgc agctctcaaa 420
   gtgacgctgg acaatgcctt catggagaac tgcgatgaga atgtgctgtg ggtggactac 480
   aagaacctca tcaaagttat agatgtgggc agcaaaatct atgtggatga cggtctcatt 540
   tccttgctgg ttaaggagaa aggcaaggac tttgtcatga ctgaggttga gaacggtggc 600
   atgcttggta gtaagaaggg agtgaacctc ccaggtgctg cggtcgacct gcctgcagtc 660
   tcagagaagg acattcagga cctgaaattt ggcgtggagc agaatgtgga catggtgttc 720
   gcttccttca tccgcaaagc tgctgatgtc catgctgtca ggaaggtgct aggggaaaag 780
   ggaaagcaca tcaagattat cagcaagatt gagaatcacg agggtgtgcg caggtttgat 840
   gagatcatgg aggccagcga tggcattatg gtggcccgtg gtgacctggg tattgagatc 900
   cctgctgaaa aagtcttcct cgcacagaag atgatgattg ggcgctgcaa cagggctggc 960
   aaacccatca tttgtgccac tcagatgttg gaaagcatga tcaagaaacc tcgcccgacc 1020
   cgcgctgagg gcagtgatgt tgccaatgca gttctggatg gagcagactg catcatgctg 1080
   tctggggaga ccgccaaggg agactaccca ctggaggctg tgcgcatgca gcacgctatt 1140
   gctcgtgagg ctgaggccgc aatgttccat cgtcagcagt ttgaagaaat cttacgccac 1200
   agtgtacacc acagggagcc tgctgatgcc atggcagcag gcgcggtgga ggcctccttt 1260
   aagtgcttag cagcagctct gatagttatg accgagtctg gcaggtctgc acacctggtg 1320
   tcccggtacc gcccgcgggc tcccatcatc gccgtcaccc gcaatgacca aacagcacgc 1380
   caggcacacc tgtaccgcgg cgtcttcccc gtgctgtgca agcagccggc ccacgatgcc 1440
   tgggcagagg atgtggatct ccgtgtgaac ctgggcatga atgtcggcaa agcccgtgga 1500
   ttcttcaaga ccggggacct ggtgatcgtg ctgacgggct ggcgccccgg ctccggctac 1560
   accaacacca tgcgggtggt gcccgtgcca tga                              1593
<210> 19
   <211> 9
   <212> PRT
   <213> Artificial Sequence
<220>
   <223> Description of Artificial Sequence: hemagglutinin epitope tag
<400> 19
   Tyr Pro Tyr Asp Val Pro Asp Tyr Ala
    1               5
<210> 20
   <211> 27
   <212> DNA
   <213> Artificial Sequence
<220>
   <223> Description of Artificial Sequence: encodes hemagglutinin epitope tag
<400> 20
   tacccctacg acgtgcccga ctacgcc 27
<210> 21
   <211> 594
   <212> PRT
   <213> Rattus norvegicus
<400> 21
   Met Glu Ser Asn His Lys Ser Gly Asp Gly Leu Ser Gly Thr Gln Lys
    1               5                  10                  15
   Glu Ala Ala Leu Arg Ala Leu Val Gln Arg Thr Gly Tyr Ser Leu Val
               20                  25                  30
   Gln Glu Asn Gly Gln Arg Lys Tyr Gly Gly Pro Pro Pro Gly Trp Asp
           35                  40                  45
   Thr Thr Pro Pro Glu Arg Gly Cys Glu Ile Phe Ile Gly Lys Leu Pro
       50                  55                  60
   Arg Asp Leu Phe Glu Asp Glu Leu Ile Pro Leu Cys Glu Lys Ile Gly
   65                  70                  75                  80
   Lys Ile Tyr Glu Met Arg Met Met Met Asp Phe Asn Gly Asn Asn Arg
                   85                  90                  95
   Gly Tyr Ala Phe Val Thr Phe Ser Asn Lys Gln Glu Ala Lys Asn Ala
               100                 105                 110
   Ile Lys Gln Leu Asn Asn Tyr Glu Ile Arg Asn Gly Arg Leu Leu Gly
           115                 120                 125
   Val Cys Ala Ser Val Asp Asn Cys Arg Leu Phe Val Gly Gly Ile Pro
       130                 135                 140
   Lys Thr Lys Lys Arg Glu Glu Ile Leu Ser Glu Met Lys Lys Val Thr
   145                 150                 155                 160
   Glu Gly Val Val Asp Val Ile Val Tyr Pro Ser Ala Ala Asp Lys Thr
                   165                 170                 175
   Lys Asn Arg Gly Phe Ala Phe Val Glu Tyr Glu Ser His Arg Ala Ala
               180                 185                 190
   Ala Met Ala Arg Arg Arg Leu Leu Pro Gly Arg Ile Gln Leu Trp Gly
           195                 200                 205
   His Pro Ile Ala Val Asp Trp Ala Glu Pro Glu Val Glu Val Asp Glu
       210                 215                 220
   Asp Thr Met Ser Ser Val Lys Ile Leu Tyr Val Arg Asn Leu Met Leu
   225                 230                 235                 240
   Ser Thr Ser Glu Glu Met Ile Glu Lys Glu Phe Asn Ser Ile Lys Pro
                   245                 250                 255
   Gly Ala Val Glu Arg Val Lys Lys Ile Arg Asp Tyr Ala Phe Val His
               260                 265                 270
   Phe Ser Asn Arg Glu Asp Ala Val Glu Ala Met Lys Ala Leu Asn Gly
           275                 280                 285
   Lys Val Leu Asp Gly Ser Pro Ile Glu Val Thr Leu Ala Lys Pro Val
       290                 295                 300
   Asp Lys Asp Ser Tyr Val Arg Tyr Thr Arg Gly Thr Gly Gly Arg Asn
   305                 310                 315                 320
   Thr Met Leu Gln Glu Tyr Thr Tyr Pro Leu Ser His Val Tyr Asp Pro
                   325                 330                 335
   Thr Thr Thr Tyr Leu Gly Ala Pro Val Phe Tyr Thr Pro Gln Ala Tyr
               340                 345                 350
   Ala Ala Ile Pro Ser Leu His Phe Pro Ala Thr Lys Gly His Leu Ser
           355                 360                 365
   Asn Arg Ala Leu Ile Arg Thr Pro Ser Val Arg Glu Ile Tyr Met Asn
       370                 375                 380
   Val Pro Val Gly Ala Ala Gly Val Arg Gly Leu Gly Gly Arg Gly Tyr
   385                 390                 395                 400
   Leu Ala Tyr Thr Gly Leu Gly Arg Gly Tyr Gln Val Lys Gly Asp Lys
                   405                 410                 415
   Arg Gln Asp Lys Leu Tyr Asp Leu Leu Pro Gly Met Glu Leu Thr Pro
               420                 425                 430
   Met Asn Thr Ile Ser Leu Lys Pro Gln Gly Val Lys Leu Ala Pro Gln
           435                 440                 445
   Ile Leu Glu Glu Ile Cys Gln Lys Asn Asn Trp Gly Gln Pro Val Tyr
       450                 455                 460
   Gln Leu His Ser Ala Ile Gly Gln Asp Gln Arg Gln Leu Phe Leu Tyr
   465                 470                 475                 480
   Lys Val Thr Ile Pro Ala Leu Ala Ser Gln Asn Pro Ala Ile His Pro
                   485                 490                 495
   Phe Thr Pro Pro Lys Leu Ser Ala Tyr Val Asp Glu Ala Lys Arg Tyr
               500                 505                 510
   Ala Ala Glu His Thr Leu Gln Thr Leu Gly Ile Pro Thr Glu Gly Gly
           515                 520                 525
   Asp Ala Gly Thr Thr Ala Pro Thr Ala Thr Ser Ala Thr Val Phe Pro
       530                 535                 540
   Gly Tyr Ala Val Pro Ser Ala Thr Ala Pro Val Ser Thr Ala Gln Leu
   545                 550                 555                 560
   Lys Gln Ala Val Thr Leu Gly Gln Asp Leu Ala Ala Tyr Thr Thr Tyr
                   565                 570                 575
   Glu Val Tyr Pro Thr Phe Ala Val Thr Thr Arg Gly Asp Gly Tyr Gly
               580                 585                 590
   Thr Phe
<210> 22
   <211> 1785
   <212> DNA
   <213> Rattus norvegicus
<400> 22
   atggaatcaa atcacaaatc cggggatgga ttgagcggca cccagaagga agcagcactc 60
   cgcgcactgg tccagcgcac aggatatagc ttggtccagg aaaatggaca aagaaaatat 120
   ggtggtcctc caccaggctg ggatactaca cccccagaaa ggggctgcga gattttcatt 180
   gggaaacttc cccgggacct ttttgaggat gaactcatac cattgtgtga aaaaattggt 240
   aaaatttatg aaatgagaat gatgatggat ttcaatggga acaacagagg ctatgcattt 300
   gtaaccttct caaataagca ggaagccaag aatgcaatca agcaacttaa taattatgaa 360
   attcggaatg gccgtctcct gggcgtctgt gccagtgtgg acaactgccg gttgtttgtg 420
   gggggaatcc ccaaaaccaa aaagagagaa gaaatcttgt cagagatgaa aaaggtcact 480
   gaaggagttg ttgatgtcat tgtctaccca agcgctgccg ataaaaccaa aaaccggggg 540
   tttgcctttg tggaatatga gagtcaccgc gcagccgcca tggctaggcg gaggctgctg 600
   ccaggaagaa ttcagttgtg gggacatcct atcgcagtag actgggcaga gccagaagtc 660
   gaagttgacg aagacacaat gtcttccgtg aaaatcctgt acgtaaggaa ccttatgctg 720
   tctacctcgg aagagatgat tgagaaggaa ttcaacagta ttaaaccagg tgctgtggaa 780
   cgggtgaaga agatccgaga ctatgctttt gtgcatttca gtaaccgaga agatgcagtt 840
   gaagccatga aggctttgaa tggcaaggtg ctggatggtt ccccaataga agtgaccttg 900
   gccaagccag tggacaagga cagttacgtt aggtacaccc ggggcaccgg gggcaggaac 960
   accatgctgc aagaatacac ctaccctctg agccatgttt atgaccctac cacaacctac 1020
   cttggagctc ctgtcttcta tactccccaa gcctacgcag ccattccaag tcttcatttc 1080
   ccagctacca aaggacatct cagcaacaga gctctcatcc ggaccccttc tgtcagagaa 1140
   atttacatga atgtccctgt aggggctgcg ggcgtgagag gactgggcgg ccgtgggtat 1200
   ttggcatata caggcctggg tcgaggatac caggtcaaag gagacaagag acaagacaaa 1260
   ctctatgacc ttctgcctgg gatggagctc accccgatga atactatctc tttaaaacca 1320
   caaggagtta aacttgctcc tcagatatta gaagaaatct gtcagaaaaa taactgggga 1380
   cagccagtgt accagctgca ctctgccatt ggacaagacc aaagacagtt attcctatac 1440
   aaagtaacta tcccagcgct ggccagccag aatcctgcga tccacccttt cacaccccca 1500
   aagctaagcg cctacgtgga tgaagcaaag aggtacgccg cagagcacac cctacagaca 1560
   ctaggcatcc ccacagaagg aggggacgct gggactacag cacccactgc cacatccgcc 1620
   actgtgtttc caggatacgc tgtccccagt gccaccgctc ctgtgtctac agcccagctc 1680
   aagcaagcag tgacacttgg acaagactta gcagcatata caacctatga ggtctaccct 1740
   acttttgcag tgaccacccg aggtgatgga tatggcacct tctga                1785
<210> 23
   <211> 586
   <212> PRT
   <213> Homo sapiens
<400> 23
<210> 24
   <211> 1761
   <212> DNA
   <213> Homo sapiens
<400> 24
   atggaatcaa atcacaaatc cggggatgga ttgagcggca ctcagaagga agcagccctc 60
   cgcgcactgg tccagcgcac aggatatagc ttggtccagg aaaatggaca aagaaaatat 120
   ggtggccctc cacctggttg ggatgctgca ccccctgaaa ggggctgtga aatttttatt 180
   ggaaaacttc cccgagacct ttttgaggat gagcttatac cattatgtga aaaaatcggt 240
   aaaatttatg aaatgagaat gatgatggat tttaatggca acaatagagg atatgcattt 300
   gtaacatttt caaataaagt ggaagccaag aatgcaatca agcaacttaa taattatgaa 360
   attagaaatg ggcgcctctt aggggtttgt gccagtgtgg acaactgccg attatttgtt 420
   gggggcatcc caaaaaccaa aaagagagaa gaaatcttat cggagatgaa aaaggttact 480
   gaaggtgttg tcgatgtcat cgtctaccca agcgctgcag ataaaaccaa aaaccgaggc 540
   tttgccttcg tggagtatga gagtcatcga gcagctgcca tggcgaggag gaaactgcta 600
   ccaggaagaa ttcagttatg gggacatggt attgcagtag actgggcaga gccagaagta 660
   gaagttgatg aagatacaat gtcttcagtg aaaatcctat atgtaagaaa tcttatgctg 720
   tctacctctg aagagatgat tgaaaaggaa ttcaacaata tcaaaccagg tgctgtggag 780
   agggtgaaga aaattcgaga ctatgctttt gtgcacttca gtaaccgaaa agatgcagtt 840
   gaggctatga aagctttaaa tggcaaggtg ctggatggtt cccccattga agtcacccta 900
   gcaaaaccag tggacaagga cagttatgtt aggtataccc gaggcacagg tggaaggggc 960
   accatgctgc aaggagagta tacctactct ttgggccaag tttatgatcc caccacaacc 1020
   taccttggag ctcctgtctt ctatgccccc cagacctatg cagcaattcc cagtcttcat 1080
   ttcccagcca ccaaaggaca tctcagcaac agagccatta tccgagcccc ttctgttaga 1140
   ggggctgcgg gagtgagagg actgggcggc cgtggctatt tggcatacac aggcctgggt 1200
   cgaggatacc aggtcaaagg agacaaaaga gaagacaaac tctatgacat tttacctggg 1260
   atggagctca ccccaatgaa tcctgtcaca ttaaaacccc aaggaattaa actcgctccc 1320
   cagatattag aagagatttg tcagaaaaat aactggggac agccagtgta ccagctgcac 1380
   tctgctattg gacaagacca aagacagcta ttcttgtaca aaataactat tcctgctcta 1440
   gccagccaga atcctgcaat ccaccctttc acacctccaa agctgagtgc ctttgtggat 1500
   gaagcaaaga cgtatgcagc cgaatacacc ctgcagaccc tgggcatccc cactgatgga 1560
   ggcgatggca ccatggctac tgctgctgct gctgctactg ctttcccagg atatgctgtc 1620
   cctaatgcaa ctgcacccgt gtctgcagcc cagctcaagc aagcggtaac ccttggacaa 1680
   gacttagcag catatacaac ctatgaggtc tacccaactt ttgcagtgac tgcccgaggg 1740
   gatggatatg gcaccttctg a                                          1761
<210> 25
   <211> 45
   <212> DNA
<213> Artificial Sequence
<220>
   <223> Description of Artificial Sequence: oligomer encoding TAT protein transduction domain
<400> 25
   catatgggaa gaaaaaaaag aagacaaaga agaagaggcc tcgag 45
<210> 26
   <211> 2274
   <212> DNA
   <213> Artificial Sequence
<220>
   <223> Description of Artificial Sequence:
   HA-rAPOBEC-CMPK construct
<400> 26
   atgggctcta gataccccta cgacgtgccc gactacgccg atatcagttc cgagacaggc 60
   cctgtagctg ttgatcccac tctgaggaga agaattgagc cccacgagtt tgaagtcttc 120
   tttgaccccc gggaacttcg gaaagagacc tgtctgctgt atgagatcaa ctggggagga 180
   aggcacagca tctggcgaca cacgagccaa aacaccaaca aacacgttga agtcaatttc 240
   atagaaaaat ttactacaga aagatacttt tgtccaaaca ccagatgctc cattacctgg 300
   ttcctgtcct ggagtccctg tggggagtgc tccagggcca ttacagaatt tttgagccga 360
   tacccccatg taactctgtt tatttatata gcacggcttt atcaccacgc agatcctcga 420
   aatcggcaag gactcaggga ccttattagc agcggtgtta ctatccagat catgacggag 480
   caagagtctg gctactgctg gaggaatttt gtcaactact ccccttcgaa tgaagctcat 540
   tggccaaggt acccccatct gtgggtgagg ctgtacgtac tggaactcta ctgcatcatt 600
   ttaggacttc caccctgttt aaatatttta agaagaaaac aacctcaact cacgtttttc 660
   acgattgctc ttcaaagctg ccattaccaa aggctaccac cccacatcct gtgggccaca 720
   gggttgaaag aattccacgc tgccatggca gacacctttc tggagcacat gtgccgcctg 780
   gacatcgact ccgagccaac cattgccaga aacaccggca tcatctgcac catcggccca 840
   gcctcccgct ctgtggacaa gctgaaggaa atgattaaat ctggaatgaa tgttgcccgc 900
   ctcaacttct cgcacggcac ccacgagtat catgagggca caattaagaa cgtgcgagag 960
   gccacagaga gctttgcctc tgacccgatc acctacagac ctgtggctat tgcactggac 1020
   accaagggac ctgaaatccg aactggactc atcaagggaa gtggcacagc agaggtggag 1080
   ctcaagaagg gcgcagctct caaagtgacg ctggacaatg ccttcatgga gaactgcgat 1140
   gagaatgtgc tgtgggtgga ctacaagaac ctcatcaaag ttatagatgt gggcagcaaa 1200
   atctatgtgg atgacggtct catttccttg ctggttaagg agaaaggcaa ggactttgtc 1260
   atgactgagg ttgagaacgg tggcatgctt ggtagtaaga agggagtgaa cctcccaggt 1320
   gctgcggtcg acctgcctgc agtctcagag aaggacattc aggacctgaa atttggcgtg 1380
   gagcagaatg tggacatggt gttcgcttcc ttcatccgca aagctgctga tgtccatgct 1440
   gtcaggaagg tgctagggga aaagggaaag cacatcaaga ttatcagcaa gattgagaat 1500
   cacgagggtg tgcgcaggtt tgatgagatc atggaggcca gcgatggcat tatggtggcc 1560
   cgtggtgacc tgggtattga gatccctgct gaaaaagtct tcctcgcaca gaagatgatg 1620
   attgggcgct gcaacagggc tggcaaaccc atcatttgtg ccactcagat gttggaaagc 1680
   atgatcaaga aacctcgccc gacccgcgct gagggcagtg atgttgccaa tgcagttctg 1740
   gatggagcag actgcatcat gctgtctggg gagaccgcca agggagacta cccactggag 1800
   gctgtgcgca tgcagcacgc tattgctcgt gaggctgagg ccgcaatgtt ccatcgtcag 1860
   cagtttgaag aaatcttacg ccacagtgta caccacaggg agcctgctga tgccatggca 1920
   gcaggcgcgg tggaggcctc ctttaagtgc ttagcagcag ctctgatagt tatgaccgag 1980
   tctggcaggt ctgcacacct ggtgtcccgg taccgcccgc gggctcccat catcgccgtc 2040
   acccgcaatg accaaacagc acgccaggca cacctgtacc gcggcgtctt ccccgtgctg 2100
   tgcaagcagc cggcccacga tgcctgggca gaggatgtgg atctccgtgt gaacctgggc 2160
   atgaatgtcg gcaaagcccg tggattcttc aagaccgggg acctggtgat cgtgctgacg 2220
   ggctggcgcc ccggctccgg ctacaccaac accatgcggg tggtgcccgt gcca       2274
<210> 27
   <211> 1590
   <212> DNA
   <213> Artificial Sequence
<220>
   <223> Description of Artificial Sequence: HA-CMPK construct
<400> 27
   ctcgagatgt acccctacga cgtgcccgac tacgccgata tccacgctgc catggcagac 60
   acctttctgg agcacatgtg ccgcctggac atcgactccg agccaaccat tgccagaaac 120
   accggcatca tctgcaccat cggcccagcc tcccgctctg tggacaagct gaaggaaatg 180
   attaaatctg gaatgaatgt tgcccgcctc aacttctcgc acggcaccca cgagtatcat 240
   gagggcacaa ttaagaacgt gcgagaggcc acagagagct ttgcctctga cccgatcacc 300
   tacagacctg tggctattgc actggacacc aagggacctg aaatccgaac tggactcatc 360
   aagggaagtg gcacagcaga ggtggagctc aagaagggcg cagctctcaa agtgacgctg 420
   gacaatgcct tcatggagaa ctgcgatgag aatgtgctgt gggtggacta caagaacctc 480
   atcaaagtta tagatgtggg cagcaaaatc tatgtggatg acggtctcat ttccttgctg 540
   gttaaggaga aaggcaagga ctttgtcatg actgaggttg agaacggtgg catgcttggt 600
   agtaagaagg gagtgaacct cccaggtgct gcggtcgacc tgcctgcagt ctcagagaag 660
   gacattcagg acctgaaatt tggcgtggag cagaatgtgg acatggtgtt cgcttccttc 720
   atccgcaaag ctgctgatgt ccatgctgtc aggaaggtgc taggggaaaa gggaaagcac 780
   atcaagatta tcagcaagat tgagaatcac gagggtgtgc gcaggtttga tgagatcatg 840
   gaggccagcg atggcattat ggtggcccgt ggtgacctgg gtattgagat ccctgctgaa 900
   aaagtcttcc tcgcacagaa gatgatgatt gggcgctgca acagggctgg caaacccatc 960
   atttgtgcca ctcagatgtt ggaaagcatg atcaagaaac ctcgcccgac ccgcgctgag 1020
   ggcagtgatg ttgccaatgc agttctggat ggagcagact gcatcatgct gtctggggag 1080
   accgccaagg gagactaccc actggaggct gtgcgcatgc agcacgctat tgctcgtgag 1140
   gctgaggccg caatgttcca tcgtcagcag tttgaagaaa tcttacgcca cagtgtacac 1200
   cacagggagc ctgctgatgc catggcagca ggcgcggtgg aggcctcctt taagtgctta 1260
   gcagcagctc tgatagttat gaccgagtct ggcaggtctg cacacctggt gtcccggtac 1320
   cgcccgcggg ctcccatcat cgccgtcacc cgcaatgacc aaacagcacg ccaggcacac 1380
   ctgtaccgcg gcgtcttccc cgtgctgtgc aagcagccgg cccacgatgc ctgggcagag 1440
   gatgtggatc tccgtgtgaa cctgggcatg aatgtcggca aagcccgtgg attcttcaag 1500
   accggggacc tggtgatcgt gctgacgggc tggcgccccg gctccggcta caccaacacc 1560
   atgcgggtgg tgcccgtgcc atgactcgag                                   1590
<210> 28
   <211> 1629
   <212> DNA
   <213> Artificial Sequence
<220>
   <223> Description of Artificial Sequence: TAT-HA-CMPK construct
<400> 28
   catatgggaa gaaaaaaaag aagacaaaga agaagaggcc tcgagatgta cccctacgac 60
   gtgcccgact acgccgatat ccacgctgcc atggcagaca cctttctgga gcacatgtgc 120
   cgcctggaca tcgactccga gccaaccatt gccagaaaca ccggcatcat ctgcaccatc 180
   ggcccagcct cccgctctgt ggacaagctg aaggaaatga ttaaatctgg aatgaatgtt 240
   gcccgcctca acttctcgca cggcacccac gagtatcatg agggcacaat taagaacgtg 300
   cgagaggcca cagagagctt tgcctctgac ccgatcacct acagacctgt ggctattgca 360
   ctggacacca agggacctga aatccgaact ggactcatca agggaagtgg cacagcagag 420
   gtggagctca agaagggcgc agctctcaaa gtgacgctgg acaatgcctt catggagaac 480
   tgcgatgaga atgtgctgtg ggtggactac aagaacctca tcaaagttat agatgtgggc 540
   agcaaaatct atgtggatga cggtctcatt tccttgctgg ttaaggagaa aggcaaggac 600
   tttgtcatga ctgaggttga gaacggtggc atgcttggta gtaagaaggg agtgaacctc 660
   ccaggtgctg cggtcgacct gcctgcagtc tcagagaagg acattcagga cctgaaattt 720
   ggcgtggagc agaatgtgga catggtgttc gcttccttca tccgcaaagc tgctgatgtc 780
   catgctgtca ggaaggtgct aggggaaaag ggaaagcaca tcaagattat cagcaagatt 840
   gagaatcacg agggtgtgcg caggtttgat gagatcatgg aggccagcga tggcattatg 900
   gtggcccgtg gtgacctggg tattgagatc cctgctgaaa aagtcttcct cgcacagaag 960
   atgatgattg ggcgctgcaa cagggctggc aaacccatca tttgtgccac tcagatgttg 1020
   gaaagcatga tcaagaaacc tcgcccgacc cgcgctgagg gcagtgatgt tgccaatgca 1080
   gttctggatg gagcagactg catcatgctg tctggggaga ccgccaaggg agactaccca 1140
   ctggaggctg tgcgcatgca gcacgctatt gctcgtgagg ctgaggccgc aatgttccat 1200
   cgtcagcagt ttgaagaaat cttacgccac agtgtacacc acagggagcc tgctgatgcc 1260
   atggcagcag gcgcggtgga ggcctccttt aagtgcttag cagcagctct gatagttatg 1320
   accgagtctg gcaggtctgc acacctggtg tcccggtacc gcccgcgggc tcccatcatc 1380
   gccgtcaccc gcaatgacca aacagcacgc caggcacacc tgtaccgcgg cgtcttcccc 1440
   gtgctgtgca agcagccggc ccacgatgcc tgggcagagg atgtggatct ccgtgtgaac 1500
   ctgggcatga atgtcggcaa agcccgtgga ttcttcaaga ccggggacct ggtgatcgtg 1560
   ctgacgggct ggcgccccgg ctccggctac accaacacca tgcgggtggt gcccgtgcca 1620
   tgactcgag                                                         1629
<210> 29
   <211> 23
   <212> DNA
   <213> Artificial Sequence
<220>
   <223> Description of Artificial Sequence: primer ND1
<400> 29
   atctgactgg gagagacaag tag 23
<210> 30
   <211> 23
   <212> DNA
   <213> Artificial Sequence
<220>
   <223> Description of Artificial Sequence: primer ND2
<400> 30
   gttcttttta agtcctgtgc atc 23
<210> 31
   <211> 35
   <212> DNA
   <213> Artificial Sequence
<220>
   <223> Description of Artificial Sequence: primer DD3
<400> 31
   aatcatgtaa atcataacta tctttaatat actga 35